(19) Europäisches Patentamt
European Patent Office
Office européen des brevets

(11) Publication number: 0 030 032
B1

(12) # EUROPEAN PATENT SPECIFICATION

(45) Date of publication of patent specification: 27.08.86

(21) Application number: 80107512.8

(22) Date of filing: 02.12.80

(51) Int. Cl.[4]: C 07 D 487/04, C 07 D 205/08, C 07 D 401/04, C 07 F 7/10, A 61 K 31/40 // (C07D487/04, 209:00, 205:00),(C07D401/04, 213:00, 205:00)

(54) **6-, 1- and 2-substituted-1-carbadethiapen-2-em-3-carboxylic acids, processes for preparing the same, pharmaceutical compositions containing the same, and intermediates.**

(30) Priority: 03.12.79 US 99275
03.12.79 US 99285
03.12.79 US 99400
03.12.79 US 99451

(43) Date of publication of application:
10.06.81 Bulletin 81/23

(45) Publication of the grant of the patent:
27.08.86 Bulletin 86/35

(84) Designated Contracting States:
AT BE CH DE FR GB IT LI LU NL SE

(56) References cited:
EP-A-0 000 380
EP-A-0 004 132
EP-A-0 007 973
EP-A-0 010 316
EP-A-0 010 317
US-A-3 950 357

Chem. Soc., Chem. Commun., 1979, no. 4, 15.02.79, pp. 236-237

(73) Proprietor: MERCK & CO. INC.
126, East Lincoln Avenue P.O. Box 2000
Rahway New Jersey 07065 (US)

(72) Inventor: Christensen, Burton G.
195 Watchung Terrace
Scotch Plains New Jersey 07060 (US)
Inventor: Shih, David H.
2 Colby Court
Manalapan New Jersey 07726 (US)

(74) Representative: Abitz, Walter, Dr.-Ing. et al
Abitz, Morf, Gritschneder P.O. Box 86 01 09
D-8000 München 86 (DE)

EP 0 030 032 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

This invention relates to 6-, 1- and 2-substituted 1-carbadethiapen-2-em-3-carboxylic acids and derivatives thereof which are useful as antibiotics and which may be represented by the following generic structural formula (I):

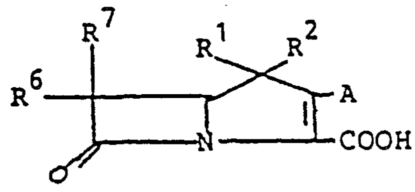

(I)

and the pharmaceutically acceptable salts and esters thereof, wherein either

a) A is $SR^8$ and $R^1$, $R^2$, $R^6$, $R^7$ and $R^8$ are independently selected from the group consisting of substituted and unsubstituted alkyl, alkenyl, and alkynyl, having from 1—10 carbon atoms; cycloalkyl, cycloalkylalkyl, and alkylcycloalkyl, having 3—6 carbon atoms in the cycloalkyl ring and 1—6 carbon atoms in the alkyl noieties; spirocycloalkyl having 3—6 carbon atoms; phenyl; aralkyl, aralkenyl, and aralkynyl wherein the aryl moiety is phenyl and the alkyl chain has 1—6 carbon atoms; heteroaryl, heteroaralkyl, heterocyclyl and heterocyclylalkyl wherein the substituent or substituents relative to the above-named radicals are selected from the group consisting of: amino, mono-, di-, and trialkylamino, hydroxyl, alkoxyl, mercapto, alkylthio, phenylthio, sulfamoyl, amidino, guanidino, nitro, chloro, bromo, fluoroiodo, cyano and carboxy; and wherein the hetero atom or atoms in the above-named heterocyclic moieties are selected from the group consisting of 1—4 oxygen, nitrogen or sulphur atoms; and wherein the alkyl moieties of the above recited substituents have 1—6 carbon atoms and $R^6$, $R^7$ and $R^8$ can also represent hydrogen; when $R^6/R^7$ is hydrogen and $R^7/R^6$ is 1-hydroxyethyl, then $R^8$ is not 2-aminoethyl or an N-derivative thereof; or

b) A is $R^8$ and $R^1$, $R^2$, $R^6$, $R^7$ and $R^8$ are independently selected from the group consisting of substituted and unsubstituted : alkyl having 1—6 carbon atoms, aralkyl, alkenyl and alkynyl having 2—6 carbon atoms, aryl and aralkyl having 6—10 ring carbon atoms and 1—6 carbon atoms in the alkyl chain, heterocyclyl, heterocyclylthio ($R^8$ is excluded) and heterocyclylalkyl having 1—5 hetero atoms selected from O, N or S in the ring and 1—6 carbon atoms in the alkyl chain, cycloalkyl, spirocycloalkyl, and cycloalkyl having 3 to 6 ring carbon atoms and 1—6 carbon atoms in the alkyl moiety; wherein the substituents on $R^1$, $R^2$, $R^6$, $R^7$ and $R^8$ are selected from chloro, bromo, fluoro, iodo, hydroxyl, amino, mono-, di- and trialkyl substituted amino (each alkyl having 1—6 carbon atoms) alkoxyl having 1—6 carbon atoms, guanidino, cyano, amidino and carboxyl; and $R^6$, $R^7$ and $R^8$ can also represent hydrogen.

This invention also relates to processes for the preparation of such compounds (I) and to pharmaceutical compositions comprising such compounds, which can be administered when an antibiotic effect is indicated.

Modified thienamycin antibiotics (1-carbadethiapen-2-em-3-carboxylic acids) are known from EP—A—4132. EP—A—380 describes substituted penicillin antibiotics. 4-Allyl-azetidine-2-one is known from J. Chem. Soc., Chem. Commun. 1979, No. 4, p. 236—237.

However, there is a continuing need for new antibiotics; for unfortunately, there is no static effectiveness of any given antibiotic because continued wide scale usage selectively gives rise to resistant strains of pathogens. In addition, the known antibiotics suffer from the disadvantage of being effective only against certain types of microorganisms. Accordingly, the search for new antibiotics continues.

Thus, it is an objection of the present invention to provide a novel class of antibiotics which are useful in animal and human therapy and in inanimate systems. These antibiotics are active against a broad range of pathogens which representatively include both gram positive bacteria such as *S. aureua, Strep. pyogenes,* and *B. subtilis,* and gram negative bacteria such as *E. coli, Pseudomonas, Proteus morganii, Serratia,* and *Klebsiella.* Further objects of this invention are to provide chemical processes for the preparation of such antibiotics and their non-toxic pharmaceutically acceptable salts; pharmaceutical compositions comprising such antibiotics; and to provide methods of treatment comprising administering such antibiotics; and compositions when an antibiotic effect is indicated.

Detailed Description of the Invention

Compounds of the present invention wherein the 2-substituent is $—SR^8$ or —H are conveniently prepared according to the following reaction diagram:

DIAGRAM I

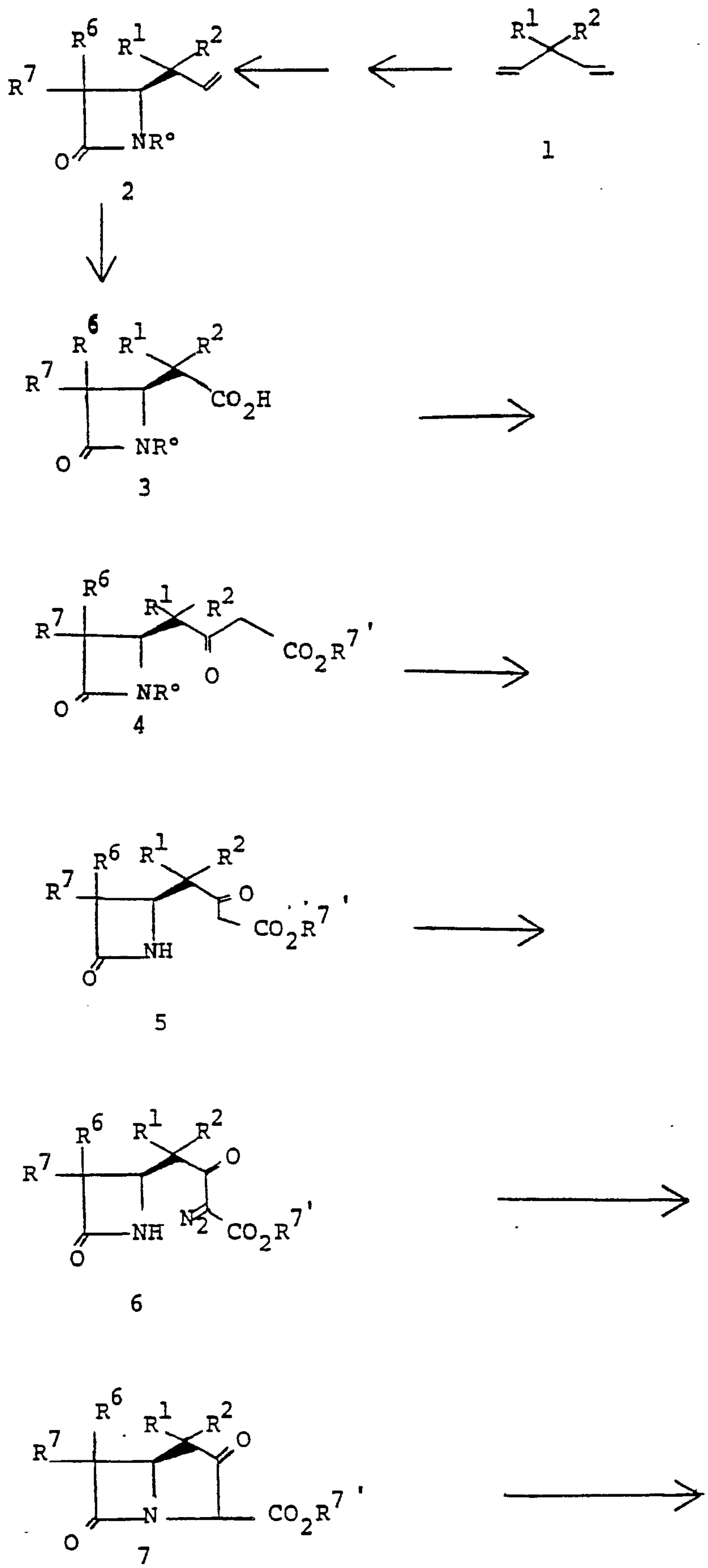

1

2

3

4

5

6

7

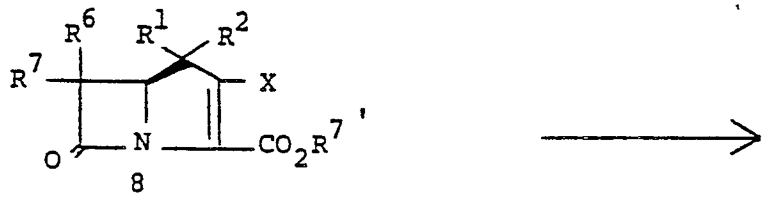

8

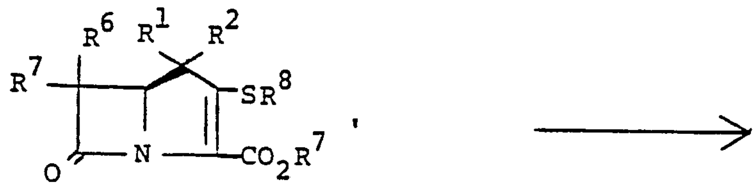

9

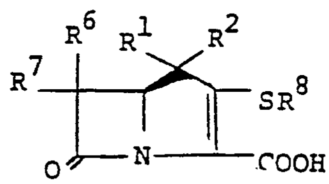

I

In words relative to diagram, the oxidation 2→3 is accomplished by treating 2 in a solvent such as methylenechloride, methanol, chloroform, or the like, with an oxidizing agent such as ozone, or the like, at a temperature of from −100° to 0°C for from 0.1 to 4 hours, followed by treating the crude product with an oxidizing agent such as m-chloroperbenzoic acid, hydrogen peroxide, peracetic acid, or the like, at a temperature of from 0°C to 100°C for from 1 to 100 hours. R° is a readily removable protecting group and is defined below.

The addition 3→4 is accomplished by treating 3 with 1,1′-carbonyldiimidazole, or the like, in a solvent such as tetrahydrofuran, dimethoxyethane, or the like, at a temperature of from 0 to 50°C, followed by the addition of 1.1 to 3.0 equivalent of $(R^{7\prime}O_2CCH_2CO_2)_2Mg$, at a temperature of from 0 to 50°C for from 1 to 48 hours. $R^{7\prime}$ is a pharmaceutically acceptable ester moiety or a readily removable carboxyl protecting groups such as p-nitrobenzyl or benzyl.

Removal of protecting group R°(4→5) is accomplished by acidic aqueous hydrolysis of 4 in a solvent such as methanol, ethanol, tetrahydrofuran, or dioxane in the presence of an acid such as hydrochloric, sulfuric or acetic at a temperature of from 0 to 100°C for from 2 to 18 hours.

The diazo species 6 is prepared from 5 by treating 5 in a solvent such as $CH_3CN$, $CH_2Cl_2$ or THF, with an azide such as p-carboxybenzenesulfonylazide, toluenesulfonylazide or methanesulfonylazide, in the presence of a base such as triethylamine, pyridine or $(C_2H_5)_2NH$, for from 1 to 50 hours at 0—25°C.

Cyclization (6→7) is accomplished by treating 6 in a solvent such as benzene, toluene or THF, at a temperature of from 50—110°C for from 1—5 hours in the presence of a catalyst such as *bis* (acetylacetonato)Cu(II) [$Cu(acac)_2$], $CuSO_4$, Cu powder, $Rh(OAc)_2$ or $Pd(OAC)_2$. Alternatively, the cyclization may be accomplished by irradiating 6 through a pyrex filter (a wave length greater than 300 nm) in a solvent such as benzene, $CCl_4$ or diethyl-ether, at a temperature of from 0—25°C for from 0.5 to 2 hours. ["OAc" = acetate]

Establishment of leaving group X(7→8) is accomplished by acylating the keto ester 7 with an acylating agent RX such as p-toluenesulfonic acid anhydride, p-nitrophenylsulfonic acid anhydride, 2,4,6-triisopropylphenylsulfonic acid anhydride, methanesulfonic acid anhydride, toluenesulfonyl chloride or p-bromophenylsulfonyl chloride, wherein X is the corresponding leaving group such as toluene sulfonyloxy, p-nitrophenylsulfonyloxy, methanesulfonyloxy, p-bromophenylsulfonyloxy and other leaving groups which are established by conventional procedures and which are well known in the art. Typically, the above acylation to establish leaving groups X is conducted in a solvent such as methylene chloride, acetonitrile or dimethylformamide, in the presence of a base such as diisopropylethylamine, triethylamine or 4-dimethylamino-pyridine at a temperature of from −20 to 40°C for from 0.5 to 5 hours. The leaving group X of intermediate 8 can also be halogen. The halogen leaving group is established by treating 7 with a

halogenating agent such as $\Phi_3PCl_2$, $\Phi PBr_2$, $(\Phi O)_3PBr_2$ or oxalyl chloride in a solvent such as $CH_2Cl_2$, $CH_3CN$ or THF, in the presence of a base such as diisopropylethylamine, triethylamine, or 4-dimethylaminopyridine [$\Phi$ = phenyl.]

The reaction 8→9 is accomplished by treating 8 in a solvent such as dioxane, dimethylformamide, dimethylsulfoxide, acetonitrile or hexamethylphosphoramide, in the presence of an approximately equivalent to excess of the mercaptan reagent $HSR^8$ wherein $R^8$ as defined above. A representative mercaptan reagent is $HSCH_2CH_2NHR^{8'}$ wherein $R^{8'}$ is hydrogen or a readily removable N-protecting group such as p-nitrobenzyloxycarbonyl or o-nitrobenzyloxy carbonal, in the presence of a base such as sodium hydrogen carbonate, potassium carbonate, triethylamine or diisopropylethylamine, at a temperature of from −40 to 25°C for from 1 to 72 hours. The mercaptan reagent, $HSCH_2CH_2NHR^{8'}$, is typically prepared by treating aminoethylmercaptan in the presence of the desired acid chloride in the presence of a base such as sodium bicarbonate or sodium hydroxide, in a solvent such as aqueous diethylether, aqueous dioxane or aqueous acetone, at a temperature of from 0 to 25°C for from 0.5 to 4 hours.

The final deblocking step 9→I is accomplished by conventional procedures such as hydrolysis or hydrogenation. Typically 9 in a solvent such as dioxane-water-ethanol or tetrahydrofuran-aqueous dipotassium hydrogen phosphate-isopropanol is treated under a hydrogen pressure of from 1 to 4 atmospheres in the presence of a hydrogenation catalyst such as palladium on charcoal or palladium hydroxide, at a temperature of from 0 to 50°C for from 0.5 to 4 hours to provide I.

Preparation of Starting Material 1 and 2

With respect to starting reagent 1, its preparation is generally described in *J. Amer. Chem. Soc., 74,* 661 (1952) by E. B. Reid and T. E. Gompf, *J. Org. Chem., 23,* 1063 (1958) by R. Ciola and K. L. Burwell, Jr., and BE—A—632,193 (1963) by R. Polster and E. Scharf. The following scheme summarizes the preparation of 1.

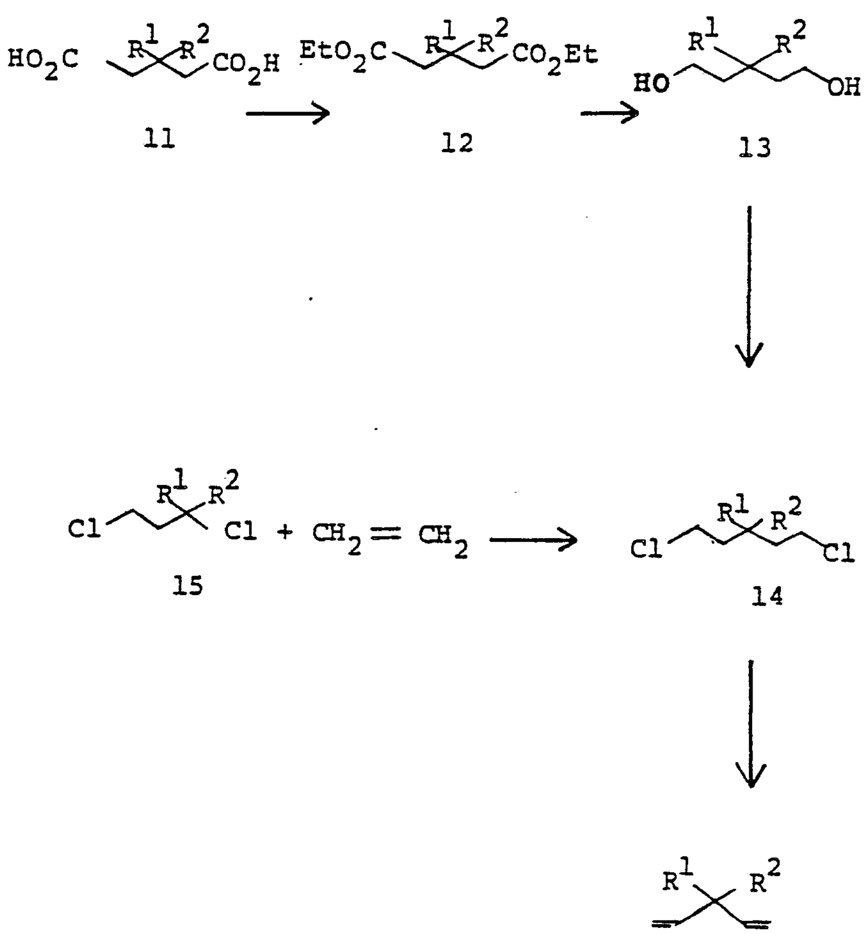

11 12 13

15 14

1

In words relative to the above scheme, the diester 12 is prepared by treating the diacid 11 with thionyl chloride at reflux for two hours followed by reacting with ethanol at 80°C for 4 hours. Reduction of the diester 12 with lithium alumium hydride in ether at reflux for 4 hours followed by hydrolysis with 10%

NaOH gives diol 13 which on further reaction with thionyl chloride gives dichloride 14. The dichloride 14 can be alternatively prepared by treating 15 with ethylene in the presence of alumium chloride. Treatment of the dichloride 14 with base such as 2-methylquinoline, DBU or sodium hydroxide in polyethylene glycol gives the expected 3-substituted 1,4-pentadiene 1.

Preparation of 2 is summarized in the following scheme:

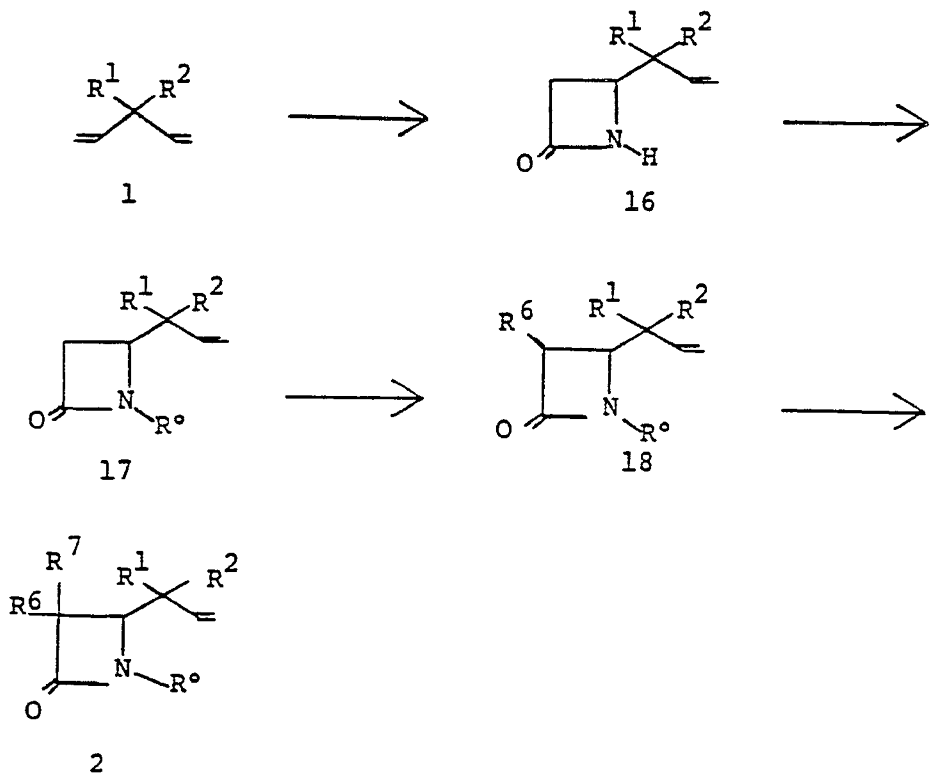

1 16 17 18 2

In words relative to above scheme, the substituted azetidinone 16 is prepared by reacting a 3-substituted 1,4-pentadiene 1 with chlorosulfonylisocyanate at 25°C to 60°C in a pressure bottle for 3—12 days, then the resulting mixture is hydrolyzed with aqueous sodium sulfite solution between pH 6.5—7.5 at 0°C to 25°C for from 5 min. to 60 min.

Azetidinone 16 is transformed (16→17) to establish the protecting group R° which may be a triorganosilyl group, such as t-butyldimethylsilyl, t-butyldiphenylsilyl, triphenylsilyl, isopropyldimethylsilyl, for example, or may be 3,4-dimethoxybenzyl, for example. Silyl protection is preferred, and typically R° is established by treating 1 in a solvent such as dimethylformamide, acetonitrile, hexamethylphosphoramide, or tetrahydrofuran with a silylating agent such as t-butyldimethylchlorosilane, t-butyldiphenylchlorosilane or triphenylchlorosilane, at a temperature of from −20°C to 25°C for from 0.5 to 24 hours in the presence of a base such as triethylamine, diisopropylethylamine, or imidazole.

Alkylation of 17 provides 18. Typically, 17 is treated with a strong base such as lithium diisopropylamide, sodium hydride, phenyl lithium or butyl lithium in a solvent such as tetrahydrofuran (THF), ether or dimethoxyethane at a temperature of from −80°C to 0°C, whereupon the alkylating agent of choice, $R^6X$ is added ($R^6$ is as described above and X is iodo, chloro or bromo; alternatively the alkylating agent may be $R^6$-tosylate, $R^6$-mesylate or an aldehyde or ketone such as acetaldehyde to provide monoalkylated species 18. When desired dialkylated species 2 may be obtained from 18 by repeating the alkylating procedure, 17→18.

In the foregoing description of the invention, suitable reagents $HSR^8$ (8→9) are representatively illustrated by the following list:

$HSCH_2CH_2CH_2NHCO_2PNB$,

$PNBO_2CNHCH_2CH_2CH_2SX$,

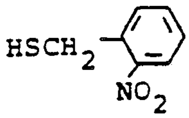

$HSCH_2CH_2NHCO_2PNB$

$HSC(CH_3)_2CH_2NHCO_2PNB$,

$HS\phi$,

$HSCH_2\phi$,

$HSC(CH_3)_3$,

$HSC\phi_3$,

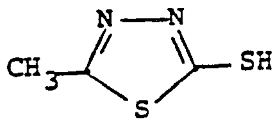

and the like ($\phi$ = phenyl; and PNB = p-nitrobenzyl),

$CH_3SH$,

$CH_3CH_2SH$,

$CH_3(CH_2)_2SH$,

$(CH_3)_2CHSH$,

$CH_3(CH_2)_3SH$,

$(CH_3)_2CH(CH_2)_2SH$,

$CH_2 \equiv CHCH_2SH$,

$CH \equiv CCH_2SH$,

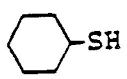

$\phi(CH_2)_3SH$($\phi$ = phenyl),

$\phi(CH_2)_2SH$,

$HO(CH_2)_2SH$,

$H_2N(CH_2)_2SH$,

$H_2N(CH_2)_3SH$,

$CH_3(CH_2)_2NH(CH_2)_2SH$,

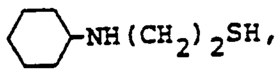

$(CH_3)_2N(CH_2)_2SH$,

$(CH_3CH_2)_2N(CH_2)_2SH$,

$HO_2C(CH_2)_2SH$,

$\phi CH_2SH$,

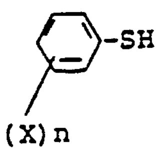

(n = 0, 1 or 2 ; X = Cl, Br, F, Cl, $OCH_3$, $CH_3NH_2$, $NH\overset{O}{\overset{\|}{C}}CH_3$ ),

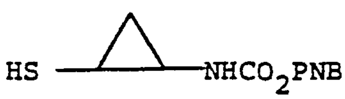

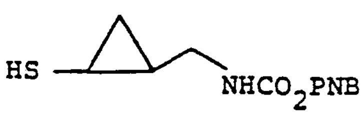

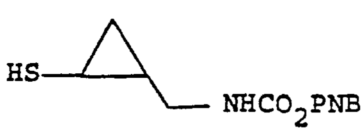

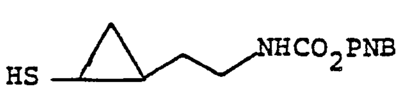

Similarly, suitable alkylating agents for establishing $R^6$ and/or $R^7$ at ultimate ring position 6 (17→18→2) are:
$\phi CH_2CHO$,
$\phi CH_2CH_2CHO$,
$CH_2O$,
$CH_3I$,
$\phi CH$ Br,
$CH_3COCH_3$.

Relative to the compounds of the present invention I:

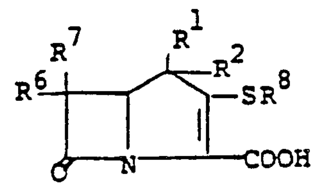

the most preferred values for $R^1$ and $R^2$ include:
ethyl,
propyl,
isopropyl,
cyclopropyl,
phenyl
benzyl
spiro-cyclopropyl

The most preferred radicals for $R^6$ and $R^7$ are: $R^6$ = H and $R^7$ is selected from hydroxymethyl, 1-hydroxyethyl, 1-hydroxypropyl, 2-hydroxypropyl, 2-hydroxyethyl; the most preferred values for $R^8$ are: aminoethylthio, aminopropylthio, aminocyclopropylthio, aminoisopropylthio, amidinoisopropylthio, and guanidinoethylthio.

Compounds of the present invention wherein the 2-substituent is $R^8$ or H are conveniently prepared according to the following reaction diagram:

DIAGRAM II

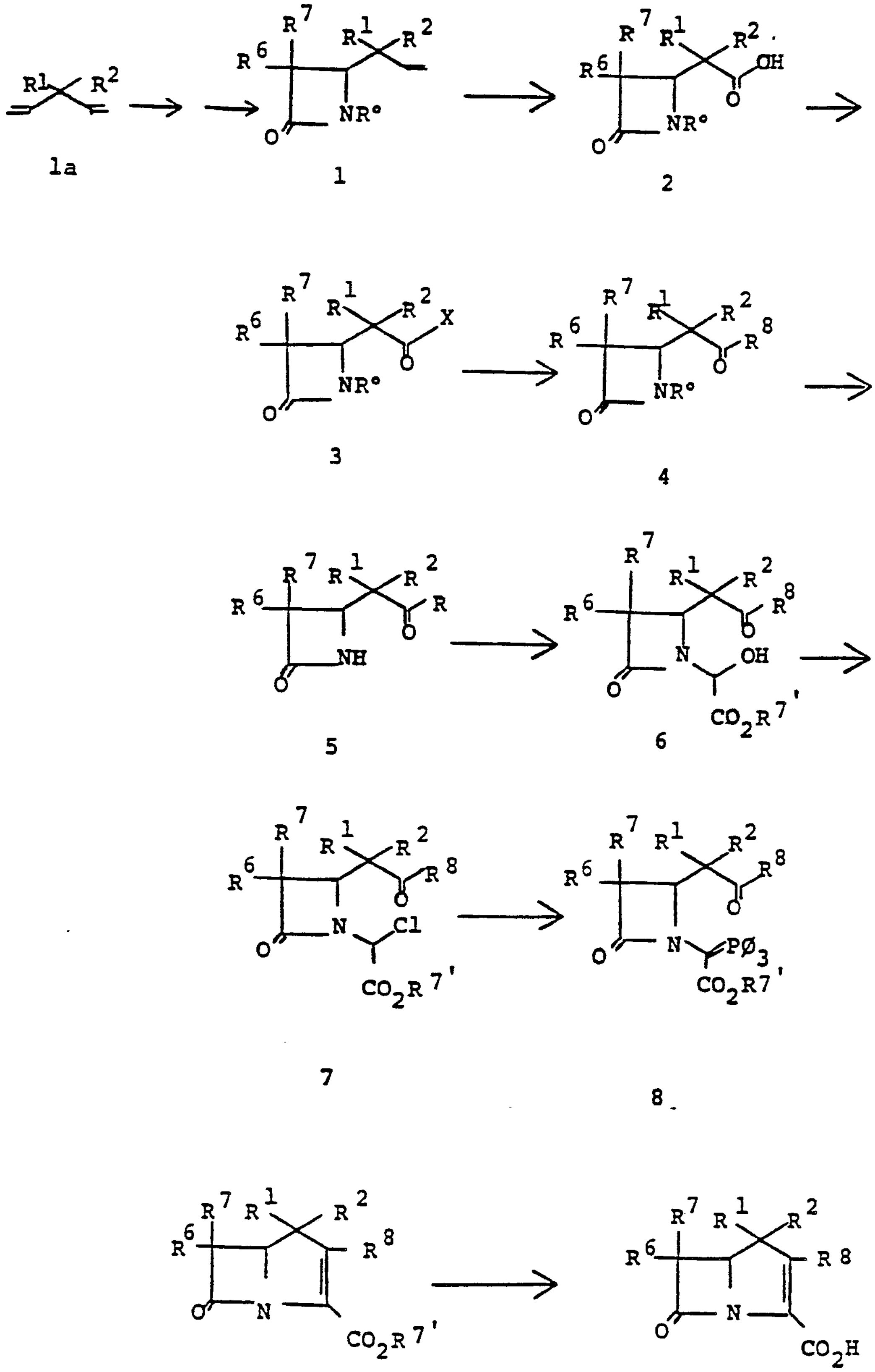

1a 1 2

3 4

5 6

7 8

9 I

In words relative to the above reaction diagram, a suitably substituted azetidinone 1 is oxidized by treating 1 in a solvent such as methylene-chloride, methanol or chloroform, with an oxidizing agent such as ozone, at a temperature of from −100° to 0°C for from 0.1 to 4 hours, followed by treating the crude product with an oxidizing agent such as m-chloroperchenzoic acid, hydrogen peroxide or peracetic acid, at a temperature of from 0°C to 100°C for from 1 to 100 hours.

The sequence 2→3→4 may be achieved by well-known methods for converting a carboxylic acid to a ketone such as by activating a carboxylic function with dicyclohexylcarbodiimide, ethylchloroformate, 2-fluoropyridine, 1-fluoro-2,4-dinitrobenzene, thionyl chloride or oxalyl chloride, followed by the neucleophilic displacement of the leaving group with a carbon neucleophile such as $R^8_2CuMgX°$, $R^8MgX°$, $LiCuRr^8_2$ or $R^8_2Cd$ to give the desired ketone 4, (X° is bromo or chloro). Or preferably by displacement of the chloride with a thiophenol or 2-mercaptopyridine to give a stable thioester 3 wherein X is phenylthio or pyridylthio which may subsequently be reacted with a Grignard reagent to yield the ketone 4.

Removal of protecting group R° (4→5) is accomplished by acidic aqueous hydrolysis of 4 in a solvent such as methanol, ethanol, tetrahydrofuran, or dioxane, in the presence of an acid such as hydrochloric, sulfuric or acetic at a temperature of from 0 to 100°C for from 2 to 18 hours.

The azetidinone 5 is then reacted with a glyoxyalate ester such as benzyl glyoxylate to give 6. In addition to benzyl, $R^{7'}$ may be any readily removable protecting group or it may be a pharmaceutically acceptable ester moiety.

The reaction 5→6 is conveniently carried out in a solvent such as benzene, toluene or xylene at a temperature of from about 25°C to reflux for from 2 to 10 hours. There is no criticality as to the precise identity of the solvent, provided only that it adequately solubilizes the reactants and be inert or substantially inert to the desired course of reaction. The halogenation reaction 6→7 may be conducted by any of any of a variety of well-known halogenation means. Suitable reagents include: $SOCl_2$, $POCl_3$, oxalyl chloride for example. A preferred means of chlorination involves treating 6 in a solvent such as tetrahydrofuran (THF), ether or $CH_2Cl_2$ with thionylchloride in the presence of 1 to 2 equivalents (relative to the thionylchloride) of a base such as pyridine, triethylamine or quinoline. Typically, the reaction is conducted at a temperature of from −30 to 25°C for from 0.5 to 1 hour. The resulting 7 is isolated, if desired, by conventional procedures for later reaction, 7→8. The intermediate 8 is prepared by treating 7 in a solvent such as dimethylformamide (DMF), dimethylsulfoxide (DMSO), THF, or dimethoxyethane (DME) with 1 to 1.5 equivalents of a phosphine such as triphenylphosphine, tributylphosphine, triethylphosphine or tris-(2-cyanoethyl)phosphine. Typically the reaction is conducted under a nitrogen atmosphere at a temperature of from −20° to 25°C., for from 0.5 to 2 hours.

Typically, the closure step 8→9 is conducted by heating 8 from 100—160°C in a solvent such as benzene, toluene, dioxane, xylene, or DMF. The carboxyl deblocking step 9→(I) may be achieved by a number of well-known procedures such as hydrolysis, hydrogenation, or photolysis of a suitable R′ group. Suitable hydrogenation catalysts for deblocking include the platinum metals and their oxides such as palladium on carbon; suitable solvents for the hydrogenation include e.g. methanol, dioxane/$H_2O$ and ethanol/$H_2O$, in the presence of hydrogen at a pressure of from about 1 to 50 bar; the hydrogenation is typically conducted for from 5 min. to 4 hours at a temperature of about 25°C in the optional presence of a mild base such as sodium bicarbonate.

The above-mentioned glyoxalate esters used to react with 5 can be prepared by oxidation of the corresponding tartaric acid diesters with oxidants such as periodic acid or lead tetracetate in a solvent such as THF, benzene, methylene chloride at −20 to 25° for 1/2 to 4 hours. The tartarate esters are prepared from dilithio tartarate or disodio tartarate by reaction with $R^{7'}X$ wherein X is chloro, bromo or iodo and R′ is as defined above in a solvent such as DMF or DMSO at 25° to 70°C. for from 4 to 48 hours. As noted above, $R^{7'}$ may be a pharmaceutically acceptable ester moiety which is not subjected to the removal step 9→I.

In the generic description of the present invention (I, above), when the 2-substituent is —$R^8$ rather than —$SR^8$, the substituent $R^8$ is preferably selected from the group consisting of hydrogen; substituted and unsubstituted: straight and branched loweralkyl having from 1 to 10 carbon atoms; spirocycloalkyl and cycloalkyl having from 3 to 6 carbon atoms; cycloalkylalkyl wherein the cycloalkyl moiety comprises 10 carbon atoms; alkylcycloalkyl wherein the alkyl moiety comprises 1 to 6 carbon atoms and the cycloalkyl moiety comprises 3 to 6 carbon atoms; phenyl; aralkyl such as benzyl and phenethyl; heterocyclyl (saturated and unsaturated) comprising mono- and bicyclic structures having from 5 to 10 ring atoms wherein one or more of the hetero atoms is selected from oxygen, nitrogen or sulphur, such as thiophene, imidazolyl, tetrazolyl and furyl; heterocyclylalkyl which comprises the immediately preceding heterocyclyl moieties and the alkyl moiety comprises from 1 to 10 carbon atoms; the substituent (or substituents) relative to the above-named radicals is selected from the group consisting of amino, hydroxyl, cyano, carboxyl, nitro, chloro, bromo, fluoro, lower alkoxy having from 1 to 6 carbon atoms, mercapto, perhalo-loweralkyl such as trifluoromethyl, loweralkylthio, guanidino, amidino, sulfamoyl, and N-substituted: sulfamoyl, amidino and guanidino wherein the N-substituent is loweralkyl having from 1 to 6 carbon atoms or aryl having 6—10 carbon atoms.

The preferred esters used as protecting groups are those where $R^{3'}$ is benzyl, p-nitrobenzyl, o-nitrobenzyl, t-butyl, bromo-t-butyl, t-butyldimethylsilyl, trimethylsilyl, trichloroethyl; or $R^{3'}$ represents pharmaceutically acceptable ester moieties such as pivaloyloxymethyl, allyl, methallyl, (2-methylthio)-ethyl, 3-methyl-2-butenyl, p-t-butylbenzyl, 5-indanyl, 3-phthalidyl.

The compounds made available by the present invention are valuable antibiotics active against various gram-positive and gram-negative bacteria and, accordingly, find utility in human and veterinary medicine. Such sensitive bacteria representatively include: *Staphylococcus aureus*, *Escherichia coli*, *Klebsiella pneumoniae*, *Serratia*, *Salmonella typhosa*, *Pseudomonas* and *Bacterium proteus*. The resulting compounds may further be utilized as additives to animal feed, for preserving foodstuffs, and as disinfectants. For example, they may be employed in aqueous compositions in concentrations ranging from 0.1 to 100 parts of antibiotic per million parts of solution in order to destroy and inhibit the growth of harmful bacteria on medical and dental equipment and as bactericides in industrial applications, for example, in waterbased paints and in the white water of paper mills to inhibit the growth of harmful bacteria.

These antibiotics may be used alone or in combination as an active ingredient in any one of a variety of pharmaceutical preparations. These antibiotics and their corresponding salts may be employed in capsule form or as tablets, powders or liquid solutions or as suspensions of elixirs. They may be administered orally, intravenously or intramuscularly.

Tablets and capsules for oral administration may be in unit dose presentation form, and may contain conventional excipients such as binding agents, for example, syrup, acacia, gelatin, sorbitol, tragacanth, or polyvinylpyrrolidone; fillers for example, lactose, sugar, maize-starch, calcium phosphate, sorbitol or glycine; lubricants, for example, magnesium stearate, talc, polyethylene glycol, silica; disintegrants, for example, potato starch or acceptable wetting agents such as sodium lauryl sulphate. The tablets may be coated according to methods well known in the art. Oral liquid preparations may be in the form of aqueous or oily suspension, solution, emulsions, or syrups; or may be presented as a dry product, for reconstitution with water or other suitable vehicles before use. Such liquid preparations may contain conventional additives such as suspending agents, for example, sorbitol, syrup, methyl cellulose, glucose/sugar syrup, gelatin, hydroxyethylcellulose, carboxymethyl cellulose, aluminum stearate gel or hydrogenated edible oils, for example almond oil, fractionated coconut oil, oily esters, propylene glycol, or ethyl alcohol; preservatives, for example methyl or propyl *p*-hydroxybenzoates or sorbic acid.

Compositions for injection may be presented in unit dose form in ampules, or in multidose container. The compositions may take such forms as suspensions, solutions, or emulsions in oily or aqueous vehicles, and may contain formulatory agents such as suspending, stabilizing and/or dispersing agents. Alternatively, the active ingredient may be in powder form for reconstitution with a suitable vehicle, e.g., sterile, pyrogen-free water, before use.

The compositions may also be prepared in suitable forms for absorption through the mucous membranes of the nose and throat or bronchial tissues and may conveniently take the form of e.g. powder or liquid sprays or inhalants, lozenges, and throat paints. For medication of the eyes or ears, the preparations may be presented as individual capsules, in liquid or semi-solid form, or may be used as drops, for example. Topical applications may be formulated in hydrophobic or hydrophilic bases as ointments, creams, or lotions.

Also, in addition to a carrier, the instant compositions may include other ingredients such as stabilizers, binders, antioxidants, preservatives, lubricators, suspending agents, viscosity agents or flavoring agents. In addition, there may also be included in the compositions other active ingredients to provide a broader spectrum of antibiotic activity.

For veterinary medicine the composition may, for example, be formulated as an intramammary preparation in either long or quick-release bases.

The dosage to be administered depends to a large extent upon the general health and weight of the subject being treated, and the route and frequency of administration — the parenteral route being preferred for generalized infections and the oral route for intestinal infections. In general, a daily oral dosage consists of from about 2 to about 600 mg. of active ingredient per kg. of body weight of the subject in one or more applications per day. A preferred daily dosage for adult humans lies in the range of from about 15 to 150 mg. of active ingredient per kg. of body weight.

The instant compositions may be administered in several unit dosage forms as, for example, in solid or liquid orally ingestible dosage form. The compositions per unit dosage, whether liquid or solid may contain from 0.1% to 99% of active material, the preferred range being from about 10—60%. The composition will generally contain from about 15 mg. to about 1500 mg. of the active ingredient; however, in general, it is preferably to employ a dosage amount in the range of from about 100 mg. to 1000 mg. In parenteral administration the unit dosage is usually the pure compound in a slightly acidified sterile water solution or in the form of a soluble powder intended for solution.

Especially preferred pharmaceutically acceptable salts and esters involving the carboxyl group of compounds of the present invention (I) are disclosed and claimed in co-pending U.S.—A—4,181,733 which application is directed, *inter alia*, to pharmaceutically acceptable salts and esters of the carboxyl group of thienamycin. It is precisely these salts and esters which are preferred in the present invention and they are prepared in a manner analogous to that disclosed in U.S.—A—4,181,733 which is incorporated herein by reference. Thus, especially preferred salts include sodium, potassium, ammonium, and the like; and especially preferred esters include e.g. pivaloxymethyl, p-t-butylbenzyl, 5-indanyl, 3-phthalidyl and 3-methyl-2-butenyl. One should note that when, in the total synthesis outlined above, $R^{3'}$ is a pharmaceutically acceptable ester moiety, there is no need for the final deblocking step if it is desired to

have the final product I in the form of a pharmaceutically acceptable ester.

Especially preferred embodiments of the present invention are those, as defined above, except that any unsubstituted amino group borne on radical $R^8$ of Structure I is derivatized according to the teachings of BE—A—848,545 (issued 5—20—77); the resulting amino group being represented thusly (partial structure):

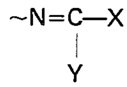

wherein X and Y are defined by the publication; species wherein X is H or lower alkyl and Y is $NH_2$ are especially preferred.

The following examples, illustrate but do not limit the product, process, compositional or method of treatment aspects of the present invention. All reaction temperatures are in °C.

Example 1
Preparation of 3,3-Dimethyl-1,4-pentadiene

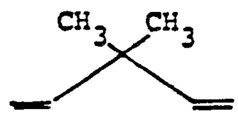

1

*Procedure a*

β,β-Dimethylglutaric acid (obtained from Aldrich Chemical Company) (one mole), is refluxed for 2 hours with thionyl chloride (68% excess). After removal of excess thionyl chloride, absolute ethanol (109% excess) is added slowly. The mixture is refluxed for 3 hours then distilled to collect the product, diethyl β,β-dimethylglutarate (98% yield).

To a suspension of lithium alumium hydride (24 g) in ether (860 ml) is added dropwise with rapid stirring a solution of diethyl β,β-dimethylglutarate (124 g in 250 ml ether). The mixture is refluxed for 6 huors, then cooled to room temperature. Water (25 ml) is added slowly. The mixture is then titrated with 10% NaOH until a clear organic layer is obtained. The organic layer is separated, dried over anhydrous sodium sulfate then evaporated *in vacuo* to give the resulting diol as an oil (90% yield), b.p.95° at 1.0 mm. The 3,3-dimethyl-1.5-pentanediol (0.5 mole) is treated with thionyl chloride (1.05 mole) at reflux for 3 hours. After removal of excess thionyl chloride *in vacuo*, the 3,3-dimethyl-1,5-dichloropentane is obtained (90% yield).

3,3-Dimethyl-1.5-dichloropentane (41 g) is added dropwise at 170°C to a mixture of 48 g of sodium hydroxide and 40 g of polyethylene glycol tetramer and the mixture is distilled to give 3,3-dimehtyl-1,4-pentadiene (66%).

*Procedure b*

At −40°C, 1,3-dichloro-3-methylbutane (50 g) is mixed with alumium chloride (5 g). The ethylene is bubbled through the mixture for 4 hours. The mixture is allowed to warm to room temperature and hydrolyzed with water. The mixture is extracted with ethyl acetate to give 3,3-dimethyl-1,5-dichloropentane.

A mixture of 0.5 mole of 3,3-dimethyl-1,5-dichloropentane, 2-methylquinoline (2 moles), and sodium iodide (0.1 mole) is refluxed in a flask equipped with a Vigreaux column at the top of which is a condenser and take-off. The diolefin 1 is collected during 8 hrs reaction. The product is dried over anhydrous sodium sulfate.

Example 2
Preparation of 3-methyl-1,4-pentadiene

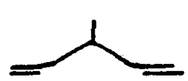

2

Following the procedure of Example 1(a), but replacing β,β-dimethylglutaric acid with an equivalent amount of β-methylglutaric acid, 3-methyl-1,4-penadiene is obtained.

Example 3
Preparation of 4-(1,1-dimethyl-pro-2-enyl)azetidin-2-one

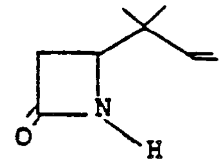

3

In a sealed tube, 3,3-dimethyl-1,4-pentadiene (9.6 g) and chlorosulfonyl isocyanate (14.2 g) are allowed to stand at room temperature for 6 days. The resulting mixture is diluted with methylene chloride and added slowly to a stirred aqeous solution which contains 20g of $Na_2SO_3$ and 50g of $K_2HPO_4$ at 0—5-C for 30 min. The organic layer is separated and dried over $Mg_2SO_4$. After evaporation, the crude product is chromatographed on silica gel GF eluting with EtOAc to give 3.

Example 4
Preparation of 4-(1-methyl-pro-2-enyl)azetidin-2-one

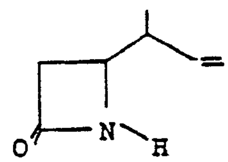

4

Following the procedure of Example 3, but replacing 3,3-dimethyl-1,4-pentadiene with 3-methyl-1,4-pentadiene, the title compound 4 is obtained.

Example 5
Preparation of 5

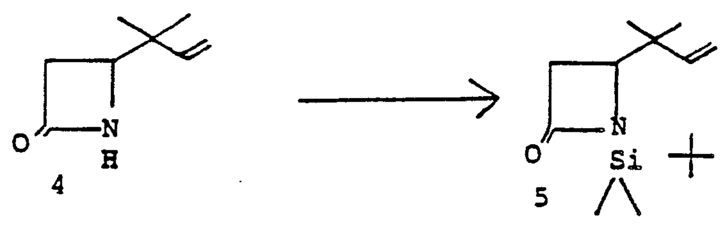

t-Butyldimethylchlorosilane (7.51 g) is added in one portion to an ice-cold, stirred solution of 4-(1,1-dimethyl-prop-2-ene)-azetidin-2-one 6.54 g) and triethylamine (5.04 g) in anhydrous dimethylformamide (100 ml). The reaction mixture is stirred at 0—5°C for 1 hour and then allowed to warm to room temperature. Most of the solvent is removed under vacuum to give a residue which is partitioned between diethyl ether (250 ml) and water. The ethereal phase is washed with 2.5N hydrochloric acid (50 ml), water (3×50 ml), and brine, dried with magnesium sulfate, filtered and evaporated under vacuum to provide a crude product which is purified by chromatography on silica gel (20% ether in petroleum ether) to yield 5.

Example 6
Preparation of 6

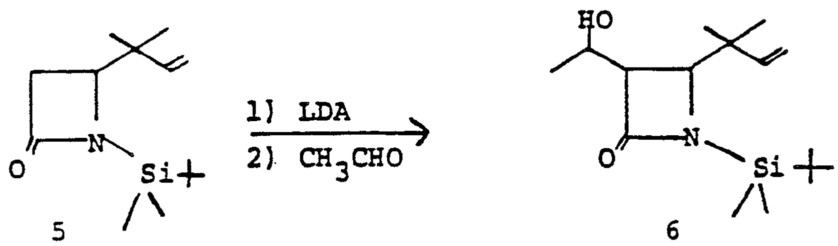


5 6

n-Butyllithium in hexane (26.25 mmol) is added slowly by syringe to a solution of diisopropylamine (26.25 mmol) in anhydrous tetrahydrofuran (100 ml) at −78°C. The resulting solution is stirred for 15 min. prior to the addition of a solution of 5 (25.0 mmol) in anhydrous tetrahydrofuran (25 ml). After stirring for 15 min. at −78°C, acetaldehyde (75 mmol) is added by syringe and the resulting solution is stirred at −78°C for 5 min. Saturated aqueous ammonium chloride solution (15 ml) is added by syringe and the reaction mixture is allowed to warm to room temperature, then diluted with ether (250 ml) and washed with 2.5N hydrochloric acid solution (2 × 50 ml), water (100 ml) and brine and dried over magnesium sulfate. Solvents are removed *in vacuo* and the residue is chromatographed on silica gel (1:1, ether: patroleum ether) to give the expected product 6.

Example 7
Preparation of 7

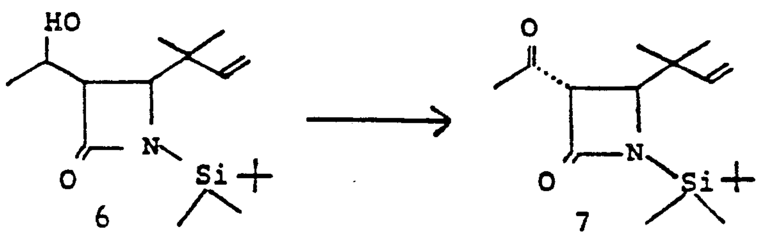

6 7

A. Trifluoroacetic anhydride (7.5 mmol) is added dropwise by syringe to a solution of dimethylsulfoxide (10 mmol) in anhydrous methylene chloride (15 ml) at −78°C. The resulting mixture is stirred at −78°C for 20 min. A solution of 6 (5.0 mmol) in methylene chloride (15 ml) is added by syringe and the cooling bath is removed. After an additional 1 hr., the reaction mixture is diluted with methylene chloride (100 ml), washed with water (50 ml) and brine and dried over magnesium sulfate. Removal of solvents *in vacuo* yields crude product which is chromatographed on silica gel (2:1, petroleum ether: ether) to yield 7.

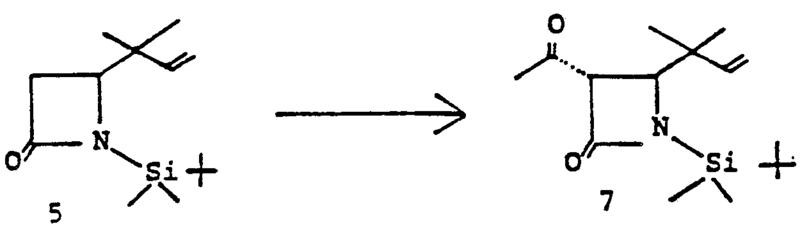

5 7

B. n-Butyllithium in hexane (4.10 mmol) is added by syringe to a solution of diisopropylamine (4.10 mmol) in anhydrous tetrahydrofuran (16 ml) at −78°C. The resulting solution is stirred at −78°C for 15 min. prior to the addition of a solution of 1-(t-butyldimethylsilyl)-4-(1,1-dimethyl-prop-2-enyl)-azetidin-2-one 5 (2.0 mmol) in anhydrous tetrahydrofuran (2 ml). After an additional 15 min. at −78°C, the reaction mixture is added *via* a Teflon tube to a mixture of N-acetylimidazole (4.1 mmol) in anhydrous tetrahydrofuran (16 ml) at −78°C. The resulting yellow reaction mixture is stirred at −78°C for 15 min., then quenched by addition of saturated aqueous ammonium chloride solution (10 ml). The reaction mixture is diluted with ether (100 ml) and washed with 2.5N hydrochloric acid solution (25 ml) water (25 ml) and brine. The organic phase is dried over magnesium sulfate and concentrated *in vacuo* to yield a crude product. This material is chromatographed on silica gel (2:1 petroleum ether: ether) to yield 7.

Example 8
Preparation of 6

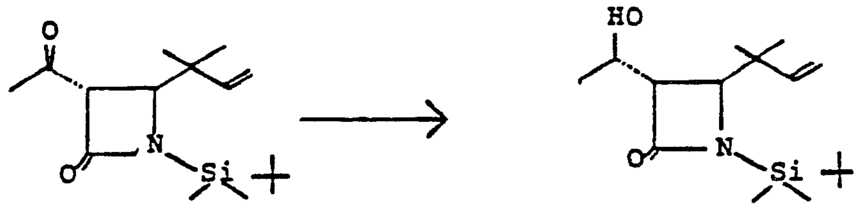

7 6

K-Selectride (potassium tri-(sec)-butylborohydride) in tetrahydrofuran (4.8 mmol) is added by syringe to a mixture of potassium iodide (2.0 mmol) and 7 (2.0 mmol) in anhydrous ether (20 ml) at room temperature. The resulting mixture is stirred at room temperature for 2.5 hours, then quenched by addition of glacial acetic acid (9.6 mmol). The resulting mixture is diluted with ethylacetate (100 ml) and filtered through celite. Removal of solvents *in vacuo* gives crude product which is chromatographed on silica gel (1:1 ether: petroleum ether) to yield 1.90 g (95%) of 6.

Example 9

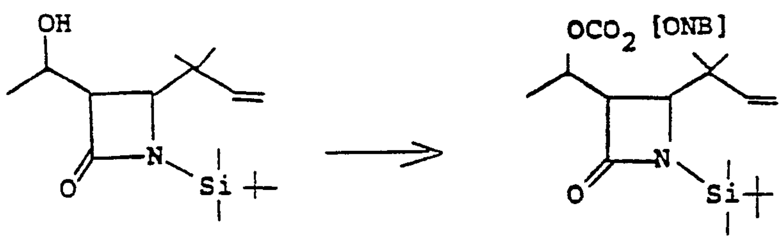

6 7a

Under anhydrous conditions at 0°C a solution of 6 (3.50 g) in 60 ml methylene chloride is treated with 4-dimethylaminopyridine (3.32 g) and o-nitrobenzylchloroformate (5.88 g). The mixture is allowed to warm to room temperature and stirred for 1 hr. The resulting mixture is washed with 0.1N HCl, water, brine and water. The organic layer is separated, dried over $Na_2SO_4$ and allowdd to evaporate *in vacuo* to give crude products. The crude products, dissolved in 20 ml ether and chilled at −5°C, give the o-nitrobenzyl alcohol (0.5 g) which is separated by filtration. Purification by HPLC (silica gel) eluting with 40% ethylacetate/cyclohexane to gives 7a.

Example 10
Preparation of 8

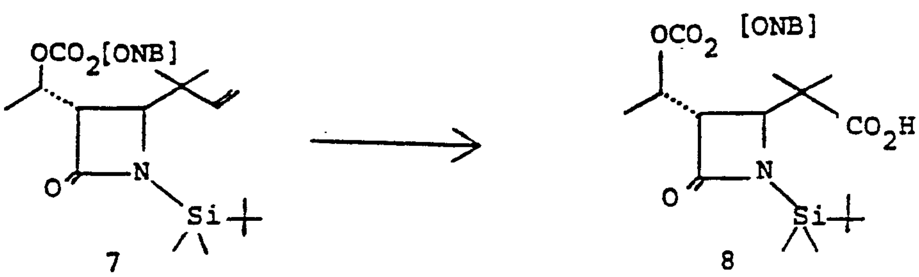

7 8

A solution of 7 (3.0 mmol) in dry methylene chloride (30 ml) is cooled to −78°C (dry ice-acetone) and a stream of ozone is bubbled through until the reaction mixture becomes blue. The ozone flow is then stopped and the reaction is purged by bubbling through nitrogen until the blue color disappears. Solid *m*-chloroperbenzoic acid (3.0 mmol) is added and the cold bath is removed. When the reaction mixture reaches room temperature, the flask is fitted with a reflux condenser and the mixture is heated at reflux for three days. Removal of solvents *in vacuo* gives crude product which is chromatographed on silica gel (2% glacial acetic acid in methylene chloride to 8.

Example 10a
Preparation of 8a

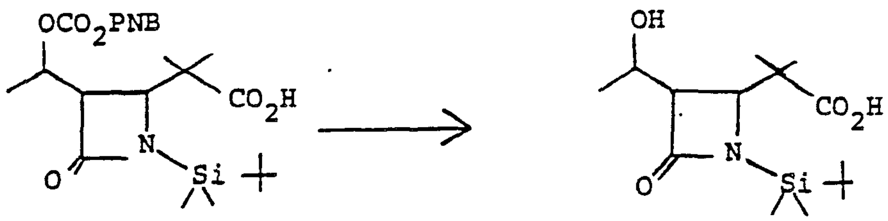

8 8a

The acid 8 (1.0 mmol) is hydrogenated in 30 ml ethyl acetate under 1 atm $H_2$ in the presence of 0.1 mmol of 10% Pd/C at room temperature for 30 min. The mixture is filtered from catalyst. The filtrate is evaporated *in vacuo* to give 8a.

Example 11
Preparation of 9

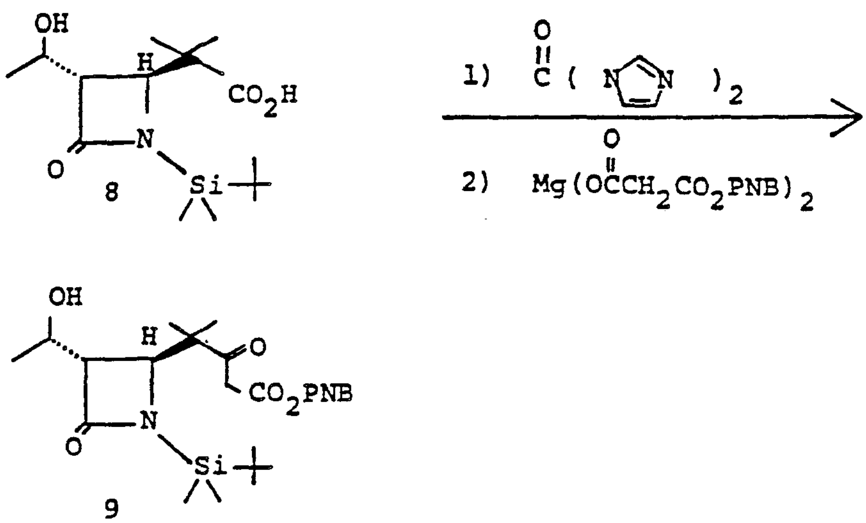


8

9

1,1-Carbonyldiimidazole (1.10 mmol) is added in one portion to a solution of 8 (1.0 mmol) in anhydrous tetrahydrofuran (5 ml) at room temperature. The resulting solution is stirred at room temperature for 6 hours. In a second flask, magnesium ethoxide (5 mmol) is added in one portion to a solution of the mono-p-nitrobenzyl ester of malonic acid (10 mmol) in anhydrous tetrahydrofuran (25 ml). The resulting mixture is stirred at room temperature for 1 hr., then the tetrahydrofuran is removed at the pump and the residue is triturated with ether to yield the magnesium salt. This magnesium salt is then added to the first reaction flask and the resulting mixture is stirred at room temperature for 18 hrs. The reaction mixture is then poured into 50 ml of ether, washed with 0.5N hydrochloric acid solution (20 ml), water (20 ml), saturated aqueous sodium bicarbonate solution (20 ml), brine and dried over magnesium sulfate. Removal of solvents *in vacuo* gives crude product which is chromatographed on silica gel (ether) to yield 9.

Example 12
Preparation of 10

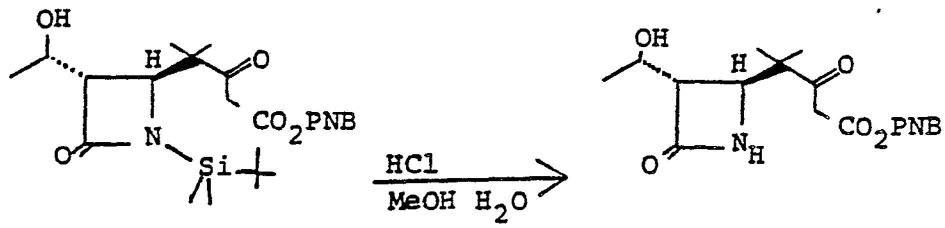


9 10

A solution of 9 (1.0 mmol) in 20 ml of 9:1 (v/v) methanol-water is cooled to 0°C. Concentrated hydrochloric acid (0.34 ml) is added and the resulting solution is stirred at 0°C for 15 min., then allowed to warm to room temperature. After 2.5 hrs., at room temperature the reaction mixture is diluted with ethyl acetate (25 ml), washed with water (10 ml) and brine, dried over magnesium sulfate and conccentrated *in vacuo* to yield 10.

Example 13
Preparation of 11

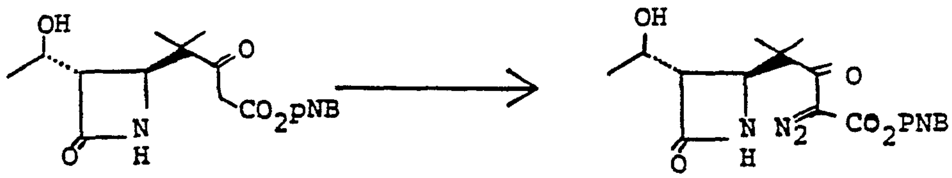

10 11

Triethylamine (263 mg) is added by syringe to a mixture of 10 (253 mg) and p-carboxybenzeensulfonyl-azide (196 mg) in dry acetonitrile (6 ml) at 0°C. When addition is complete the cooling bath is removed and the reaction mixture is stirred at room temperature for 1 hour. The mixture is then diluted with ethyl acetate (50 ml) and filtered. The filtrate is concentrated *in vacuo* and the residue is chromatographed on a short silica gel column (ethyl acetate) to yield 11.

Example 14
Preparation of 12

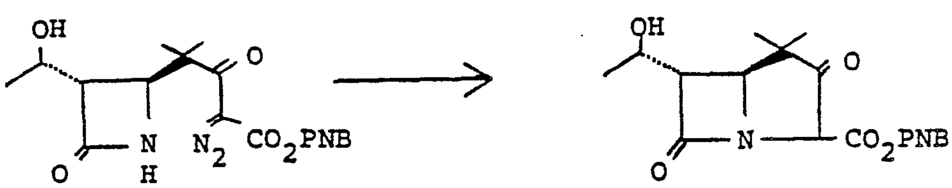

11 12

A suspension of 11 (56.4 mg) and rhodium (II) acetate (0.1 mg) in dry benzene (3 ml) is deoxygenated by bubbling through nitrogen for 10 minutes. The mixture is then heated to 78°C for 1 hour. During heating the solid starting material gradually goes into solution. The mixture is then cooled, filtered to remove the catalyst, and the filtrate is concentrated *in vacuo* to yield 12.

Example 15
Preparation of *p*-Nitrobenzyloxycarbonylaminoethanethiol

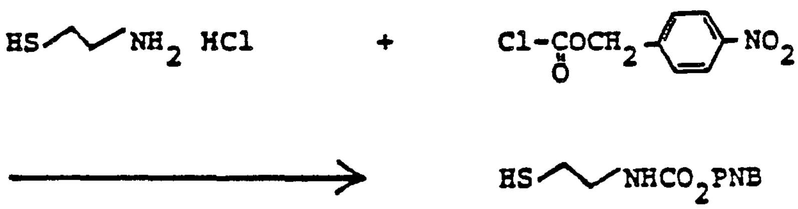

To 600 ml diethyl ether ($Et_2O$) — 75 ml $H_2O$ in an ice bath with stirring is added 3.2 g cysteamine hydrochloride (mw = 114; 28.1 mmole). A solution of 7.14 g $NaHCO_3$ (mw = 84; 85 mmole) in 75 ml $H_2O$ is added. The ice bath is removed, and at room temperature a solution of 6.75 g *p*-nitrobenzylchloroformate (mw = 216; 31.3 mmole) in 270 ml $Et_2O$ is added dropwise over a period of one hour. After 10 additional minutes, the layers are separated. The ether layer is extracted with 150 ml 0.25 N HCl, and then with 200 ml brine. Each aqueous layer is then backwashed successively with 100 ml $Et_2O$. The combined $Et_2O$ layers are dried over anhydrous $MgSO_4$, filtered, and concentrated under a $N_2$ stream. The crystalline residue is slurried in a small amount of ether, filtered, and the pale yellow crystals are dried under high vacuum to give 4.7 g. *p*-nitrobenzyloxycarbonylaminoehanethiol (65% yield). NMR ($CDCl_3$): 8.18 (*d*, J=8Hz, aromatic protons ortho to nitro), 7.47 (*d*, J=8Hz, aromatic protons meta to nitro), 5.27 (—N$\underline{H}$—), 5.20 (*s*, —C$\underline{H_2}$—NH—), 2.67 (*m*, —C$\underline{H_2}$—SH), 1.35 (*t*, J=8.5Hz, —S$\underline{H}$) in ppm downfield from TMS. IR ($CHCl_3$) solution: carbonyl 1725 $cm^{-1}$. M.S.: molecular ion-256 (M—47) at 209, (M—136) at 120, $^{+}CH_2\phi pNO_2$ at 1.36.

Example 15a

Following the procedure of Example 15, N-p-nitrobenzyloxycarbonylaminocyclopropylthio is obtained when an equivalent amount of aminocyclopropylthiol hydrochloride is substituted for the cysteamine hydrochloride of Example 15.

Example 16
Preparation of 13

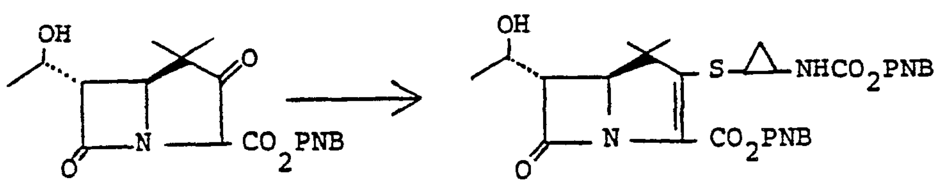

12 13

The starting material 12 (51 mg) is dissolved in acetonitrile (3 ml) and the resulting solution is cooled to 0°C. Diisopropylethylamine (22 mg) is added by syringe and the resulting solution is stirred at 0°C for 1 minute prior to the addition of a solution of freshly recrystallized p-toluene sulfonic anhydride (51 mg) in dry acetonitrile (1 ml). The resulting solution is stirred at 0°C for 1 hour to provide p-nitrobenzyl 4,4-dimethyl 3-(p-toluene-sulfonyloxy)-6-[hydroxyethyl]-1-azabicyclo[3.2.0]hept-2-en-7-one-2-carboxylate, then cooled to −25°C. Diisopropylethylamine (80.5 mg) is added by syringe followed shortly thereafter by a solution of N-p-nitrobenzyloxycarbonylaminocyclopropylthiol (40 mg) in 1 ml of dry actonitrile. The reaction mixture is then stored in a refrigerator for 70 hr. The mixture is diluted with 25 ml of ethyl acetate washed with brine and dried over magnesium sulfate. Solvents are removed *in vacuo* to yield crude product which is chromatographed on a silica gel plate to yield 13.

Example 17

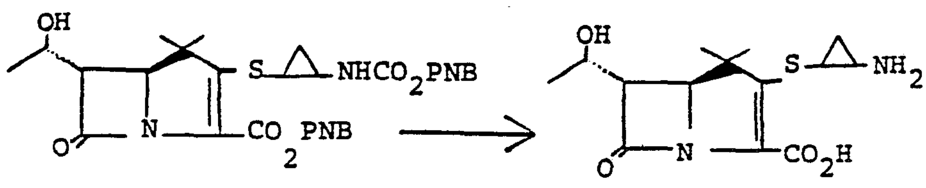

13 14

A mixture of 13 (10 mg) and 10% Pd/C-Bolhofer type in tetrahydrofuran (2 ml), 0.1M dipotassium

hydrogen phosphate solution (1.4 ml) and 2-propanol (0.2 ml) is hydrogenated at 40 psi on the Parr shaker for 30 minutes. The mixture is then filtered and the catalyst is washed with water. The combined filtrate and washings are extracted with ethyl acetate-ethyl ether then concentrated to ~3 ml and lyophilized to give 14.

Example 18

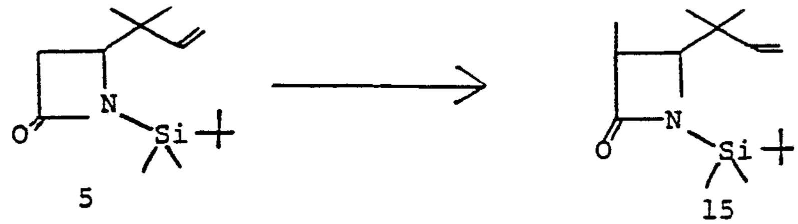

5

15

THF, 20 ml, is placed under $N_2$, treated with 1.54 ml diisoproylamine and cooled to −78°C. A solution of n-butyl lithium 1.97M in hexane (5.6 ml) is added dropwise over 5 min. The reaction mixture is stirred at −78°C for 10 min. and then treated with 5 (2.14 g) in 15 ml THF which is added dropwise over 5 min. After another 10 min. hexamethylphosphoramide (1.97 ml) is added. The mixture is stirred another 10 min., then treated with 2 ml of methyl iodide. The reaction mixture is stirred at −78°C for 15 min. and allowed to warm to 25°C and stirred for 15 min. The reaction mixture is diluted with EtOAc, washed once with pH7 phosphate buffer then dried and evaporated. The residue is chromatographed on silica gel using 25% EtOAc/$C_6H_6$ as eluant to give 15.

Example 19

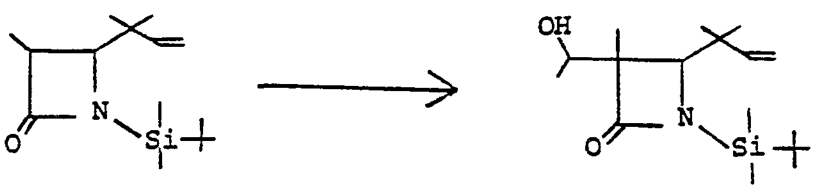

15

16

To a solution of 1.1 equivalents of freshly prepared lithium diisopropylamide in anhydrous tetrahydrofuran under a nitrogen atmosphere at −78°C is added a solution of 15 in anhydrous tetrahydrofuran which has been cooled to −78°C. After two minutes, the resulting lithium enolate is treated with 3 equivalents of acetaldehyde. The solution is stirred for 30 minutes at −78° and then poured into water. The aqueous phase is saturated with sodium chloride and extracted with ethyl acetate. The combined ethyl acetate solutions are dried over magnesium sulfate and filtered. The filtrate is evaporated under reduced pressure to give the crude product. Purification by chromatography on silica gel using ethyl acetate/benzene gives 16.

Example 20

Following the procedure of the foregoing Examples, the following substituted azetidinones useful in the preparation of the compound of the present invention are obtained when the suggested substitution of reagents is made.

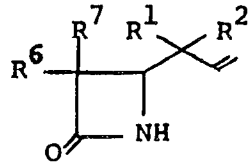

| | $R^1$ | $R^2$ | $R^6$ | $R^7$ |
|---|---|---|---|---|
| 1.) | $CH_3$ | $CH_3$ | H | 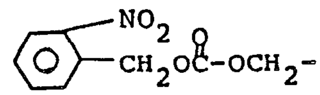 |
| 2.) | $CH_3$ | Et | H | $CH_3$ |
| 3.) | $CH_3$ | 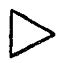 | H | 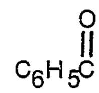 |
| 4.) | $CH_3$ | 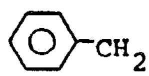 | H | 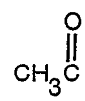 |
| 5.) | $CH_3$ | 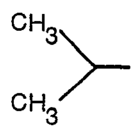 | H | 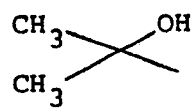 |
| 6.) | $CH_3$ | Ph | H | 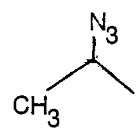 |
| 7.) | $CH_3$ | $CH_3CH_2CH_2$ | $CH_3$ | 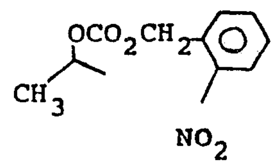 |
| 8.) | Et | Et | $CH_3CH_2$ | 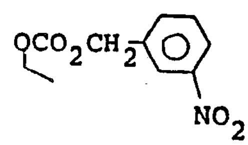 |
| 9.) | Et | $CH_3$ | H | 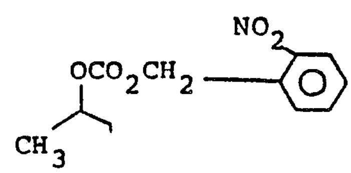 |
| 10.) | Et | Et | $CH_3$ | 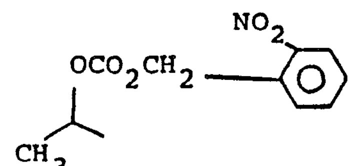 |
| 11.) | 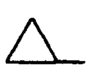 | $CH_3$ | $CH_3$ | 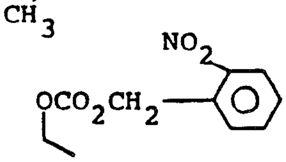 |

| | $R^1$ | $R^2$ | $R^6$ | $R^7$ |
|---|---|---|---|---|
| 12.) | $CH_3$ | $CH_3$ | H | 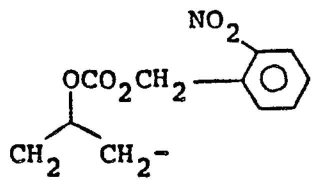 |
| 13.) | $CH_3$ | Et | H | 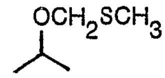 |
| 14.) | 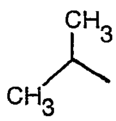 | $CH_3$ | H | Ph– |
| 15.) | $CH_3$ | $CH_3$ | H | 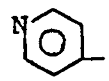 |
| 16.) | $CH_3$ | Et | H | 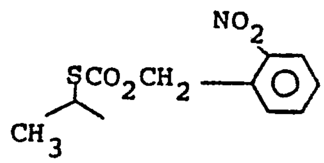 |
| 17.) | $R^1 + R^2$ = spirocyclopropyl | | H | 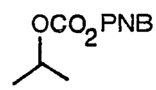 |
| 18.) | $CH_2CH_2Br$ | $CH_3$ | H | 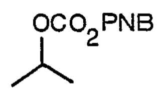 |

Example 21

Following the foregoing text and Examples, the following species (I) are obtained when the ß-lactams of Example 20 are carried through the standard procedure (Examples 10—14) to the corresponding bicyclic keto ester, followed by establishment of the thio side chain of choice and deblocking (Examples 16 and 17).

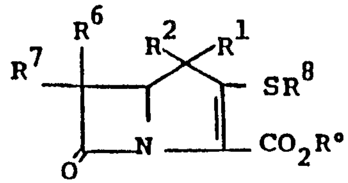

I

| Compound | $R^1$ | $R^2$ | $R^6$ | $R^7$ | $R^o$ | $R^8$ |
|---|---|---|---|---|---|---|
| 1.) | $CH_3$ | $-CH_3$ | H | $HOCH_2$ | $Na^+$ | 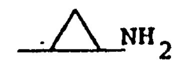 |
| 2.) | Et | $-CH_3$ | H | $-CH_3$ | $Na^+$ | 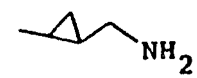 |
| 3.) | $CH_3CH_2CH_2$ | $-CH_3$ | H | 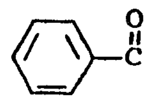 | H | 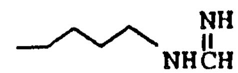 |
| 4,) | 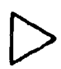 | $-CH_3$ | H | $(CH_3)_2C(OH)-$ | $CH_2O\overset{O}{\overset{\|}{C}}CMI_3$ | $-CH_3$ |
| 5.) | $PhCH_2$ | $CH_2$ | H | $CH_3CH(N_3)-$ | H | 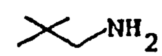 |
| 6.) | Ph | $CH_3$ | $-CH_3$ | $CH_3CH(OH)-$ | H | 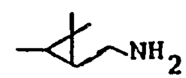 |
| 7.) | $CH_3$ | $CH_3$ | $CH_3CH_2-$ | $HOCH_2-$ | 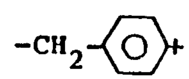 | $-C_2H_5$ |
| 8.) | 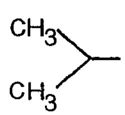 | $CH_3$ | $CH_3$ | $CH_3\overset{O}{\overset{\|}{C}}-$ | H | $-CF_2CH_2NH_2$ |

| Compound | $R^1$ | $R^2$ | $R^6$ | $R^7$ | $R^o$ | $R^8$ |
|---|---|---|---|---|---|---|
| 9.) | $C_4H_9$ | $-CH_2CH_2NH_2$ | H | $\phi CH_2CH(OH)-$ | H | $-Ph$ |
| 10.) | Et | $CH_3CH_2$ | $CH_3$ | $CH_3CH(OH)-$ | H | 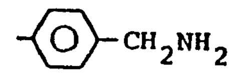 |
| 11.) | $CH_3$ | 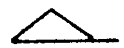 | $CH_3$ | $HOCH_2-$ | $Na^+$ | $CH_3$ |
| 12.) | 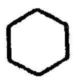 | $CH_3$ | H | $CH_3CH(OH)CH_2-$ | $(C_2H_5)_4N^+$ | $CH_2NH_2$ |
| 13.) | 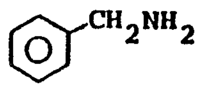 | $CH_3$ | H | $CH_3CH(OCH_2SCH_3)-$ | H | 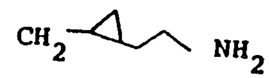 |
| 14.) | Ph | $CH_3$ | H | 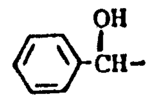 | H | $CH_2CH_2\overset{NH}{\overset{\|}{C}}-NH_2$ |
| 15.) | $CH_3$ | $CH_3CH(CH_3)-$ | H | 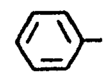 | H | 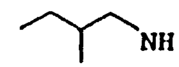 |
| 16.) | $CH_3$ | $CH_3$ | H | 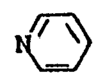 | $Na^+$ | 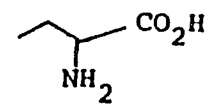 |
| 17.) | Et | $CH_3$ | H | 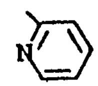 | H | 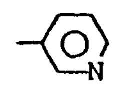 |
| 18.) | $CH_3$ | $CH_3$ | H | $CH_3CH(SH)-$ | $K^+$ | $-CH_3$ |
| 19.) | $R^1 + R^2$ = spirocyclopropyl | | H | $CH_3CH(OH)-$ | Na | $-CH_2CH_2NH\overset{NH}{\overset{\|}{C}}-H$ |

Example A
Preparation of 8

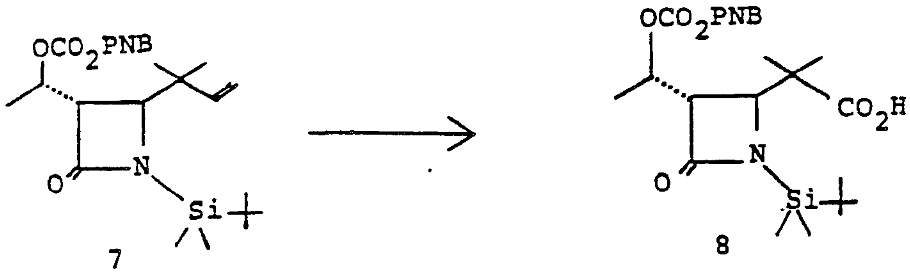

7 8

A solution of 7 (3.0 mmol) in dry methylene chloride (30 ml) is cooled to −78°C (dry ice-acetone) and a stream of ozone is bubbled through until the reaction mixture becomes blue. The ozone flow is then stopped and the reaction is purged by bubbling through nitrogen until the blue color disappears. Solid *m*-chloroperbenzoic acid (3.0 mmol) is added and the cold bath is removed. When the reaction mixture reaches room temperature, the flask is fitted with a reflux condenser and the mixture is heated at reflux for three days. Removal of solvents *in vacuo* gives crude product which is chromatographed on silica gel (2% glacial acetic acid in methylene chloride) to 8.

Example B
Preparation of 11

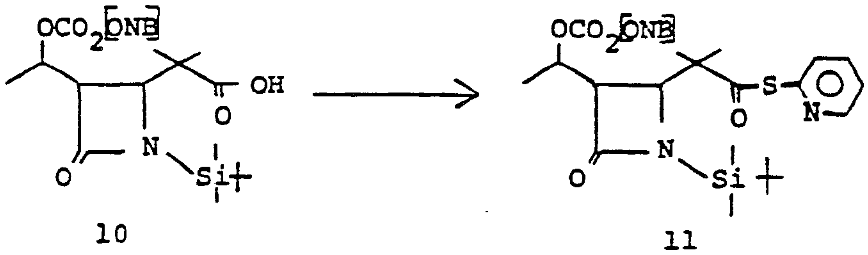

10 11

The azetidinone 10 (0.851 g) is dissolved in 20 ml $CH_2Cl_2$ and cooled to 0°C under $N_2$. Oxalyl chloride (0.8 ml) is added dropwise over 5 min. followed by 1 drop of DMF. The mixture is stirred at 0° for 5 min. and then at 25°C for 15 min. The solvent and excess oxalyl chloride are evaporated under reduced pressure. The residue is the desired acid chloride. The acid chloride is dissolved in 20 ml $CH_2Cl_2$ and cooled to 0°, under $N_2$. Mercaptopyridine (0.4 g) and pyridine (0.8 ml) are added. The reaction mixture is stirred at 0°C for.5 min., then allowed to warm to room temperature. The mixture is diluted with $CH_2Cl_2$ and washed with water, dried over $Na_2SO_4$ and evaporated *in vacuo*. The residue is chromatographed on silica gel using 50% EtOAc/$C_6H_5$ as eluant, to give the thio ester 11.

Example C

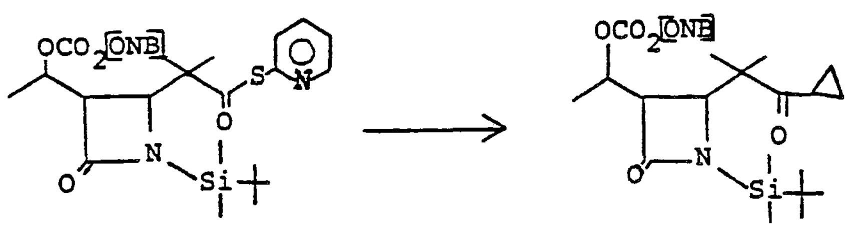

12 13

The thio ester 12 (64 mg) in 2 ml THF is treated with a solution of cyclopropyl magnesium bromide (0.25 M in $Et_2O$, 2.6 ml). The mixture is allowed to stir at 0° for 1 hour. A saturated $NH_4Cl$ aqueous solution is added and the mixture is allowed to stir for 10 minutes. The organic phase is separated. The aqueous phase is extracted twice with $CH_2Cl_2$. The combined organic extracts are dried and evaporated. Preparative t.l.c. of the residue using silica gel and 50% EtOAc/benzene gives the desired product 13.

Example D

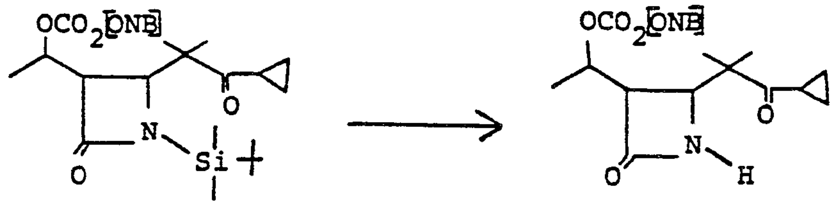

12 13

A solution of 12 (1.0 mmol) in 20 ml of MeOH is cooled to 0°C. Hydrochloric acid (2.5N, 1 eq) is added and the resulting solution is stirred at 0°C for 1 hr., then allowed to warm to room temperature. The mixture is diluted with ethyl acetate (25 ml), washed with water (10 ml) and brine, dried over magnesium sulfate and concentrated *in vacuo* to give 13.

Example E
Preparation of 14

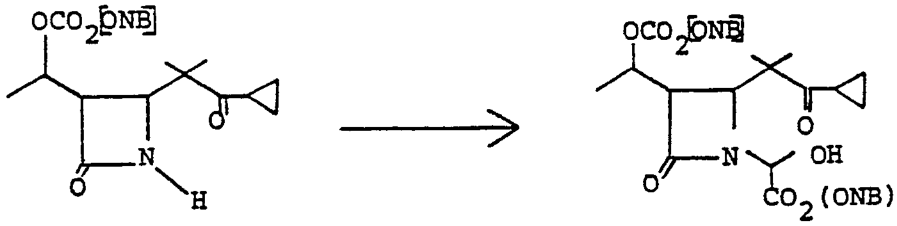

13 14

The azetidinone 13 (0.8 g) and *p*-nitrobenzyl glyoxylate hydrate (1.5 g) are refluxed in benzene (100 ml) for 6 hrs. The reaction apparatus is equipped with a Dean-Stark trap for removal of water azeotropically. The solution is cooled, evaporated, and chromatographed on silica gel eluting with 50% EtOAc/cyclohexane to give product 14.

Example F
Preparation of 16

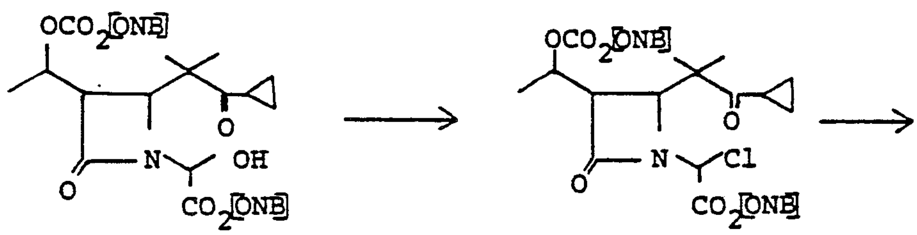

14 15

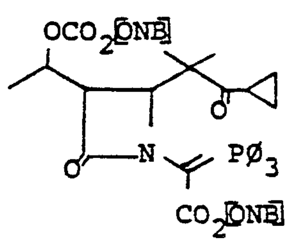

16

Under $N_2$, at −20°C, the carbinal 14 (0.8 g) in 5 ml THF is treated with thionyl chloride (204 mg) and

pyridine (136 mg) for 10 min., then the mixture is allowed to warm to room temperature. The mixture is diluted with 10 ml benzene and filtered from solids. Evaporation of filtrate *in vacuo* gives the expected chloride which is then treated with triphenylphosphine (468 mg) in 5 ml DMF and stirred at room temperature for 1 hr. After evaporation of solvent *in vacuo*, the residue is dissolved in 70 ml $CH_2Cl_2$ and washed with 0.5M sodium phosphate buffer (pH 6.9). The organic layer is separated, dried over $MgSO_4$ and chromatographed on silica gel eluting with 30% ethyl acetate/$CH_2Cl_2$ to give 16.

Example G
Preparation of 17

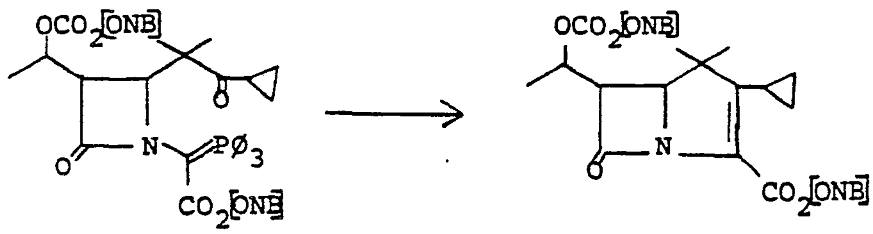

16 17

The ylide 16 (61 mg) is dissolved in 3 ml xylene and heated at 140° under $N_2$ for 1.5 hr. The mixture is cooled to 25°C. Xylene is removed under reduced pressure. The residue chromatographed on silica gel plates gives the desired product 17.

Example H
Preparation of 18

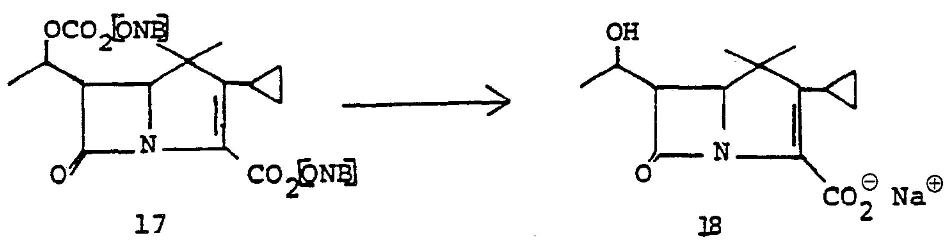

17 18

The carbapenem ester 17 (10 mg) is dissolved in 1 ml dioxane. To the solution is added 1 ml water, 0.2 ml ethanol, 10 mg $NaHCO_3$, and 5 mg 10% Pd/C. The mixture is hydrogenated for 20 min. at 40 psi.

The mixture is filtered from catalyst, and the filtrate is extracted with 3 × 5 ml ether. The aqueous layer is separated and chromatgraphed on an XAD—2 column eluted with water then 10% THF/water to give the title compound 18.

Example I

Following the procedures by the foregoing text and Examples, the following 1,2,6-substituted-1-carbadethiapen-2-em-3-carboxylic acid (I) are obtained by strict analogy when the suggested substitution of reagents is made.

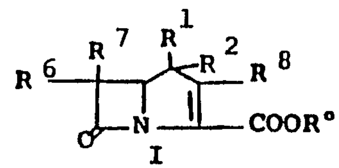

I

TABLE V

| Compound | $R^6$ | $R^7$ | $R^8$ | $R^1$ | $R^2$ | $R^o$ |
|---|---|---|---|---|---|---|
| 1.) | 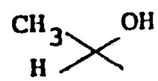 | H | 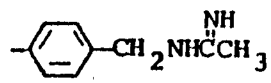 | $CH_3$ | $CH_3$ | H |
| 2.) | 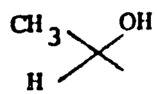 | H | 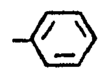 | $CH_2CH_2NH_2$ | $CH_3$ | H |
| 3.) | 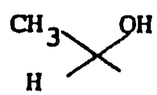 | H | 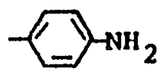 | $CH_3$ | $C_2H_5$ | H |
| 4.) | 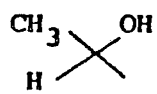 | H | 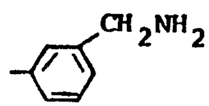 | $CH_3$ | 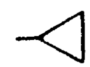 | $-CH_2O\overset{O}{\overset{\|}{C}}CMe_3$ |
| 5.) | 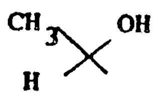 | H | 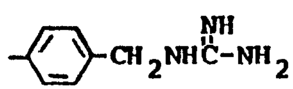 | $CH_3$ | $CH_3$ | H |
| 6.) | 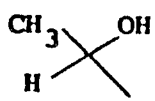 | H | $-CH_2CH_2NH\overset{NH}{\overset{\|}{C}}-CH_3$ | $CH_3$ | $CH_3$ | H |

| Compound | $R^6$ | $R^7$ | $R^8$ | $R^1$ | $R^2$ | $R^o$ |
|---|---|---|---|---|---|---|
| 7.) | 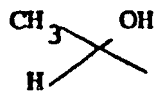 | H | 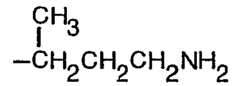 | $CH_3$ | $CH_3$ | H |
| 7a.) | 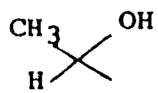 | H | 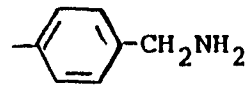 | $CH_3$ | $CH_3$ | H |
| 8.) | 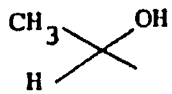 | H | 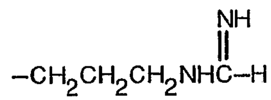 | $CH_3$ | $CH_3$ | H |
| 9.) | 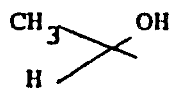 | H | 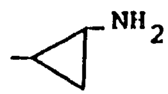 | $CH_3$ | $CH_3$ | H |
| 10.) | 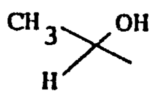 | $CH_3$ | 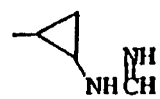 | $CH_3$ | $CH_3$ | H |
| 11.) | 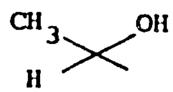 | $CH_3$ | 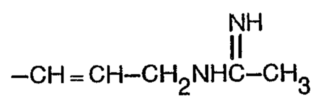 | $C_2H_5$ | $CH_3$ | H |
| 12.) | 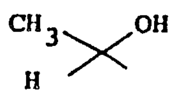 | H | 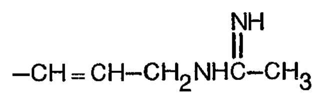 | $C_2H_5$ | $CH_3$ | H |
| 13.) | 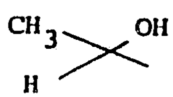 | H | 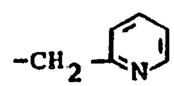 | $CH_3$ | $CH_3$ | H |

| Compound | $R^6$ | $R^7$ | $R^8$ | $R^1$ | $R^2$ | $R^0$ |
|---|---|---|---|---|---|---|
| 14.) | 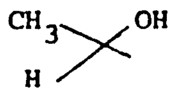 | H | 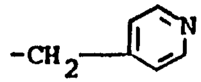 | $CH_3$ | $CH_3$ | H |
| 15.) | 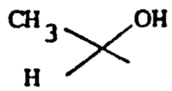 | H | 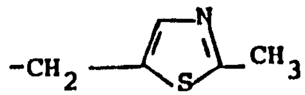 | $CH_3$ | $CH_3$ | Na |
| 16.) | $-CH_2OH$ | H | 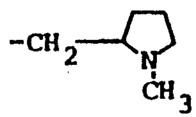 | $CH_3$ | $CH_3$ | H |
| 17.) | $-CH_2OH$ | $CH_3$ | 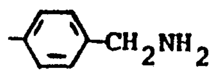 | $CH_3$ | Et | H |
| 18.) | $-CH_2OH$ | $CH_3$ | 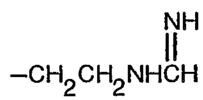 | $CH_3$ | $CH_3$ | H |
| 19.) | 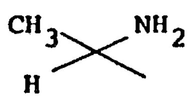 | H | $CH_3$ | $CH_3$ | $CH_3$ | H |
| 20.) | 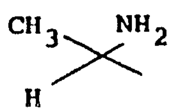 | H | 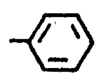 | $CH_3$ | $CH_3$ | H |
| 21.) | 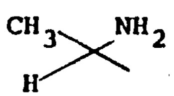 | H | 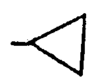 | $C_2H_5$ | $CH_3$ | H |

| Compound | $R^6$ | $R^7$ | $R^8$ | $R^1$ | $R^2$ | $R^0$ |
|---|---|---|---|---|---|---|
| 22.) | $-CH_2NH_2$ | $CH_3$ | 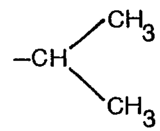 | $CH_3$ | $CH_3$ | H |
| 23.) | 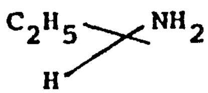 | H | 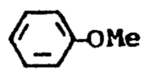 | $CH_3$ | $CH_2CH_2CH_3$ | H |
| 24.) | 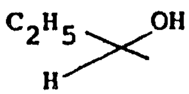 | H | 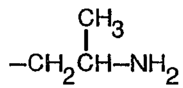 | $CH_3$ | 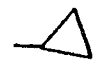 | H |
| 25.) | 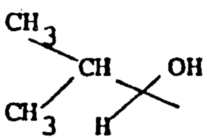 | H | 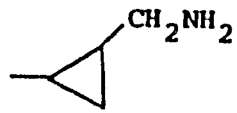 | $CH_3$ | $CH_3$ | H |
| 26.) | 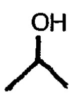 | H | 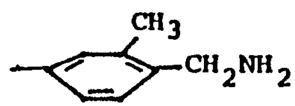 | $CH_3$ | $CH_3$ | H |
| 27.) | 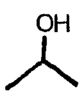 | H | $-CH_2CH_2CH_2NH_2$ | $-CH_2-CH_2-$ | | |

Example J

Preparation of Pharmaceutical Compositions

One such unit dosage form is prepared by mixing 120 mg of 1,1-dimethyl-6-(1-hydroxymethyl)-2-(2-aminocyclopropylthio)-1-carbadethiapen-2-em-3-carboxylic acid with 20 mg of lactose and 5 mg of magnesium stearate and placing the 145 mg. mixture into a No. 3 gelatin capsule. Similarly, by employing more of the active ingredient and less lactose, other dosage forms can be put up in No. 3 gelatin capsules and should it be necessary to mix more than 145 mg. of ingredients together larger capsules such as compressed tablets and pills can also be prepared. The following examples are illustrative of the preparation of pharmaceutical formulations:

| TABLET | PER TABLET |
|---|---|
| 1,1-dimethyl-6-(1-hydroxymethyl)-2-(2-aminocyclopropylthio)-1-carbadethiapen-2-em-3-carboxylic acid | 125 mg. |
| Dicalcium Phosphate | 192 mg. |
| Cornstarch, U.S.P. | 6 mg. |
| Lactose, U.S.P. | 190 mg. |
| Magnesium Stearate | Balance |

The active ingredient is blended with the dicalcium phosphate, lactose and about half of the cornstarch. The mixture is then granulated with 15% cornstarch paste (6 mg) and rough-screened. It is dried at 45°C and screened again through No. 16 screens. The balance of the cornstarch and the magnesium stearate is added and the mixture is compressed into tablets, approximately 1.27 cm in diameter each weighing 800 mg.

Parenteral Solution
Ampoule:

| | | |
|---|---|---|
| 1,1-dimethyl-6-(1-hydroxymethyl)-2-(2-aminocyclopropylthio)-1-carbadethiapen-2-em-3-carboxylic acid | | 500 mg |
| Dilutent: Sterile Water for Injection | | 5 cc. |

Opthalmic Solution

| | | |
|---|---|---|
| 1,1-dimethyl-6-(1-hydroxymethyl)-2-(2-aminocyclopropylthio)-1-carbadethiapen-2-em-3-carboxylic acid | | 100 mg. |
| Hydroxypropylmethyl cellulose | | 5 mg. |
| Sterile Water | to | 1 ml. |

Otic Solution

| | | |
|---|---|---|
| 1,1-dimethyl-6-(1-hydroxymethyl)-2-(2-aminocyclopropylthio)-1-carbadethiapen-2-em-3-carboxylic acid | | 100 mg. |
| Benzalkonium chloride | | 0.1 mg. |
| Sterile Water | to | 1 ml. |

Topical Ointment

| | |
|---|---|
| 1,1-dimethyl-6-(1-hydroxymethyl)-2-(2-aminocyclopropylthio)-1-carbadethiapen-2-em-3-carboxylic acid | 100 mg. |
| Polyethylene Glycol 4000 U.S.P. | 400 mg. |
| Polyethylene Glycol 400 U.S.P. | 1.0 gram. |

**Claims for the Contracting States: BE CH DE FR GB IT LI LU NL SE**

1. A compound having the structure:

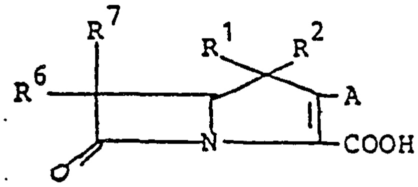

(I)

and the pharmaceutically acceptable salts and esters thereof; wherein either

a) A is $SR^8$ and $R^1$, $R^2$, $R^6$, $R^7$ and $R^8$ are independently selected from the group consisting of substituted and unsubstituted alkyl, alkenyl, and alkylnyl, having from 1—10 carbon atoms; cycloalkyl, cycloalkylalkyl, and alkylcycloalkyl, having 3—6 carbon atoms in the cycloalkyl ring and 1—6 carbon atoms in the alkyl moieties; spirocycloalkyl having 3—6 carbon atoms; phenyl; aralkyl, aralkenyl, and aralkynyl wherein the aryl moiety is phenyl and the alkyl chain has a 1—6 carbon atoms; heteroaryl, heteroaralkyl, heterocyclyl and heterocyclylalkyl wherein the substituent or substituents relative to the above-named radicals are selected from the group consisting of: amino, mono-, di-, and trialkylamino, hydroxyl, alkoxyl, mercapto, alkylthio, phenylthio, sulfamoyl, amidino, guanidino, nitro, chloro, bromo, fluoroiodo, cyano, and carboxy; and wherein the hetero atom or atoms in the above-named heterocyclic moieties are selected from the group consisting of 1—4 oxygen, nitrogen or sulphur atoms; and wherein the alkyl moieties of the above recited substituents have 1—6 carbon atoms and $R^6$, $R^7$ and $R^8$ can also represent hydrogen; when $R^6/R^7$ is hydrogen and $R^7/R^6$ is 1-hydroxyethyl, then $R^8$is not 2-aminoethyl or an N-derivative thereof; or b) A is $R^8$ and $R^1$, $R^2$, $R^6$, $R^7$ and $R^8$ are independently selected from the group consisting of substituted and unsubstituted: alkyl having 1—6 carbon atoms, arakyl, alkenyl and alkynyl having 2—6 carbon atoms, aryl and aralkyl having 6—10 ring carbon atoms and 1—6 carbon atoms in the alkyl chain, heterocyclyl-heterocyclythio ($R^8$ is excluded) and heterocyclyl-alkyl having 1—5 hetero atoms selected from O, N or S in the ring and 1—6 carbon atoms in the alkyl chain, cycloalkyl, spirocycloalkyl, and cycloalkylalkyl having 3 to 6 ring carbon atoms and 1—6 carbon atoms in the alkyl moiety; wherein the substituents on $R^1$, $R^2$, $R^6$, $R^7$ and $R^8$ are selected from chloro, bromo, fluoro, iodo, hydroxyl, amino, mono-, di- and trialkyl substituted amino (each alkyl having 1—6 carbon atoms) alkoxyl having 1—6 carbon atoms, guanidino, cyano, amidino and carboxyl; and $R^6$, $R^7$ and $R^8$ can also represent hydrogen.

2. A compound according to Claim 1 a) wherein $R^1$ and $R^2$ are selected from alkyl having 1—10 carbon atoms, cycloalkyl having 3—6 carbon atoms, spirocycloalkyl having 3—6 carbon atoms, benzyl or phenyl; and $R^6$ is H or methyl and $R^7$ is alkyl having from 1—10 carbon atoms, phenyl, aralkyl, wherein the aryl moiety is phenyl and the alkyl chain has 1—6 carbon atoms or hydroxyl-substituted alkyl having from 1—10 carbon atoms, phenyl or aralkyl wherein the aryl moiety is phenyl and the alkyl chain has 1—6 carbon atoms.

3. A compound according to Claim 2 wherein $R^1$ and $R^2$ are selected from spirocyclopropyl, methyl, ethyl, isopropyl, t-butyl or phenyl and $R^7$ is 1-hydroxyethyl, methyl or hydroxymethyl.

4. A compound according to Claim 1 a) wherein $R^8$ is selected from the group consisting of:

H,

$CH_3$,

$(CH_2)_2NH_2$,

$C(CH_3)_2CH_2NH_2$,

$C(CH_3)_2CH_2NH{-}\overset{\overset{NH}{\|}}{C}{-}H$,

$C(CH_3)_2CH_2NH{-}\overset{\overset{NH}{\|}}{C}{-}CH_3$,

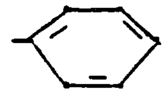

,

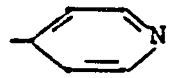

,

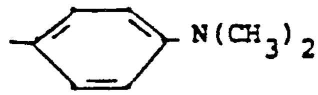

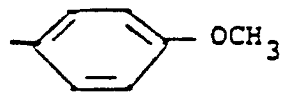

$CH_2CH_2CH_2NH_2$,

$CH_2CH(CH_3)NH_2$,

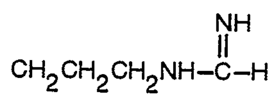

,

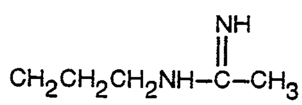

,

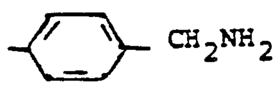

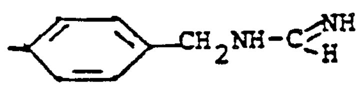

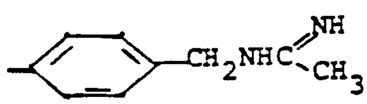

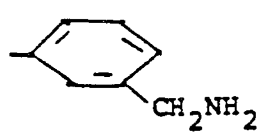

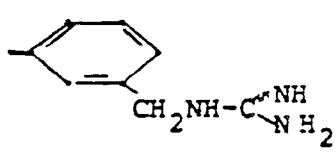

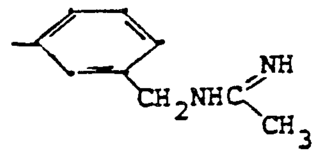

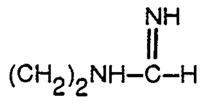

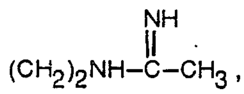

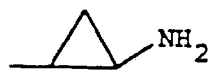

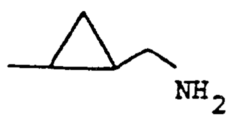

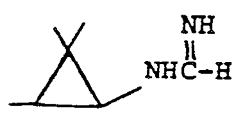

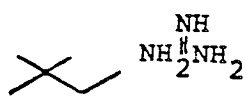

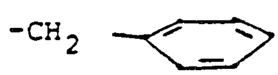

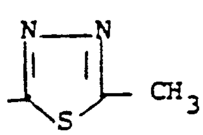

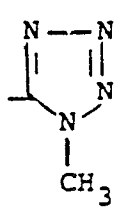

$\cdot CH(CH_3)CH_2NH_2$,

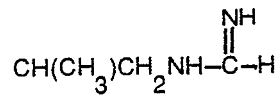 or

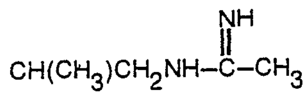

5. A compound according to Claim 4 wherein $R^6$ is H or methyl; $R^7$ is selected from:

$-CH_2OH$

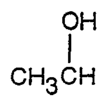

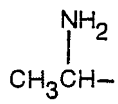

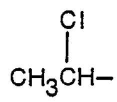

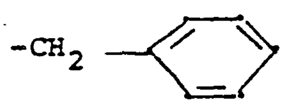

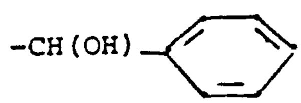

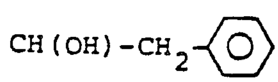

$-CH_3$

$-CH_2CH_3$

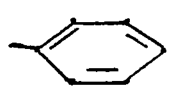 ;

and $R_1$ and $R_2$ are selected from spirocyclopropyl; phenyl; cycloalkyl having 3—6 carbon atoms; alkyl having 1—6 carbon atoms; cyclopropylalkyl having 4—9 carbon atoms.

6. A compound according to Claim 1 selected from the group consisting of:

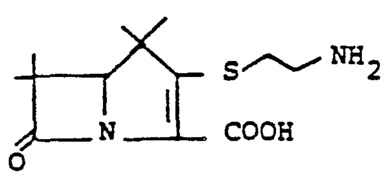

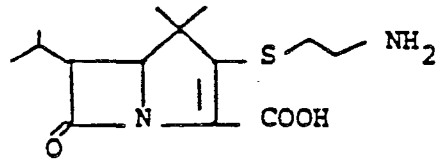

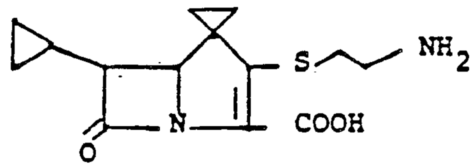

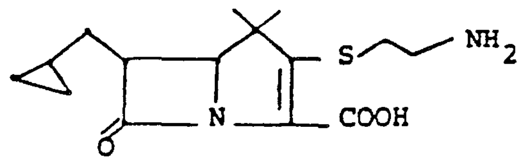

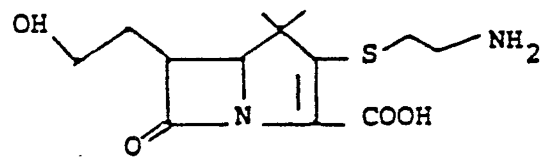

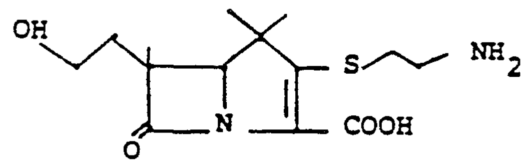

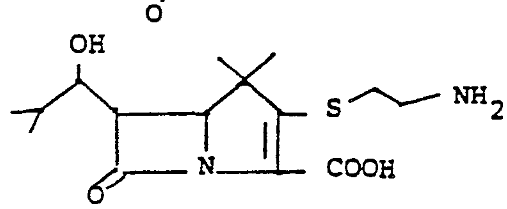

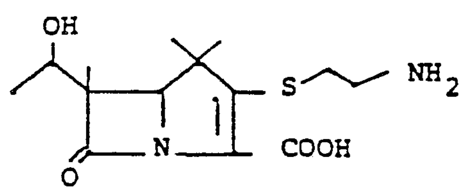

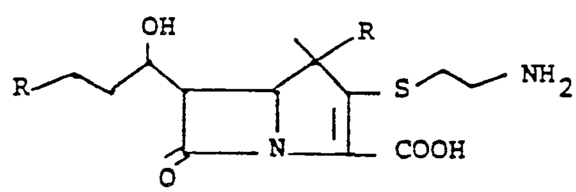

(R = phenyl, m-aminomethylphenyl, o-, p-, m-hydroxyphenyl; R' is phenyl)

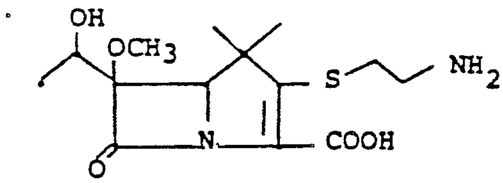

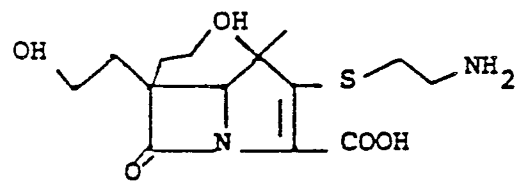

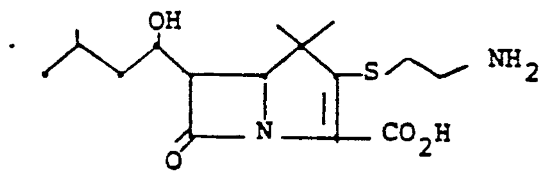

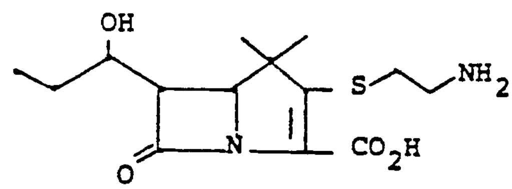

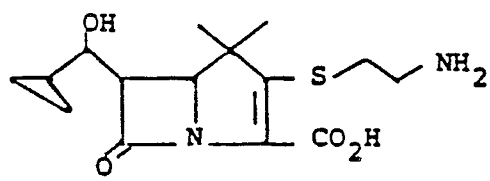

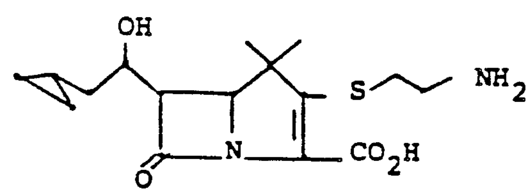

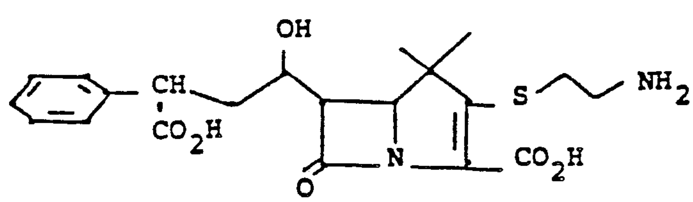

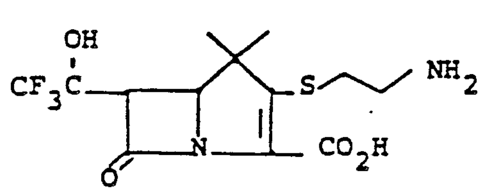

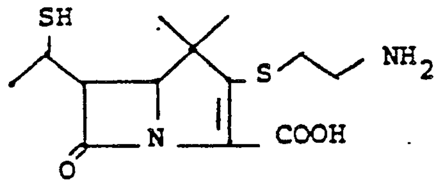

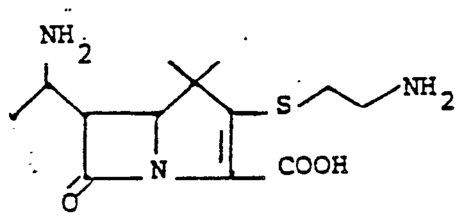

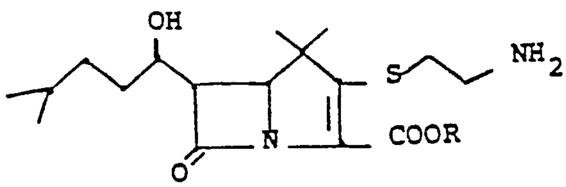

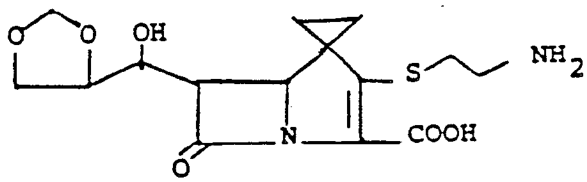

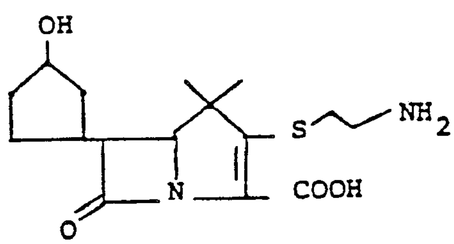

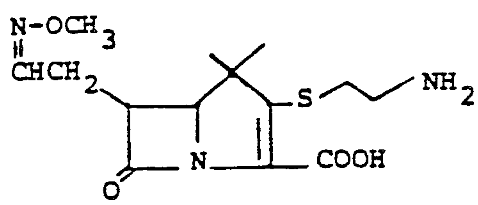

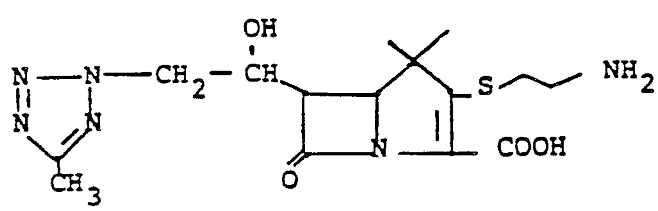

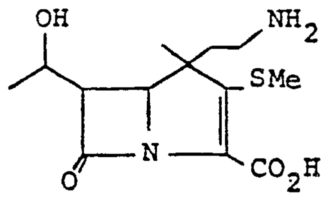

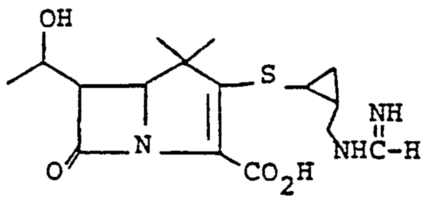

7. A compound according to Claim 6 wherein the aminoethylthio side chain,

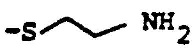,

is replaced by a member of the group consisting of

$-SCF_3$,

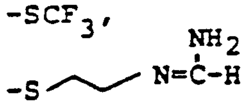,

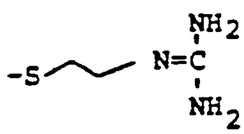,

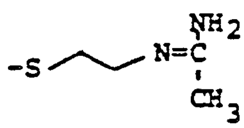,

$-S-(CH_2)_n-NH_2$,

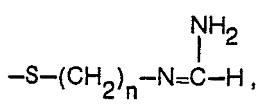

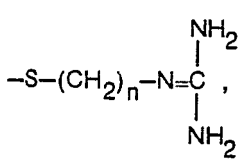

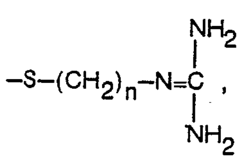

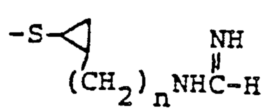

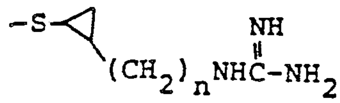

n = 1, 3, 4, 5 or 6.

8. The compound of the formula:

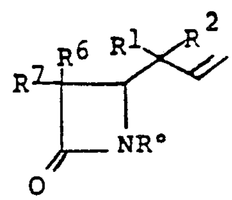

wherein R° is H or a readily removable protecting group; and wherein $R^1$, $R^2$, $R^6$, and $R^7$ are as defined in claim 1.

9. The compound of Claim 8 wherein R° is triloweralkylsilyl, wherein the alkyl moieties have from 1 to 6 carbon atoms.

10. A compound of the formula:

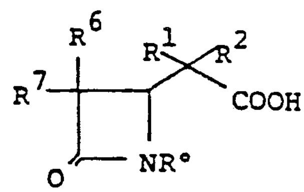

wherein R° is hydrogen or a readily removable protecting group; and wherein $R^7$, $R^6$, $R^1$, and $R^2$ are as defined in claim 1.

11. A compound of the formula:

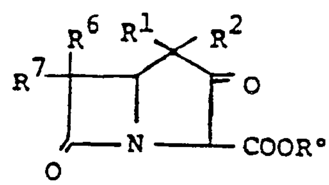

wherein R° is hydrogen, a salt cation, a pharmaceutically acceptable ester moiety, or a readily removable protecting group; and wherein $R^7$, $R^6$, $R^1$, and $R^2$ are as defined in claim 1.

12. A process for preparing:

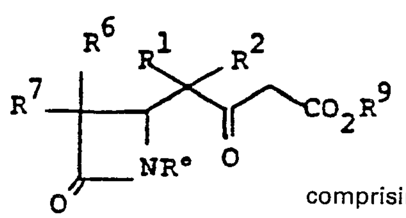

comprising:

oxidizing 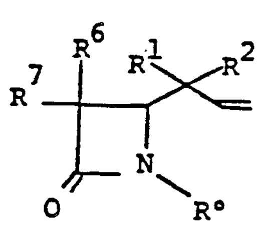 to form: 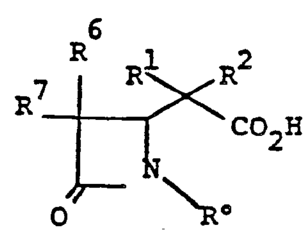

followed by treating with $R^9O_2CCH_2CO_2^{\ominus}$; wherein $R^9$ is a pharmaceutically acceptable ester moiety or a readily removable protecting group and wherein $R^1$, $R^2$, and $R^6$ and $R^7$ are as defined in claim 1.

13. The process of Claim 12 wherein $R^9O_2CCH_2CO_2^{\ominus}$ is utilized as $(R^9O_2CCH_2CO_2)_2Mg$.

14. A process for preparing a compound of claim 1 a) having the structure wherein A is $SR^8$ and the pharmaceutically acceptable salts and esters thereof comprising the steps of acylating or halogenating and then treating with the thio reagent $HSR^8$ a compound of the formula

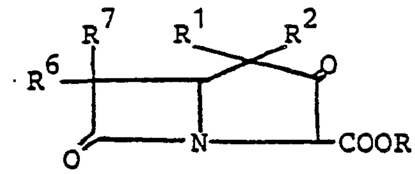

wherein $R^6$, $R'$, $R^1$, $R^2$, and $R^8$ are defined by independent Claim 1 and R is a pharmaceutically acceptable ester moiety or a readily removable carboxyl protecting group; or

b) having the structure wherein A is $R^8$ comprising oxidizing:

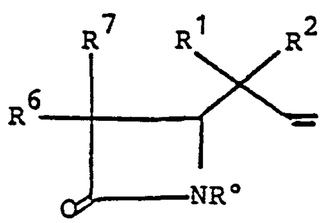

to form

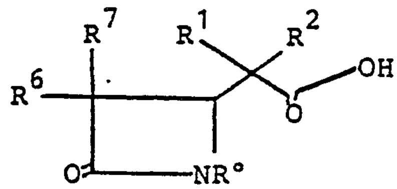

followed by reaction with dicyclohexylcarbodiimide, ethylchloroformate, 2-fluoropyridine, 1-fluoro-2, 4-dinitrobenzene, thionylchloride or oxalylchloride and treating with $R^8{}_2$ Cu MgX°, $R^8$ MgX°, Li Cu $R^8{}_2$ or $R^8{}_2$Cd wherein X° is bromo or chloro to establish $R^8$

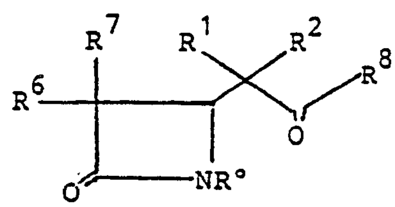

followed by N-deprotecting and treating with a glyoxylate to yield

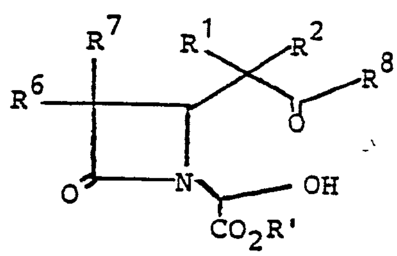

followed by halogenating with a conventional halogenating agent and treating with a triorganophosphine to form

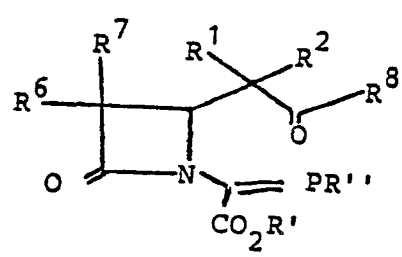

followed by cyclizing and deprotecting wherein R° is a readily removable protecting group; R′ is a readily removable protecting group or pharmaceutically acceptable ester moiety; and R″ is alkyl having up to 4 carbon atoms or phenyl.

15. An antibiotic pharmaceutical composition comprising a therapeutically effective amount of a compound according to Claim 1 and a pharmaceutical carrier thereof.

16. A compound according to Claim 1b wherein $R^1$ and $R^2$ are alkyl, having 1—6 carbon atoms, spirocyclopropyl, benzyl or phenyl; $R^6$ is hydrogen and $R^7$ is substituted or unsubstituted: alkyl having 1—6 carbon atoms or phenylalkyl; wherein the substituent is OH or $NH_2$; and $R^8$ is selected from:

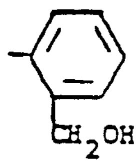

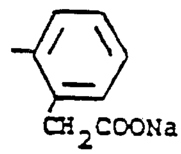

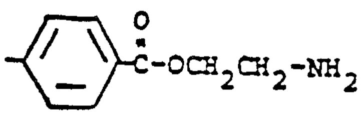

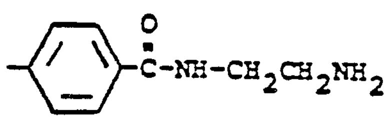

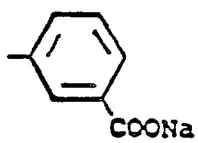

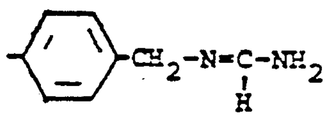

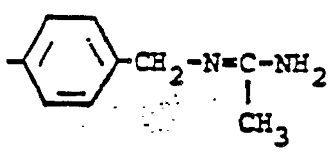

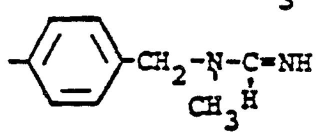

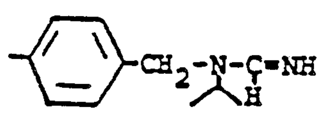

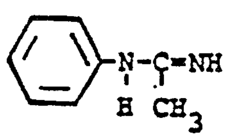

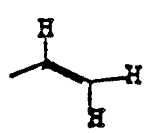

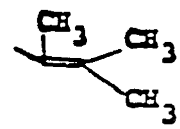

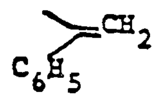

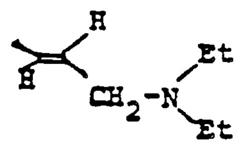

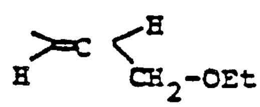

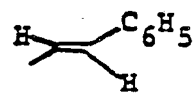

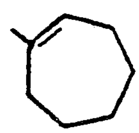

$-C\equiv CH$

$-C\equiv CCH_2NH_2$

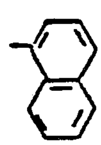

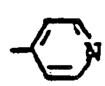

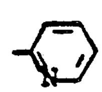

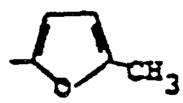

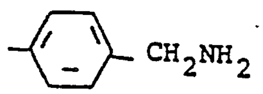

17. A compound selected according to Claim 16 wherein $R^1$ and $R^2$ are methyl, ethyl, isopropyl, cyclopropyl, t-butyl or phenyl; $R^7$ is 1-hydroxyethyl, methyl, or hydroxymethyl; and $R^6$ is hydrogen.

18. A compound according to Claims 1—7, 16 and 17 wherein $R^8$ is selected from the group consisting of:

hydrogen, $-CH_2CH_2CH_2NH_2$, $-CH_2CH_2NH_2$, $-CH_3$, $-CH_2CH_2CH_2OH$, $-CH_2CH_2COOH$,

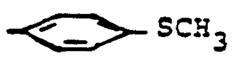 , $-CH_2CH{=}CH{-}SCH_3$, 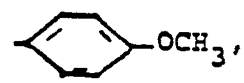 ,

$-CH{=}CHCH_2CH_2NH_2$, $-CH_2CH_2CH_2CH_2NH_2$, 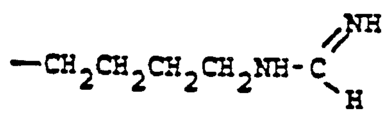 ;

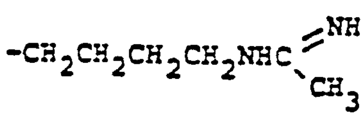 , 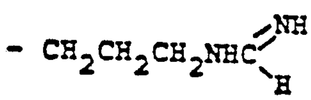 ,

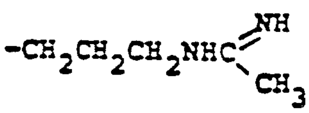 , 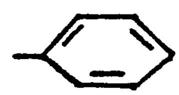 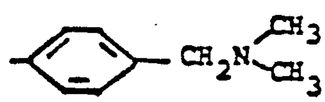 ,

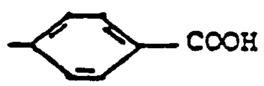 , 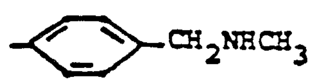 , 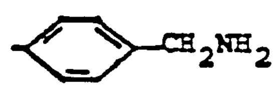 ,

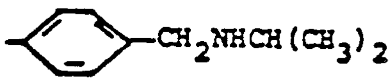 , 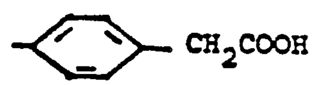 ,

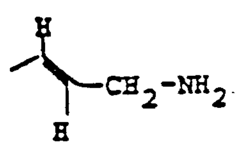

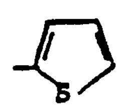

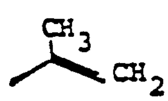

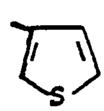

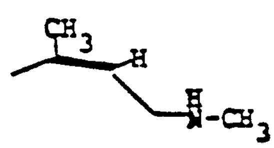

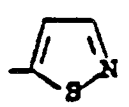

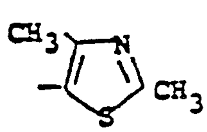

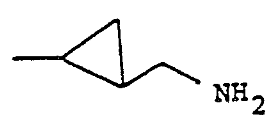

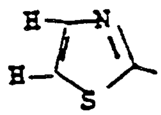

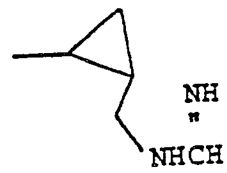

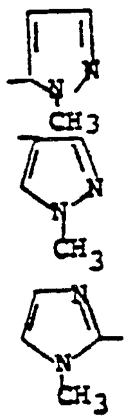

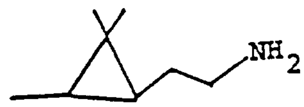

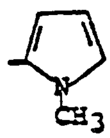

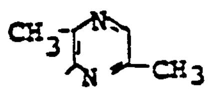

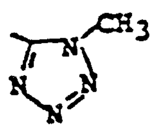

19. A compound according to Claim 18 wherein $R^1$ and $R^2$ are alkyl having 1—6 carbon atoms, phenyl, cyclopropyl, or spirocyclopropyl.

20. A compound according to Claim 1b) selected from:

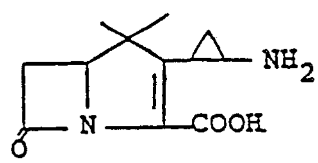
,

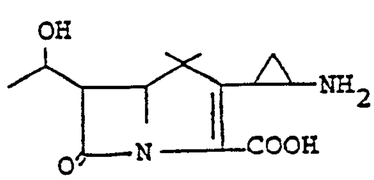
,

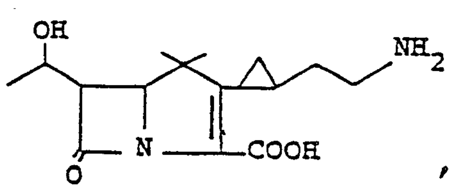
,

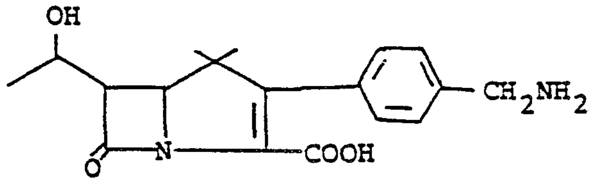

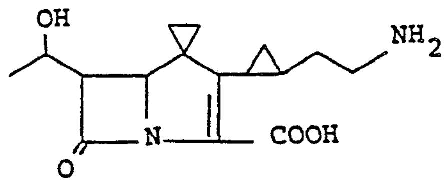

,

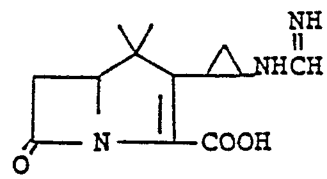

,

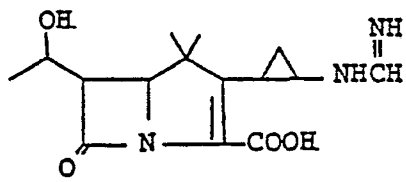

,

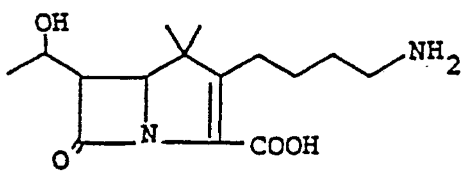

21. A process for preparing a compound according to Claim 1b) comprizing cyclizing:

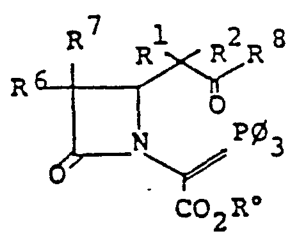

wherein φ is phenyl, and R° is a pharmaceutically acceptable ester moiety or a readily removable blocking group.

22. An antibiotic pharmaceutical composition causing a therapeutically effective amount of a compound according to Claims 1b) and 16—20 and a pharmaceutical carrier therefor.

23. A compound having the formula:

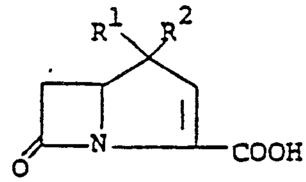

and its pharmaceutically acceptable salts and esters wherein $R^1$ and $R^2$ are selected from the group consisting of substituted and unsubstituted: loweralkyl having from 1—6 carbon atoms, phenyl, phenylloweralkyl, cycloalkyl having from 3 to 6 carbon atoms, cycloalkylakyl having 1 to 7 carbon atoms in the chain and 3—6 carbon atoms in the ring and spirocycloalkyl having 3—6 carbon atoms; wherein said substituents on $R^1$ and $R^2$ are selected from the group consisting of halogen, hydroxyl, amino, and substituted amino, azido, cyano, carboxyl, alkoxyl, and mono-, di- and trialkylamino, each alkyl radical of the foregoing having 1—6 carbon atoms.

24. A compound according to Claim 23 wherein $R^1$ and $R^2$ are selected from methyl, phenyl, ethyl, cyclopropyl, propyl, isopropyl, and spiro-cyclopropyl.

25. A compound having the structure:

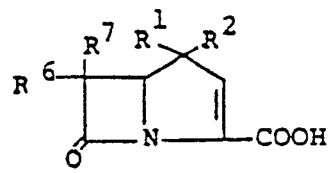

and the pharmaceutically acceptable salts and esters thereof; wherein $R^1$, $R^2$, $R^6$ and $R^7$ are independently selected from the group consisting of hydrogen, and substituted and unsubstituted: alkyl having 1—6 carbon atoms; phenyl; phenylalkyl wherein the alkyl moiety has 1—6 carbon atoms; cycloalkyl and cycloalkylalkyl having 3 to 6 ring carbon atoms and 1—6 carbon atoms in the alkyl moiety; and spirocycloalkyl having 3 to 6 carbon atoms; wherein the substituent or substituents on $R^1$, $R^2$, $R^6$ and $R^7$ are selected from chloro, bromo, fluoro, hydroxyl, amino, mono-, di-, and trialkylamino (each alkyl having 1—6 carbon atoms), alkoxyl having 1—6 carbon atoms, cyano and carboxyl; wherein $R^1$ and $R^2$ are not hydrogen.

26. A compound according to Claim 25 wherein $R^1$ and $R^2$ are selected from: alkyl, cyclopropyl, spirocyclopropyl, and benzyl and phenyl; $R^6$ is alkyl and phenylalkyl substituted by hydroxyl or amino; and $R^7$ is hydrogen or alkyl or phenylalkyl substituted by hydroxyl or amino.

27. A compound according to Claim 26 wherein $R^1$ and $^2$ are selected from: methyl, ethyl, isopropyl, t-butyl, spirocyclopropyl, or phenyl; $R^6$ is 1-hydroxyethyl, methyl, or hydroxymethyl; and $R^7$ is hydrogen.

28. A compound according to Claim 25 having the structure:

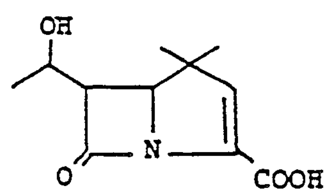

29. A compound according to Claim 25 having the structure:

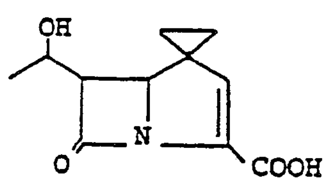

**Claims for the Contracting State: AT**

1. A process for preparing:

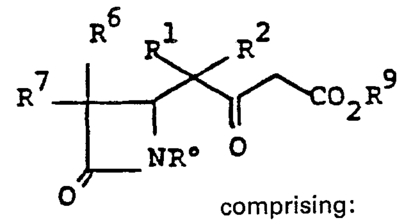

comprising:

oxidizing 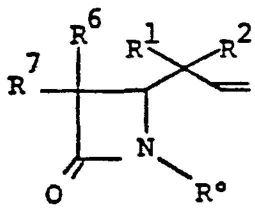 to form: 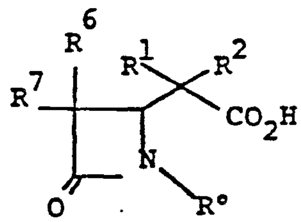

followed by treating with $R^9O_2CCH_2CO_2^{\ominus}$; wherein $R^1$, $R^2$, $R^6$, and $R^7$ are independently selected from the group consisting of

substituted and unsubstituted: alkyl, alkenyl, and alkynyl, having from 1—10 carbon atoms; cycloalkyl cycloalkylalkyl, and alkylcycloalkyl, having 3—6 carbon atoms in the cycloakyl ring and 1—6 carbon atoms in the alkyl moieties; spirocycloalkyl having 3—6 carbon atoms; phenyl; aralkyl, aralkenyl, and aralkynyl wherein the aryl moiety is phenyl and the alkyl chain has 1—6 carbon atoms; heteroaryl, heteroaralkyl, heterocyclyl and heterocyclylalkyl wherein the substituent or substituents relative to the above-named radicals are selected from the group consisting of: amino, mono-, di-, and trialkylamino, hydroxyl, alkoxyl, mercapto, alkylthio, phenylthio, sulfamoyl, amidino, guanidino, nitro, chloro, bromo, fluoroidodo, cyano and carboxy; and wherein the hetero atom or atoms in the above-named heterocyclic moieties are selected from the group consisting of 1—4 oxygen, nitrogen or sulphur atoms; and wherein the alkyl moieties of the above-recited substituents have 1—6 carbon atoms; and $R^6$ and $R^7$ can also be hydrogen; $R^9$ is a pharmaceutically acceptable ester moiety or a readily removable protecting group and $R^0$ is hydrogen or a readily removable protecting group.

2. The process of Claim 1 wherein $R^9O_2CCH_2CO_2^{\ominus}$ is utilized as $(R^9O_2CCH_2CO_2)_2Mg$.

3. A process for preparing a compound having the structure:

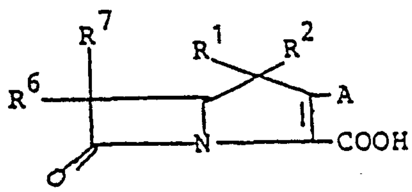

and the pharmaceutically acceptable salts and esters thereof:

a) wherein A is $SR^8$ and $R^1$, $R^2$, $R^6$, $R^7$ and $R^8$ are independently selected from the groups consisting of substituted or unsubstituted: alkyl, alkenyl, and alkynyl, having from 1—10 carbon atoms; cycloalkyl, cycloalkylalkyl, and alkycycloalkyl, having 3—6 carbon atoms in the cycloalkyl ring and 1—6 carbon atoms in the alkyl moieties; spirocycloalkyl having 3—6 carbon atoms; phenyl; aralkyl, aralkenyl, and aralkynyl wherein the aryl moiety is phenyl and the alkyl chain has 1—6 carbon atoms; heteroaryl, heteroaralkyl, heterocyclyl and heterocyclylalkyl wherein the substituent or substituents relative to the above-named radicals are selected from the group consisting of: amino, mono-, di-, and trialkylamino, hydroxyl, alkoxyl, mercapto, alkylthio, phenylthio, sulfamoyl, amidino, guanidino, nitro, chloro, bromo, fluoroiodo, cyano and carboxy; and wherein the hetero atom or atoms in the above-named heterocyclic moieties are selected from the group consisting of 1—4 oxygen, nitrogen or sulphur atoms; and wherein the alkyl moieties of the above recited substituents have 1—6 carbon atoms; and $R^6$, $R^7$ and $R^8$ can also represent hydrogen; when $R^6/R^7$ is hydrogen and the $R^7/R^6$ is 1-hydroxyethyl, then $R^8$ is not 2-aminoethyl or an N-derivative thereof;

comprising the steps of acylating or halogenating and then treating with the thio reagent $HSR^8$ a compound of the formula

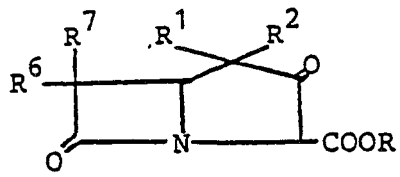

wherein $R^6$, $R^7$, $R^1$, $R^2$ and $R^8$ are defined as above

and R is a pharmaceutically acceptable ester moiety or a readily removable carboxyl protecting group; or

b) wherein A is $R^8$ and $R^1$, $R^2$, $R^6$, $R^7$ and $R^8$ are independently selected from the group consisting of substituted and unsubstituted: alkyl having 1—6 carbon atoms, aralkyl, alkenyl and alkynyl having 2—6 carbon atoms, aryl and aralkyl having 6—10 ring carbon atoms and 1—6 carbon atoms in the alkyl chain, heterocyclyl, heterocyclylthio ($R^8$ is excluded) and heterocyclylalkyl having 1—5 hetero atoms selected from O, N or S in the ring and 1—6 carbon atoms in the alkyl chain, cycloalkyl, spirocycloalkyl, and cycloalkylalkyl having 3 to 6 ring carbon atoms and 1—6 carbon atoms in the alkyl moiety; wherein the substituents on $R^1$, $R^2$, $R^6$, $R^7$ and $R^8$ are selected from chloro, bromo, fluoro, iodo, hydroxyl, amino, mono-, di- and trialkyl substituted amino (each alkyl having 1—6 carbon atoms) alkoxyl having 1—6 carbon atoms, guanidino, cyano, amidino and carboxyl; and $R^6$, $R^7$ and $R^8$ can also represent hydrogen; comprising oxidizing:

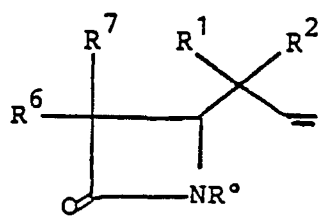

to form

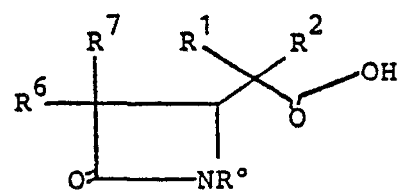

followed by reaction with dicyclohexylcarbodiimide, ethylchloroformate, 2-fluoropyridine, 1-fluoro-2, 4-dinitrobenzene, thionylchloride or oxalylchloride and treating with $R^8_2$ Cu MgX°, $R^8$ MgX°, Li Cu $R^8_2$ or $R^8_2$Cd wherein X° is bromo or chloro to establish $R^8$

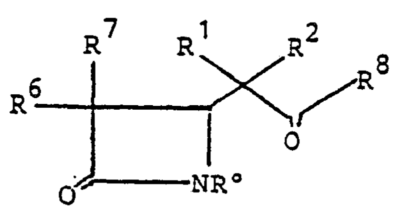

followed by N-deprotecting and treating with a glyoxylate to yield

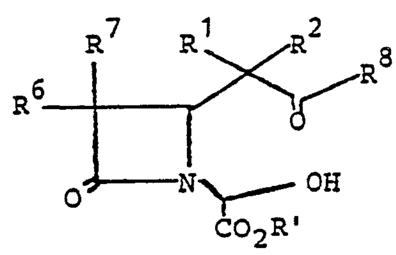

followed by halogenating with a conventional halogenating agent and treating with a triorganophosphine to form

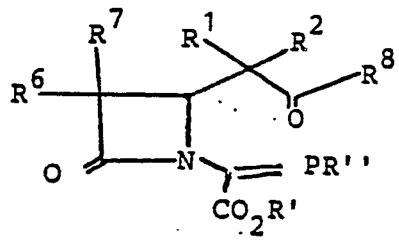

followed by cyclizing and deprotecting wherein R° is a readily removable protecting group; R' is a readily removable protecting group or pharmaceutically acceptable ester moiety; and R'' is alkyl having up to 4 carbon atoms or phenyl.

4. A process for preparing a compound having the structure:

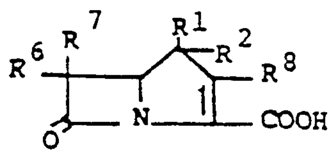

and the pharmaceutically acceptable salts and esters thereof; wherein $R^1$, $R^2$, $R^6$, $R^7$ and $R^8$ are independently as defined in claim 1, and comprising cyclizing:

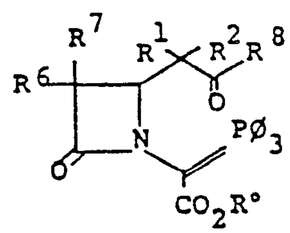

wherein ϕ is phenyl, and R° is a pharmaceutically acceptable ester moiety or a readily removable blocking group.

**Patentansprüche für die Vertragsstaaten: BE CH DE FR GB IT LI LU NL SE**

1. Eine Verbindung mit der Struktur:

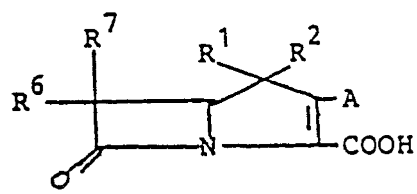

und die pharmazeutisch annehmbaren Salze und Ester davon; worin entweder

a) A für $SR^8$ steht, und $R^1$, $R^2$, $R^6$, $R^7$ und $R^8$ unabhängig voneinander aus der Gruppe ausgewählt sind, die aus substituiertem und unsubstituiertem Alkyl, Alkenyl und Alkinyl mit 1 bis 10 Kohlenstoffatomen; Cycloalkyl, Cycloalkylalkyl und Alkylcycloalkyl mit 3 bis 6 Kohlenstoffatomen in dem Cycloalkylring und 1 bis 6 Kohlenstoffatomen in den Alkylresten; Spirocycloalkyl mit 3 bis 6 Kohlenstoffatomen; Phenyl; Aralkyl, Aralkenyl und Aralkinyl, wobei der Arylrest Phenyl ist und die Alkylkette 1 bis 6 Kohlenstoffatome hat; Heteroaryl, Heteroaralkyl, Heterocyclyl und Heterocyclylalkyl besteht, wobei der Substituent oder die Substituenten bezüglich der oben genannten Reste aus der Gruppe ausgewählt sind, die aus: Amino,

Mono-, Di- und Trialkylamino, Hydroxyl, Alkoxyl, Mercapto, Alkylthio, Phenylthio, Sulfamoyl, Amidino, Guanidino, Nitro, Chlor, Brom, Fluoriod, Cyano und Carboxy besteht; und wobei das Heteroatom oder die Heteroatome in den oben genannten heterocyclischen Resten aus der Gruppe ausgewählt sind, die aus 1 bis 4 Sauerstoff-, Stickstoff- oder Schwefelatomen besteht; und wobei die Alkylreste in den oben aufgezählten Substituenten 1 bis 6 Kohlenstoffatome haben, und $R^6$, $R^7$ und $R^8$ auch Wasserstoff bedeuten können; und wobei, falls $R^6/R^7$ Wasserstoff ist und $R^7/R^6$ 1-Hydroxyethyl ist, $R^8$ nicht 2-Aminoethyl oder ein N-Derivat davon ist; oder

b) A für $R^8$ steht, und $R^1$, $R^2$, $R^6$, $R^7$ und $R^8$ unabhängig voneinander aus der Gruppe ausgewählt sind, die aus substituiertem und unsubstituiertem: Alkyl mit 1 bis 6 Kohlenstoffatomen, Aralkyl, Alkenyl und Alkinyl mit 2 bis 6 Kohlenstoffatomen, Aryl und Aralkyl mit 6 bis 10 Ringkohlenstoffatomen und 1 bis 6 Kohlenstoffatomen in der Alkylkette, Heterocyclyl, Heterocyclylthio (ausgeschlossen $R^8$) und Heterocyclylalkyl mit 1 bis 5 aus O, N oder S ausgewählten Heteroatomen im Ring und 1 bis 6 Kohlenstoffatomen in der Alkylkette, Cycloalkyl, Spirocycloalkyl und Cycloalkylalkyl mit 3 bis 6 Ringkohlenstoffatomen und 1 bis 6 Kohlenstoffatomen in dem Alkylrest besteht; wobei die Substituenten an $R^1$, $R^2$, $R^6$, $R^7$ und $R^8$ aus Chlor, Brom, Fluor, Iod, Hydroxyl, Amino, mono-, di- und trialkylsubstituiertem Amino (jedes Alkyl hat 1 bis 6 Kohlenstoffatome), Alkoxyl mit 1 bis 6 Kohlenstoffatomen, Guanidino, Cyano, Amidino und Carboxyl ausgewählt sind; und $R^6$, $R^7$ und $R^8$ auch Wasserstoff bedeuten können.

2. Eine Verbindung nach Anspruch 1 a), worin $R^1$ und $R^2$ aus Alkyl mit 1 bis 10 Kohlenstoffatomen, Cycloalkyl mit 3 bis 6 Kohlenstoffatomen, Spirocycloalkyl mit 3 bis 6 Kohlenstoffatomen, Benzyl oder Phenyl ausgewählt sind; $R^6$ H oder Methyl ist, und $R^7$ Alkyl mit 1 bis 10 Kohlenstoffatomen, Phenyl, Aralkyl, wobei der Arylrest Phenyl ist und die Alkylkette 1 bis 6 Kohlenstoffatome hat, oder hydroxylsubstituiertes Alkyl mit 1 bis 10 Kohlenstoffatomen, hydroxylsubstituiertes Phenyl oder hydroxylsubstituiertes Aralkyl, wobei der Arylrest Phenyl ist und die Alkylkette 1 bis 6 Kohlenstoffatome hat, bedeutet.

3. Eine Verbindung nach Anspruch 2, worin $R^1$ und $R^2$ aus Spirocyclopropyl, Methyl, Ethyl, Isopropyl, tert.Butyl oder Phenyl ausgewählt sind, und $R^7$ 1-Hydroxyethyl, Methyl oder Hydroxymethyl ist.

4. Eine Verbindung nach Anspruch 1 a), worin $R^8$ aus der Gruppe ausgewählt ist, die aus:

H,

$CH_3$,

$(CH_2)_2NH_2$,

$C(CH_3)_2CH_2NH_2$,

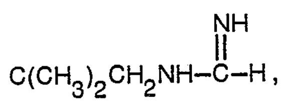

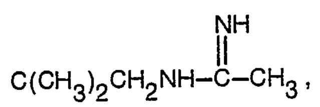

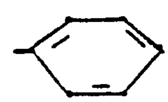

,

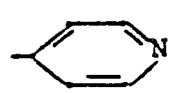

,

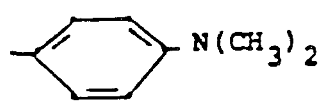

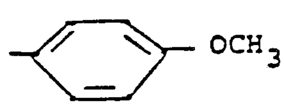

$CH_2CH_2CH_2NH_2$,

$CH_2CH(CH_3)NH_2$,

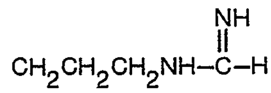,

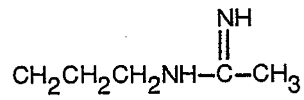,

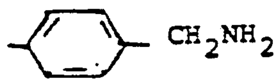

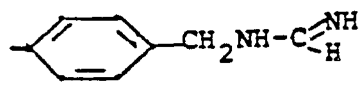

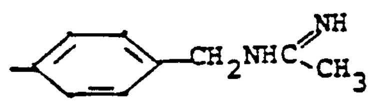

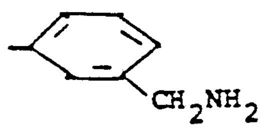

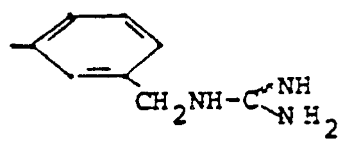

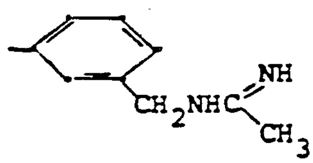

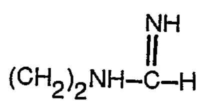

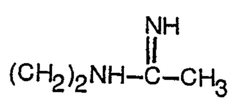,

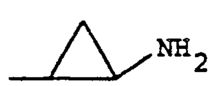

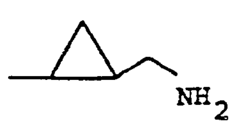

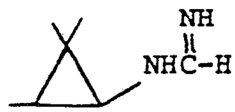

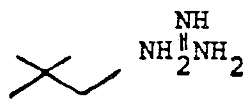

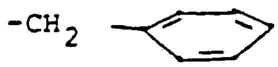

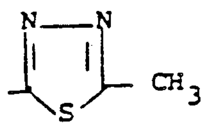

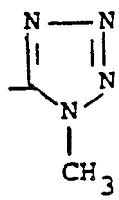

$CH(CH_3)CH_2NH_2$,

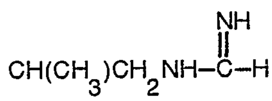 oder

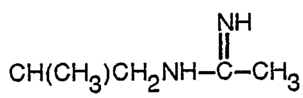 besteht

5. Eine Verbindung nach Anspruch 4, worin $R^6$ H oder Methyl ist; $R^7$ aus:

$-CH_2OH$

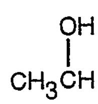

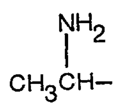

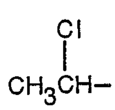

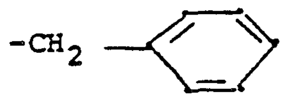

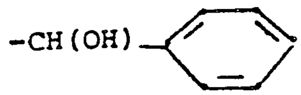

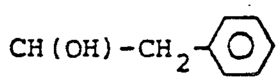

$-CH_3$

$-CH_2CH_3$

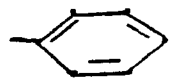 ;

ausgewählt ist; und R[1] und R[2] aus Spirocyclopropyl; Phenyl; Cycloalkyl mit 3 bis 6 Kohlenstoffatomen; Alkyl mit 1 bis 6 Kohlenstoffatomen; und Cyclopropylalkyl mit 4 bis 9 Kohlenstoffatomen ausgewählt sind.

6. Eine Verbindung nach Anspruch 1, ausgewählt aus der Gruppe, die aus:

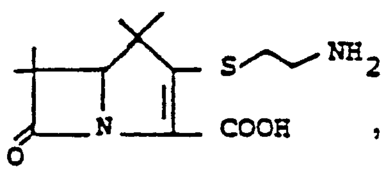 ,

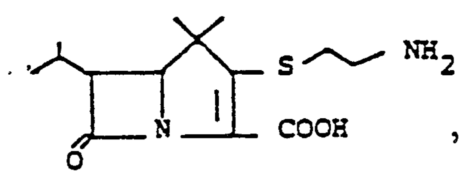 ,

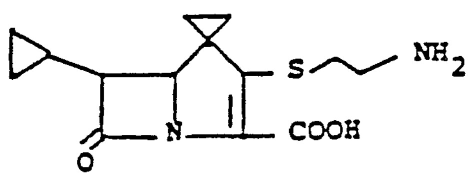 ,

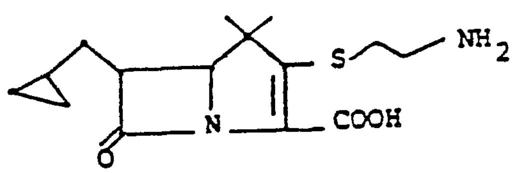 ,

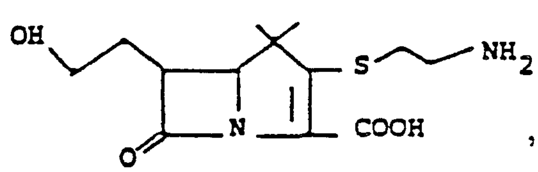 ,

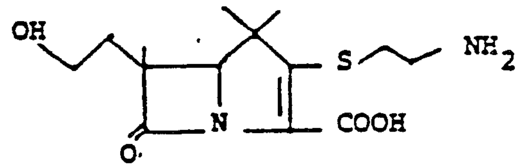

,

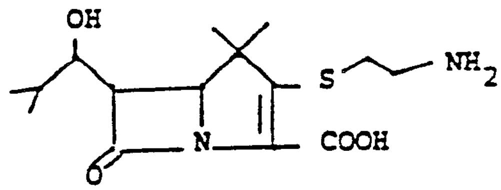

,

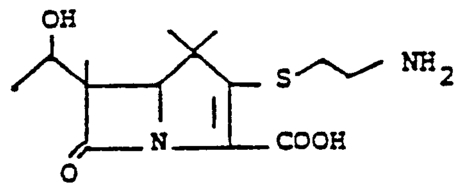

,

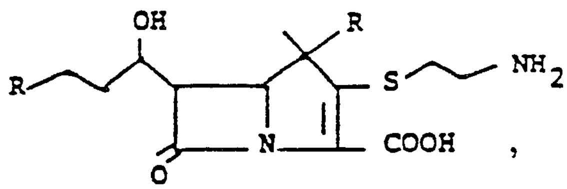

,

(R = Phenyl, m-Aminomethylphenyl, o-, p-, m-Hydroxyphenyl; R' ist Phenyl)

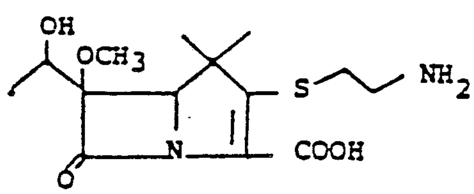

,

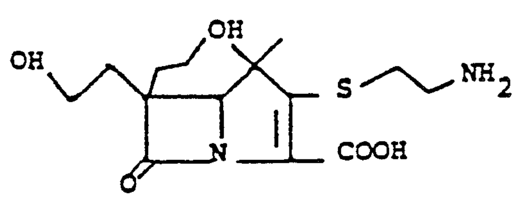

,

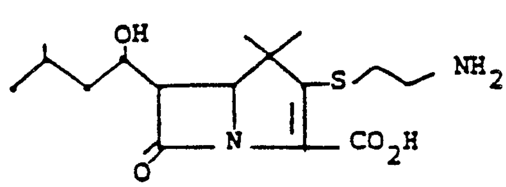

,

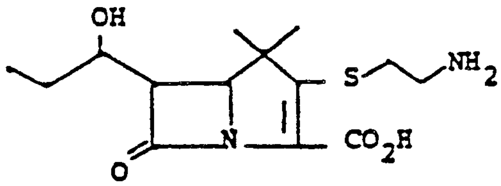
,

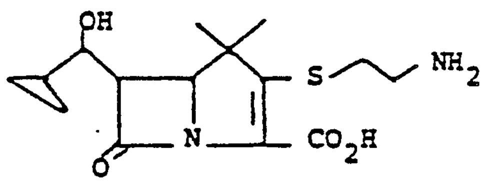
,

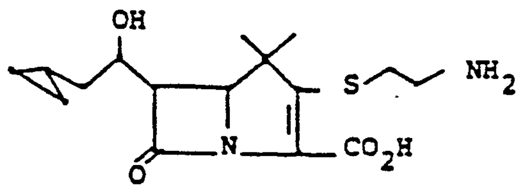
,

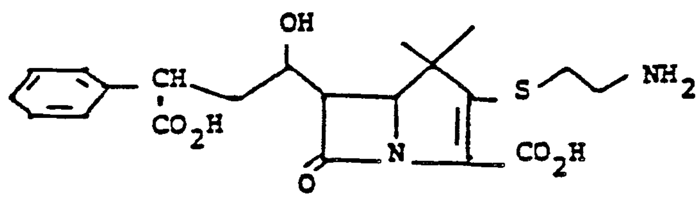
,

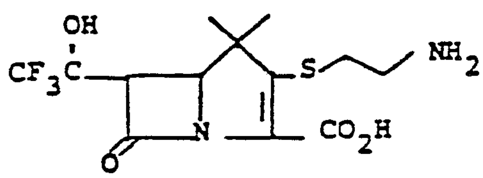
,

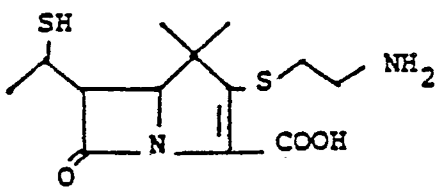
,

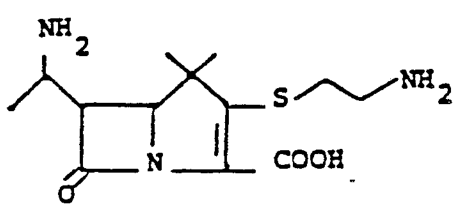
,

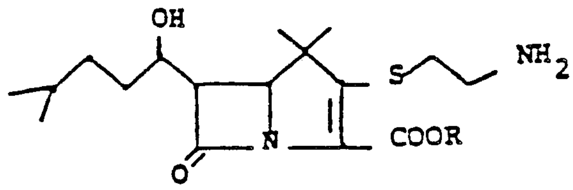

,

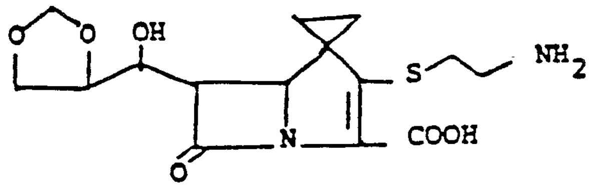

,

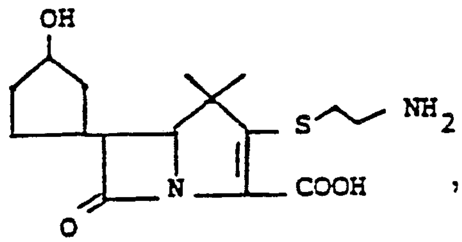

,

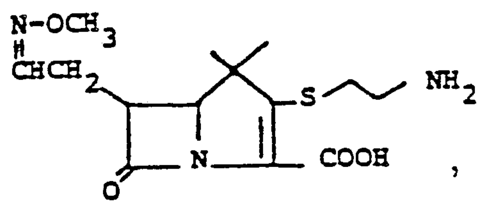

,

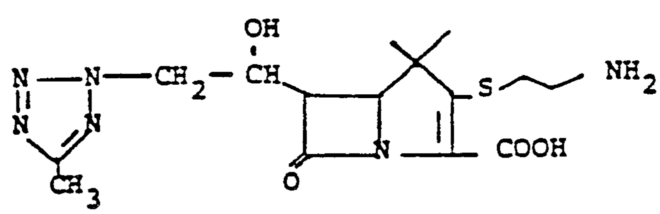

,

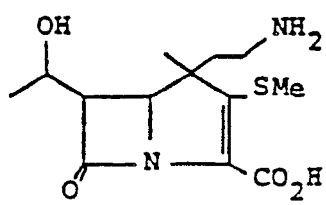

,

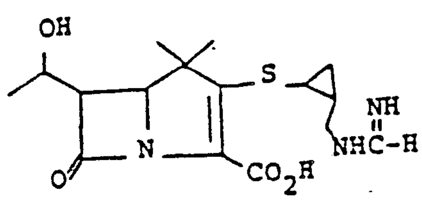

besteht.

7. Eine Verbindung nach Anspruch 6, worin die Aminoethylthioseitenkette,

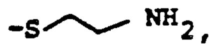

durch ein Glied der Gruppe ersetzt ist, die aus:

$-SCF_3$,

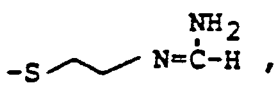

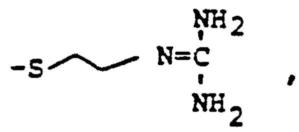

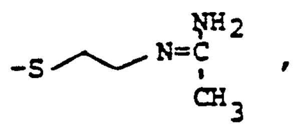

$-S-(CH_2)_n-NH_2$,

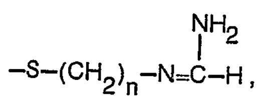

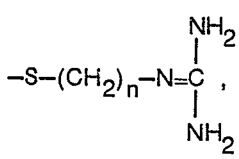

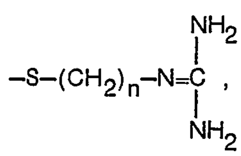

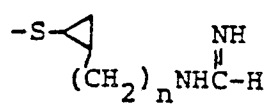

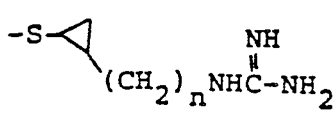

besteht (n = 1, 3, 4, 5 oder 6).

8. Die Verbindung der Formel:

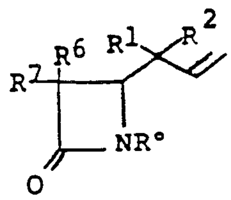

worin R° H oder eine leicht entfernbare Schutzgruppe ist; und worin $R^1$, $R^2$, $R^6$ und $R^7$ wie in Anspruch 1 definiert sind.

9. Die Verbindung des Anspruchs 8, worin R° Triniedrigalkylsilyl ist, wobei die Alkylreste 1 bis 6 Kohlenstoffatome haben.

10. Eine Verbindung der Formel:

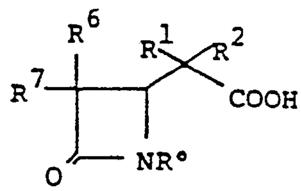

worin R° Wasserstoff oder eine leicht entfernbare Schutzgruppe ist; und worin $R^7$, $R^6$, $R^1$ und $R^2$ wie in Anspruch 1 definiert sind.

11. Eine Verbindung der Formel:

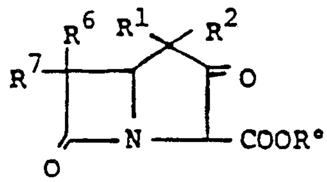

worin R° Wasserstoff, ein Salzkation, ein pharmazeutisch annehmbarer Esterrest oder eine leicht entfernbare Schutzgruppe ist; und worin $R^7$, $R^6$, $R^1$ und $R^2$ wie in Anspruch 1 definiert sind.

12. Ein Verfahren zur Herstellung von:

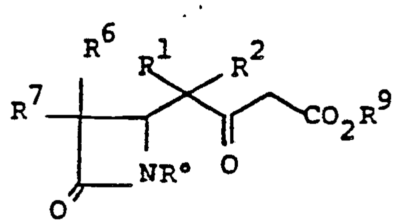

umfassend das Oxidieren von:

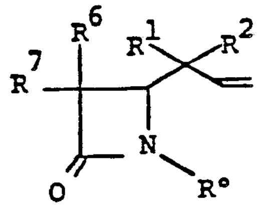

unter Bildung von:

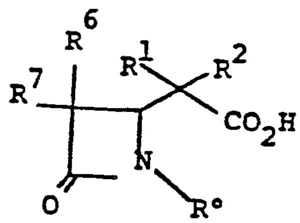

gefolgt vom Behandeln mit $R^9O_2CCH_2CO_2^{\ominus}$; worin $R^9$ ein pharmazeutisch annehmbarer Esterrest oder eine leicht entfernbare Schutzgruppe ist, und worin $R^1$, $R^2$, $R^6$ und $R^7$ wie in Anspruch 1 definiert sind.

13. Das Verfahren des Anspruchs 12, worin $R^9O_2CCH_2CO_2^{\ominus}$ als $(R^9O_2CCH_2CO_2)_2Mg$ eingesetzt wird.

14. Ein Verfahren zur Herstellung einer Verbindung von Anspruch 1

a) mit der Struktur, in der A $SR^8$ ist, und der pharmazeutisch annehmbaren Salze und Ester davon, umfassend die Stufen des Acylierens oder Halogenierens und dann des Behandelns einer Verbindung der Formel

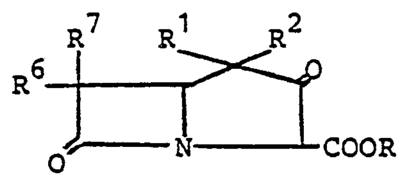

mit dem Thioreagens $HSR^8$, worin $R^6$, $R^7$, $R^1$, $R^2$ und $R^8$ durch den unabhängigen Anspruch 1 definiert sind, und R ein pharmazeutisch annehmbarer Esterrest oder eine leicht entfernbare Carboxylschutzgruppe ist; oder

b) mit der Struktur, worin A $R^8$ ist, umfassend das Oxidieren von:

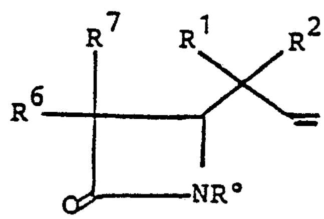

unter Bildung von

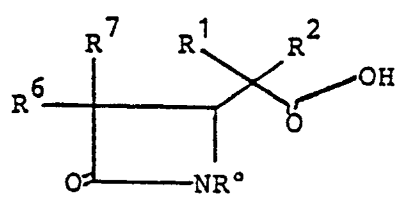

gefolgt von der Umsetzung mit Dicyclohexylcarbodiimid, Ethylchlorformiat, 2-Fluorpyridin, 1-Fluor-2,4-dinitrobenzol, Thionylchlorid oder Oxalylchlorid, und der Behandlung mit $R_2^8$ Cu MgX°, $R^8$ MgX°, Li Cu $R_2^8$ oder $R_2^8$Cd, worin X° Brom oder Chlor ist, um $R^8$ unter Bildung von

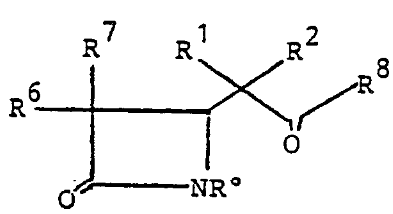

einzuführen, gefolgt von dem Abspalten der N-Schutzgruppe und dem Behandeln mit einem Glyoxylat unter Bildung von

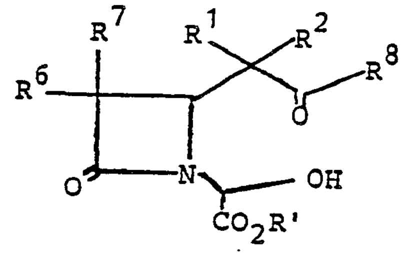

gefolgt von dem Halogenieren mit einem üblichen Halogenierungsmittel und dem Behandeln mit einem Triorganophosphin unter Bildung von

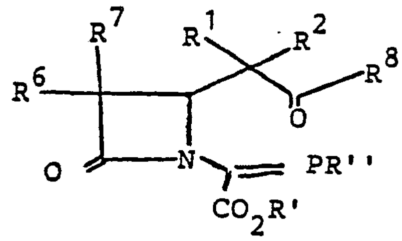

gefolgt vom Cyclisieren und Schutzgruppenabspalten; wobei R° eine leicht entfernbare Schutzgruppe ist; R′ eine leicht entfernbare Schutzgruppe oder ein pharmazeutisch annehmbarer Esterrest ist; und R″ Alkyl mit bis zu 4 Kohlenstoffatomen oder Phenyl ist.

15. Eine antibiotische, pharmazeutische Zusammensetzung, enthaltend eine therapeutisch wirksame Menge einer Verbindung nach Anspruch 1 und einen pharmazeutischen Träger dafür.

16. Eine Verbindung nach Anspruch 1b, worin $R^1$ und $R^2$ Alkyl mit 1 bis 6 Kohlenstoffatomen, Spirocyclopropyl, Benzyl oder Phenyl sind; $R^6$ Wasserstoff ist; und $R^7$ substituiertes oder unsubstituiertes: Alkyl mit 1 bis 6 Kohlenstoffatomen oder Phenylalkyl ist; worin der Substituent OH oder $NH_2$ ist; und $R^8$ aus:

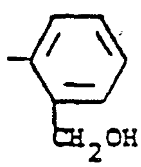

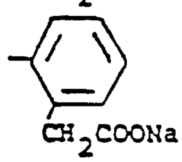

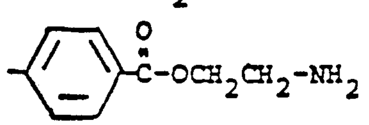

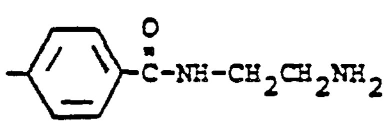

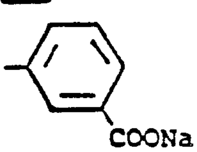

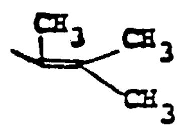

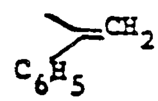

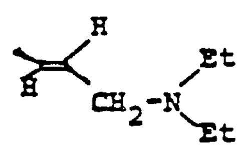

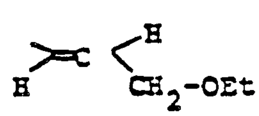

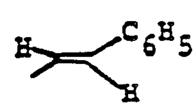

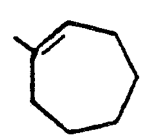

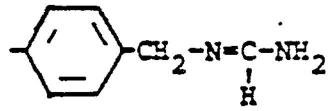

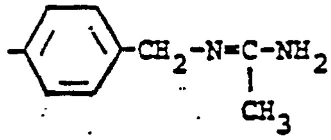

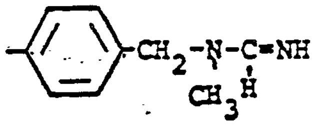

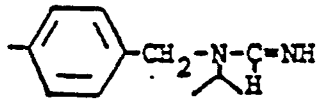

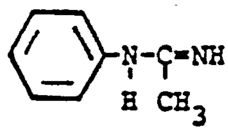

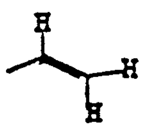

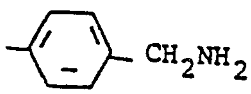

$-C\equiv CH$

$-C\equiv CCH_2NH_2$

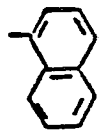

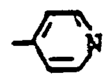

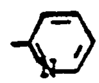

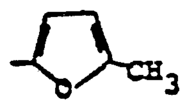

ausgewählt ist.

17. Eine Verbindung nach Anspruch 16, worin $R^1$ und $R^2$ Methyl, Ethyl, Isopropyl, Cyclopropyl, tert.Butyl oder Phenyl sind; $R^7$ 1-Hydroxyethyl, Methyl oder Hydroxymethyl ist; und $R^6$ Wasserstoff ist.

18. Eine Verbindung nach den Ansprüchen 1 bis 7, 16 und 17, worin $R^8$ aus der Gruppe ausgewählt ist, die aus Wasserstoff,

$-CH_2CH_2CH_2NH_2$, $-CH_2CH_2NH_2$, $-CH_3$, $-CH_2CH_2CH_2OH$, $-CH_2CH_2COOH$,

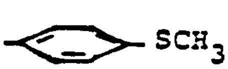 , $-CH_2CH{=}CH{-}SCH_3$, 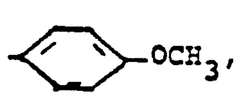,

$-CH{=}CHCH_2CH_2NH_2$, $-CH_2CH_2CH_2CH_2NH_2$, 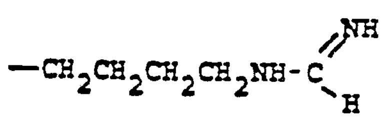 ;

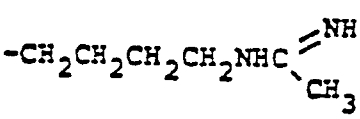 , 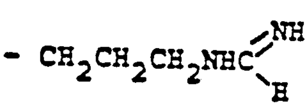 ,

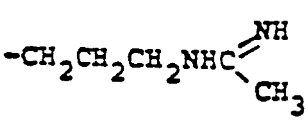 , 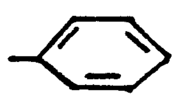 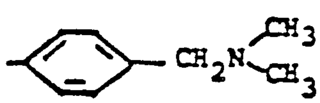 ,

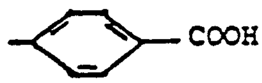 , 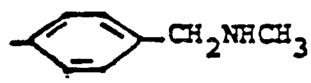 , 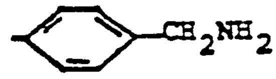 ,

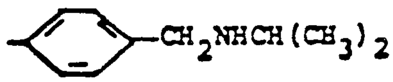 , 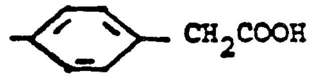 ,

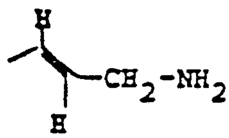

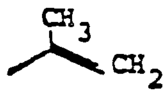

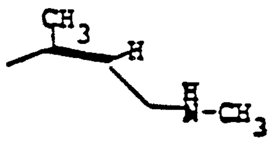

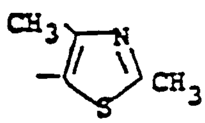

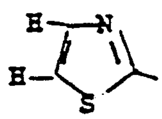

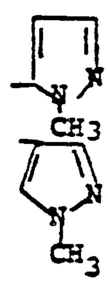

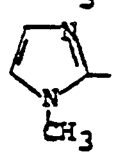

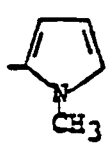

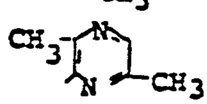

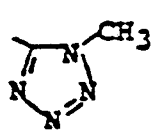

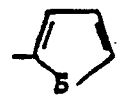

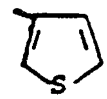

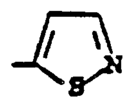

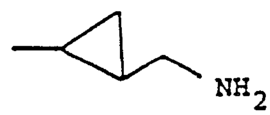

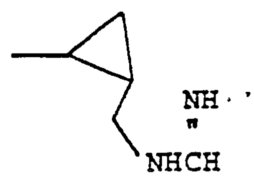

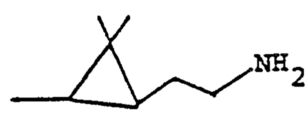

besteht.

19. Eine Verbindung nach Anspruch 18, worin $R^1$ und $R^2$ Alkyl mit 1 bis 6 Kohlenstoffatomen, Phenyl, Cyclopropyl oder Spirocyclopropyl sind.

20. Eine Verbindung nach Anspruch 1 b), ausgewählt aus:

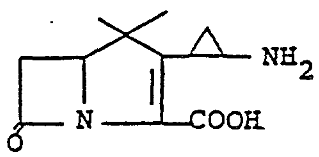

,

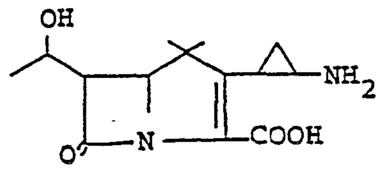

,

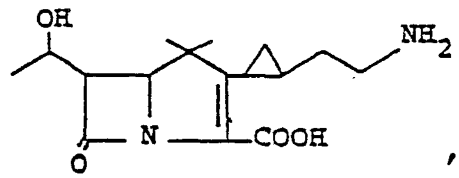

,

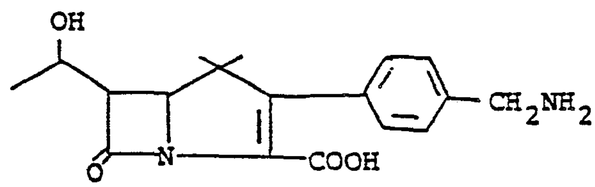

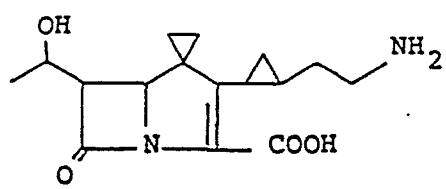

,

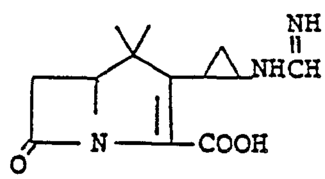

,

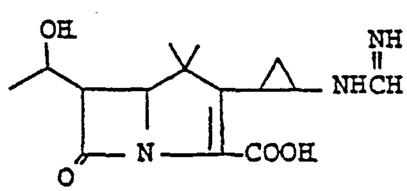

,

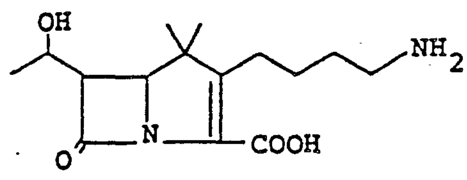

21. Ein Verfahren zur Herstellung einer Verbindung nach Anspruch 1 b), umfassend das Cyclisieren von:

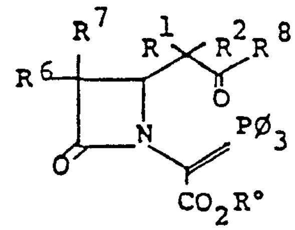

worin ϕ Phenyl ist, und R° ein pharmazeutisch annehmbarer Esterrest oder eine leicht entfernbare Schutzgruppe ist.

22. Eine antibiotische, pharmazeutische Zusammensetzung, enthaltend eine therapeutisch wirksame Menge einer Verbindung nach den Ansprüchen 1 b) und 16 bis 20, und einen pharmazeutischen Träger dafür.

23. Eine Verbindung mit der Formel:

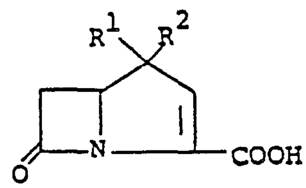

und ihre pharmazeutisch annehmbaren Salze und Ester, worin $R^1$ und $R^2$ aus der Gruppe ausgewählt sind, die aus substituiertem und unsubstituiertem: Niedrigalkyl mit 1 bis 6 Kohlenstoffatomen, Phenyl, Phenylniedrigalkyl, Cycloalkyl mit 3 bis 6 Kohlenstoffatomen, Cycloalkylalkyl mit 1 bis 7 Kohlenstoffatomen in der Kette und 3 bis 6 Kohlenstoffatomen im Ring, und Spirocycloalkyl mit 3 bis 6 Kohlenstoffatomen besteht; wobei die genannten Substituenten an $R^1$ und $R^2$ aus der Gruppe ausgewählt sind, die aus Halogen, Hydroxyl, Amino und substituiertem Amino, Azido, Cyano, Carboxyl, Alkoxyl und Mono-, Di- und Trialkylamino besteht, wobei jeder Alkylrest der vorstehenden Gruppen 1 bis 6 Kohlenstoffatome aufweist.

24. Eine Verbindung nach Anspruch 23, worin $R^1$ und $R^2$ aus Methyl, Phenyl, Ethyl, Cyclopropyl, Propyl, Isopropyl und Spirocyclopropyl ausgewählt sind.

25. Eine Verbindung mit der Struktur:

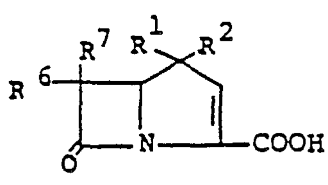

und die pharmazeutisch annehmbaren Salze und Ester davon; worin $R^1$, $R^2$, $R^6$ und $R^7$ unabhängig voneinander aus der Gruppe ausgewählt sind, die aus Wasserstoff, und substituiertem und unsubstituiertem: Alkyl mit 1 bis 6 Kohlenstoffatomen; Phenyl; Phenylalkyl, wobei der Alkylrest 1 bis 6 Kohlenstoffatome hat; Cycloalkyl und Cycloalkylalkyl mit 3 bis 6 Ringkohlenstoffatomen und 1 bis 6 Kohlenstoffatomen im Alkylrest; und Spirocycloalkyl mit 3 bis 6 Kohlenstoffatomen besteht; wobei der Substituent oder die Substituenten an $R^1$, $R^2$, $R^6$ und $R^7$ aus Chlor, Brom, Fluor, Hydroxyl, Amino, Mono-, Di- und Trialkylamino (jedes Alkyl hat 1 bis 6 Kohlenstoffatome), Alkoxyl mit 1 bis 6 Kohlenstoffatomen, Cyano und Carboxyl ausgewählt sind; und wobei $R^1$ und $R^2$ nicht Wasserstoff sind.

26. Eine Verbindung nach Anspruch 25, worin $R^1$ und $R^2$ aus: Alkyl, Cyclopropyl, Spirocyclopropyl, Benzyl und Phenyl ausgewählt sind; $R^6$ Alkyl und Phenylalkyl ist, das durch Hydroxyl oder Amino substituiert ist; und $R^7$ Wasserstoff oder Alkyl oder Phenylalkyl ist, das durch Hydroxyl oder Amino substituiert ist.

27. Eine Verbindung nach Anspruch 26, worin $R^1$ und $R^2$ aus: Methyl, Ethyl, Isopropyl, tert.Butyl, Spirocyclopropyl oder Phenyl ausgewählt sind; $R^6$ 1-Hydroxyethyl, Methyl oder Hydroxymethyl ist; und $R^7$ Wasserstoff ist.

28. Eine Verbindung nach Anspruch 25 mit der Struktur:

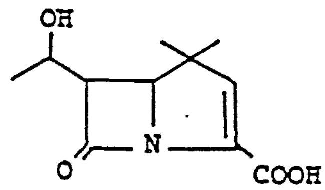

29. Eine Verbindung nach Anspruch 25 mit der Struktur:

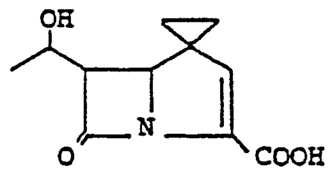

**Patentansprüche für den Vertragsstaat: AT**

1. Ein Verfahren zur Herstellung von:

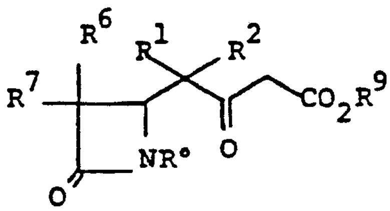

umfassend das Oxidieren von:

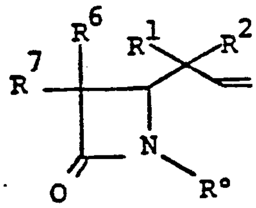

unter Bildung von:

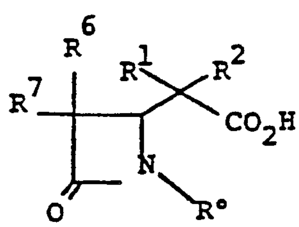

gefolgt vom Behandeln mit $R^9O_2CCH_2CO_2^{\ominus}$; worin $R^1$, $R^2$, $R^6$ und $R^7$ unabhängig voneinander aus der Gruppe ausgewählt sind, die aus substituiertem und unsubstituiertem: Alkyl, Alkenyl und Alkinyl mit 1 bis 10 Kohlenstoffatomen; Cycloalkyl, Cycloalkylalkyl und Alkylcycloalkyl mit 3 bis 6 Kohlenstoffatomen in dem Cycloalkylring und 1 bis 6 Kohlenstoffatomen in den Alkylresten; Spirocycloalkyl mit 3 bis 6 Kohlenstoffatomen; Phenyl; Aralkyl, Aralkenyl und Aralkinyl, wobei der Arylrest Phenyl ist und die Alkylkette 1 bis 6 Kohlenstoffatome hat; Heteroaryl, Heteroaralkyl, Heterocyclyl und Heterocyclylalkyl besteht, wobei der Substituent oder die Substituenten bezüglich der oben genannten Reste aus der Gruppe ausgewählt sind, die aus: Amino, Mono-, Di- und Trialkylamino, Hydroxyl, Alkoxyl, Mercapto, Alkylthio, Phenylthio, Sulfamoyl, Amidino, Guanidino, Nitro, Chlor, Brom, Fluriod, Cyano und Carboxy besteht; und wobei das Heteroatom oder die Heteroatome in den oben genannten heterocyclischen Resten aus der Gruppe ausgewählt sind, die aus 1 bis 4 Sauerstoff-, Stickstoff- oder Schwefelatomen besteht; und wobei die Alkylrest in den oben aufgezählten Substituenten 1 bis 6 Kohlenstoffatome haben; und $R^6$ und $R^7$ auch Wasserstoff bedeuten können; $R^9$ ein pharmazeutisch annehmbarer Esterrest oder eine leicht entfernbare Schutzgruppe ist, und $R^o$ Wasserstoff oder eine leicht entfernbare Schutzgruppe ist.

2. Das Verfahren des Anspruchs 1, worin $R^9O_2CCH_2CO_2^{\ominus}$ als $(R^9O_2CCH_2CO_2)_2Mg$ eingesetzt wird.

3. Ein Verfahren zur Herstellung einer Verbindung mit der Struktur:

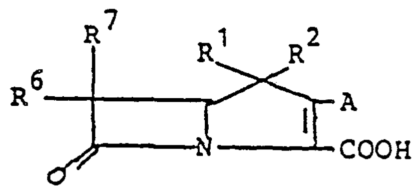

und der pharmazeutisch annehmbaren Salze und Ester davon; worin entweder

a) A für $SR^8$ steht, und $R^1$, $R^2$, $R^6$, $R^7$ und $R^8$ unabhängig voneinander aus der Gruppe ausgewählt sind, die aus substituiertem und unsubstituiertem: Alkyl, Alkenyl und Alkinyl mit 1 bis 10 Kohlenstoffatomen; Cycloalkyl, Cycloalkylalkyl und Alkylcycloalkyl mit 3 bis 6 Kohlenstoffatomen in dem Cycloalkylring und 1 bis 6 Kohlenstoffatomen in den Alkylresten; Spirocycloalkyl mit 3 bis 6 Kohlenstoffatomen; Phenyl; Aralkyl, Aralkenyl und Aralkinyl, wobei der Arylrest Phenyl ist und die Alkylkette 1 bis 6 Kohlenstoffatome hat; Heteroaryl, Heteroaralkyl, Heterocyclyl und Heterocyclylalkyl besteht, wobei der Substituent oder die Substituenten bezüglich der oben genannten Reste aus der Gruppe ausgewählt sind, die aus: Amino, Mono-, Di- und Trialkylamino, Hydroxyl, Alkoxyl, Mercapto, Alkylthio, Phenylthio, Sulfamoyl, Amidino, Guanidino, Nitro, Chlor, Brom, Fluriod, Cyano und Carboxy besteht; und wobei das Heteroatom oder die Heteroatome in den oben genannten heterocyclischen Resten aus der Gruppe ausgewählt sind, die aus 1 bis 4 Sauerstoff-, Stickstoff- oder Schwefelatomen besteht; und wobei die Alkylrest in den oben aufgezählten Substituenten 1 bis 6 Kohlenstoffatome haben; und $R^6$, $R^7$ und $R^8$ auch Wasserstoff bedeuten können; und wobei, falls $R^6/R^7$ Wasserstoff ist und $R^7/R^6$ 1-Hydroxyethyl ist, $R^8$ nicht 2-Aminoethyl oder ein N-Derivat davon ist; umfassend die Stufen des Acylierens oder Halogenierens und dann des Behandelns einer Verbindung der Formel

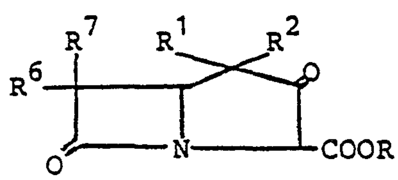

mit dem Thioreagens $HSR^8$, worin $R^6$, $R^7$, $R^1$, $R^2$ und $R^8$ wie oben definiert sind und R ein pharmazeutisch annehmbarer Esterrest oder eine leicht entfernbare Carboxylschutzgruppe ist; oder

b) A für $R^8$ steht, und $R^1$, $R^2$, $R^6$, $R^7$ und $R^8$ unabhängig voneinander aus der Gruppe ausgewählt sind, die aus substituiertem und unsubstituiertem: Alkyl mit 1 bis 6 Kohlenstoffatomen, Aralkyl, Alkenyl und Alkinyl mit 2 bis 6 Kohlenstoffatomen, Aryl und Aralkyl mit 6 bis 10 Ringkohlenstoffatomen und 1 bis 6 Kohlenstoffatomen in der Alkylkette, Heterocyclyl, Heterocyclylthio (ausgeschlossen $R^8$) und Heterocyclylalkyl mit 1 bis 5 aus O, N oder S ausgewählten Heteroatomen im Ring und 1 bis 6 Kohlenstoffatomen in der Alkylkette, Cycloalkyl, Spirocycloalkyl und Cycloalkylalkyl mit 3 bis 6 Ringkohlenstoffatomen und 1 bis 6 Kohlenstoffatomen in dem Alkylrest besteht; wobei die Substituenten an $R^1$, $R^2$, $R^6$, $R^7$ und $R^8$ aus Chlor, Brom, Fluor, Iod, Hydroxyl, Amino, mono-, di- und trialkylsubstituiertem Amino (jedes Alkyl hat 1 bis 6 Kohlenstoffatome), Alkoxyl mit 1 bis 6 Kohlenstoffatomen, Guanidino, Cyano, Amidino und Carboxyl ausgewählt sind; und $R^6$, $R^7$ und $R^8$ auch Wasserstoff bedeuten können; umfassend das Oxidieren von:

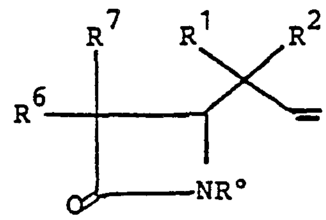

unter Bildung von

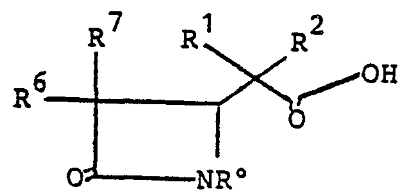

gefolgt von der Umsetzung mit Dicyclohexylcarbodiimid, Ethylchlorformiat, 2-Fluorpyridin, 1-Fluor-2,4-dinitrobenzol, Thionylchlorid oder Oxalylchlorid, und der Behandlung mit $R_2^8$ Cu MgX°, $R^8$ MgX°, Li Cu $R_2^8$ oder $R_2^8$Cd, worin X° Brom oder Chlor ist, um $R^8$ unter Bildung von

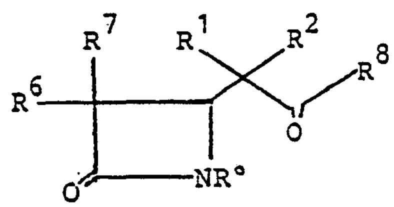

einzuführen, gefolgt von dem Abspalten der N-Schutzgruppe und dem Behandeln mit einem Glyoxylat unter Bildung von

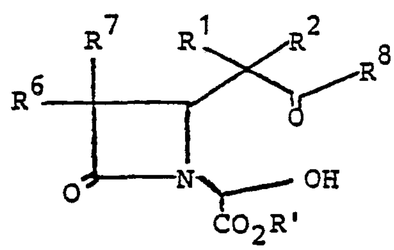

gefolgt von dem Halogenieren mit einem üblichen Halogenierungsmittel und dem Behandeln mit einem Triorganophosphin unter Bildung von

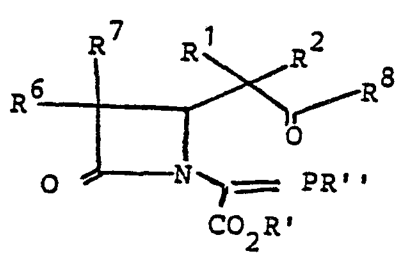

gefolgt vom Cyclisieren und Schutzgruppenabspalten; wobei R° eine leicht entfernbare Schutzgruppe ist; R′ eine leicht entfernbare Schutzgruppe oder ein pharmazeutisch annehmbarer Esterrest ist; und R′′ Alkyl mit bis zu 4 Kohlenstoffatomen oder Phenyl ist.

4. Ein Verfahren zur Herstellung einer Verbindung mit der Struktur

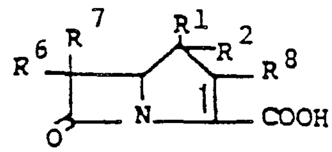

und der pharmazeutisch annehmbaren Salze und Ester davon; worin $R^1$, $R^2$, $R^6$, $R^7$ und $R^8$ unabhängig voneinander wie in Anspruch 1 definiert sind, umfassend das Cyclisieren von

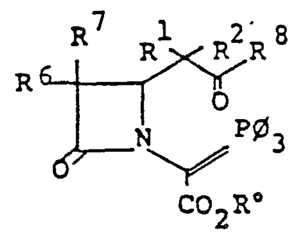

worin ϕ Phenyl ist, und R° ein pharmazeutisch annehmbarer Esterrest oder eine leicht entfernbare Schutzgruppe ist.

**Revendications pour les Etats contractants: BE CH DE FR GB IT LI LU NO SE**

1. Composé ayant la structure.

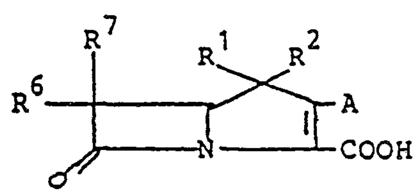

et ses sels et esters pharmaceutiquement acceptables; dans lesquels soit

a) A est $SR^8$ et $R^1$, $R^2$, $R^6$, $R^7$ et $R^8$ sont indépendamment choisis dans le groupe constitué d'alkyle, alcényle et alcynyle substitué et non substitué, ayant de 1 à 10 atomes de carbone; de cycloalkyle, cycloalkylalkyle et alkylcycloalkyle, ayant de 3 à 6 atomes de carbone dans le cycle cycloalkyle et de 1 à 6 atomes de carbone dans les parties alkyles; de spirocycloalkyle ayant de 3 à 6 atomes de carbone; du phényle; d'aralkyle, aralcényle, et aralcynyle dans lesquels la partie aryle est le phényle et la chaîne alkyle a de 1 à 6 atomes de carbone; d'hétéroaryle, hétéroaralkyle, hétérocyclyle et hétérocyclylalkyle dans lesquels le substituant ou les substituants relatifs aux radicaux mentionnés ci-dessus sont choisis dans le groupe constitué de: amino, mono-, di-, et trialkylamino, hydroxyle, alcoxyle, mercapto, alkylthio, phénylthio, sulfamoyle, amidino, guanidino, nitro, chloro, bromo, fluoro, iodo, cyano et carboxy; et dans lesquels le ou les hétéroatomes dans les parties hétérocycliques mentionnées ci-dessus sont choisies dans le groupe constitué de 1 à 4 atomes d'oxygène, d'azote ou de soufre; et dans lesquels les parties alkyles des substituants cités ci-dessus ont de 1 à 6 atomes de carbone et $R^6$, $R^7$ et $R^8$ peuvent aussi représenter l'hydrogène; quand $R^6/R^7$ est l'hydrogène et $R^7/R^6$ est le l'hydroxyéthyle, alors $R^8$ n'est pas le 2-aminoéthyle ou un de ses dérivés azoté, soit

b) A est $R^8$ et $R^1$, $R^2$, $R^6$, $R^7$ et $R^8$ sont choisis indépendamment dans le groupe constitué de radicaux substitués ou non: alkyle ayant de 1 à 6 atomes de carbone, aralkyle, alcényle et alcynyle ayant de 2 à 6 atomes de carbone, aryle et aralkyle ayant de 6 à 10 atomes de carbone dans le cycle et de 1 à 6 atomes de carbone dans la chaîne alkyle, hétérocyclyle, hétérocyclylthio ($R^8$ est exclu) et hétérocyclylalkyle ayant de 1 à 5 hétéroatomes dans le cycle choisis parmi O, N ou S et de 1 à 6 atomes de carbone dans la chaîne alkyle, cycloalkyle, spirocycloalkyle, et cycloalkylalkyle ayant de 3 à 6 atomes de carbone dans le cycle et de 1 à 6 atomes dans la partie alkyle; dans lesquels les substituants sur $R^1$, $R^2$, $R^6$, $R^7$ et $R^8$ sont choisis parmi les chloro, bromo, fluoro, iodo, hydroxyle, amino, mono-, di, et trialkyl-amino substitué, (chaque alkyle ayant de 1 à 6 atomes de carbone) alcoxyle ayant de 1 à 6 atomes de carbone, guanidino, cyano, amidino et carboxyle; $R^6$, $R^7$ et $R^8$ peuvent aussi représenter l'hydrogène.

2. Composé selon la revendication 1 a) dans lequel $R^1$ et $R^2$ sont choisis parmi les: alkyle ayant de 1 à 10 atomes de carbone, cycloalkyle ayant de 3 à 6 atomes de carbone, spirocycloalkyle ayant de 3 à 6 atomes de carbone, benzyle ou phényle; et $R^6$ est H ou méthyle et $R^7$ est alkyle ayant de 1 à 10 atomes de carbone, phényle, aralkyle dans lequel la partie aryle est phényle et la chaîne alkyle a 1—6 atomes de carbone ou alkyle hydroxyle substitué ayant de 1 à 10 atomes de carbone, phényle, hydroxyle substitué ou aralkyle, hydroxyle substitué dans lequel la partie aryle est phényle et la chaîne alkyle a de 1 à 6 atomes de carbone.

3. Composé selon la revendication 2 dans lequel $R^1$ et $R^2$ sont choisis parmi le spirocyclopropyle, méthyle, éthyle, isopropyle, t-butyle ou phényle et $R^7$ est 1-hydroxyéthyle, méthyle ou hydroxyméthyle.

4. Composé selon la revendication 1 a) dans lequel $R^8$ est choisi dans le groupe constitué de:

H,

$CH_3$,

$(CH_2)_2NH_2$,

$C(CH_3)_2CH_2NH_2$,

$C(CH_3)_2CH_2NH{-}C({=}NH){-}H$,

$C(CH_3)_2CH_2NH{-}C({=}NH){-}CH_3$,

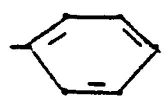
,

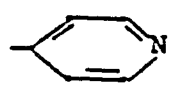
,

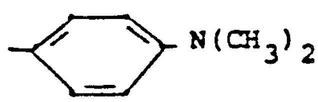

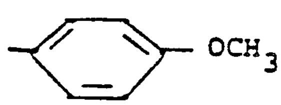

$CH_2CH_2CH_2NH_2$,

$CH_2CH(CH_3)NH_2$,

$CH_2CH_2CH_2NH{-}C({=}NH){-}H$,

$CH_2CH_2CH_2NH{-}C({=}NH){-}CH_3$,

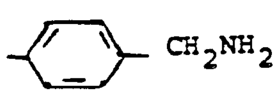

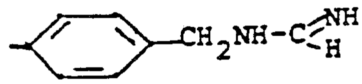

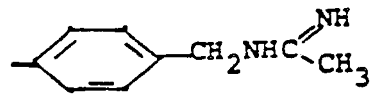

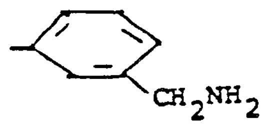

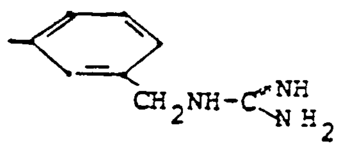

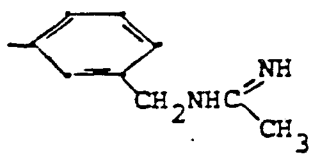

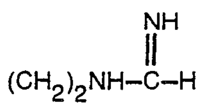

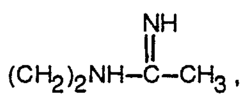

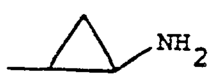

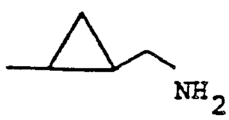

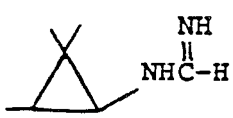

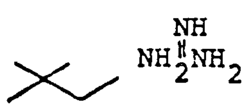

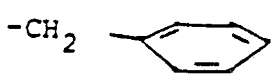

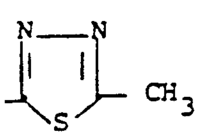

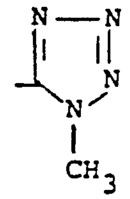

$CH(CH_3)CH_2NH_2$,

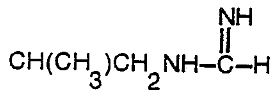 ou

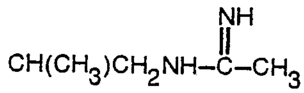

5. Composé selon la revendication 4 dans lequel $R^6$ est H ou méthyle; $R^7$ est choisi parmi:

$-CH_2OH$

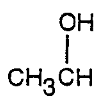

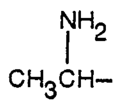

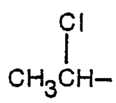

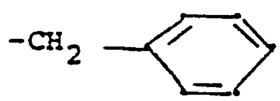

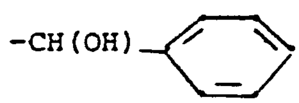

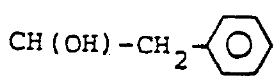

$-CH_3$

$-CH_2CH_3$

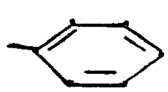 ;

et $R^1$ et $R^2$ sont choisis parmi le spirocyclopropyle; phényle; cycloalkyle ayant 3—6 atomes de carbone; alkyle ayant 1—6 atomes de carbone; cyclopropylalkyle ayant 4—9 atomes de carbone.

6. Composé selon la revendication 1 choisi dans le groupe constitué de:

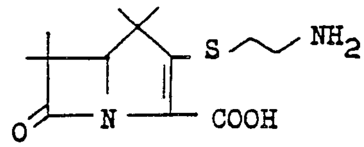

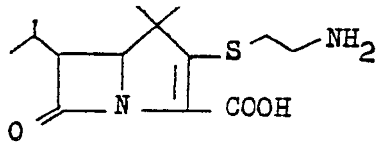

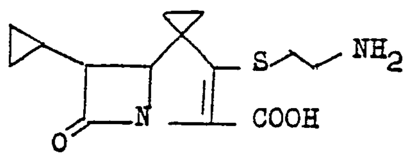

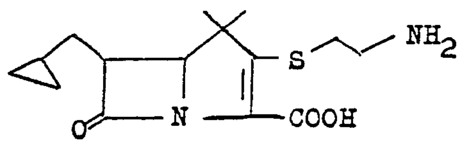

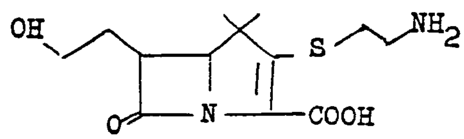

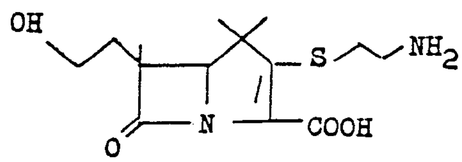

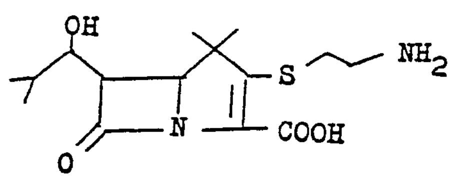

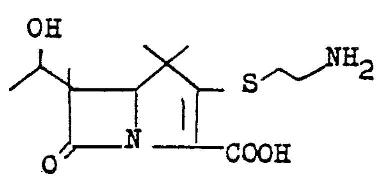

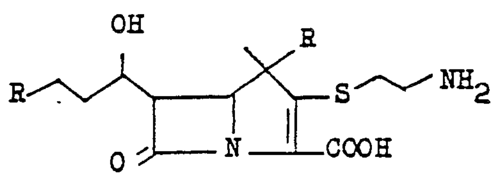

(R = phényle, m-aminométhylphényle, o-, p, m-hydroxyphényle; R' est phényle)

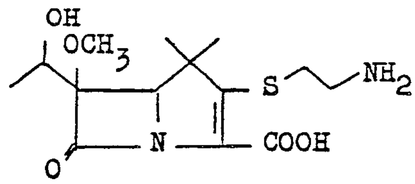

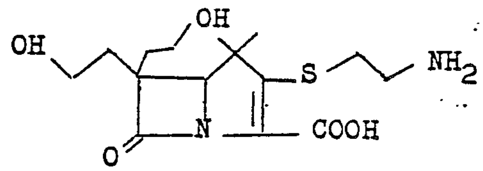

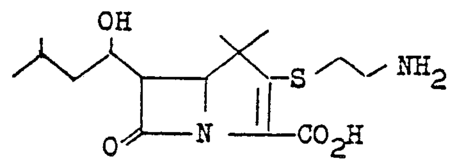

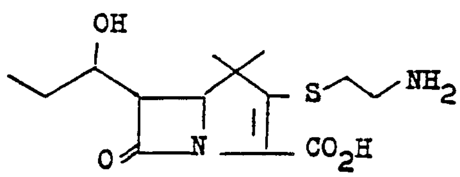

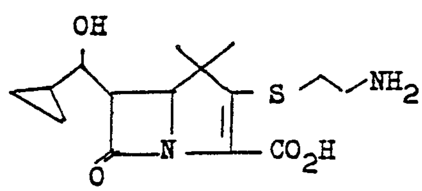

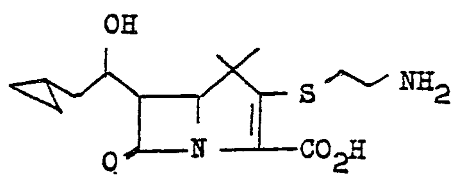

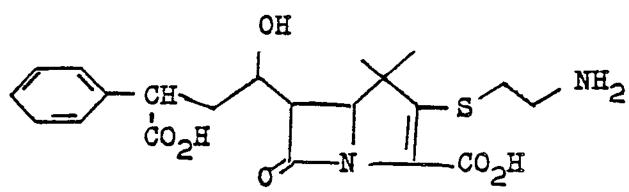

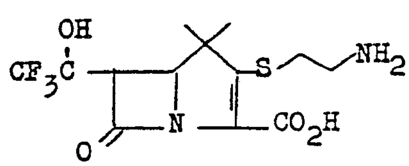

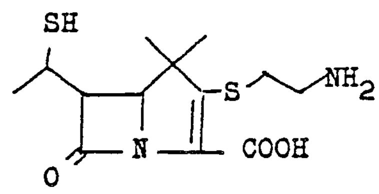

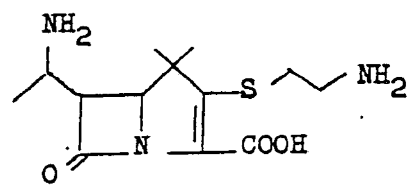

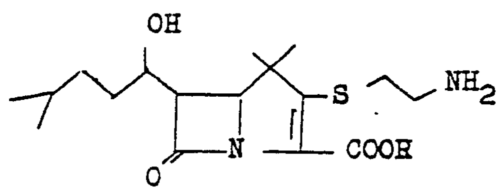

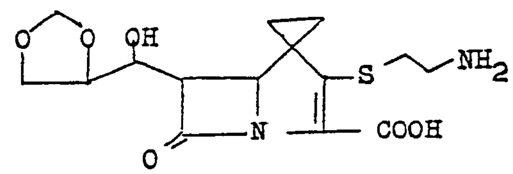

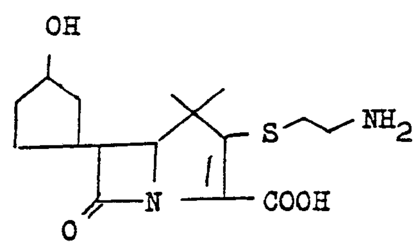

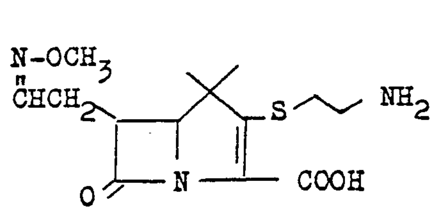

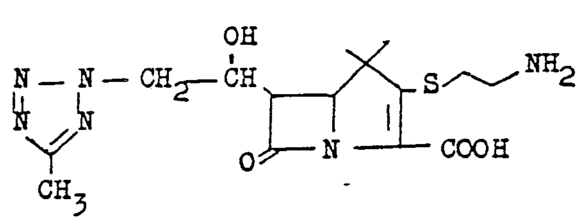

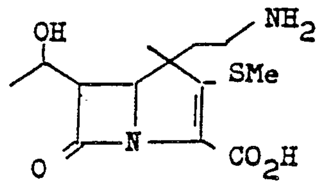

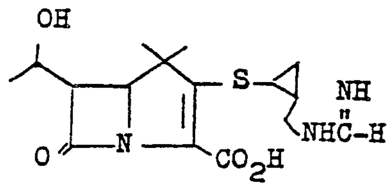

7. Composé selon la revendication 6, dans lequel le chaîne latérale,

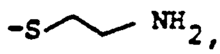,

est remplacée par un membre du groupe constitué de:

$-SCF_3$,

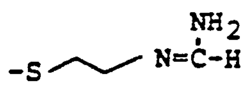,

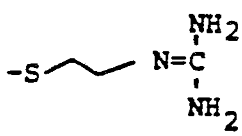,

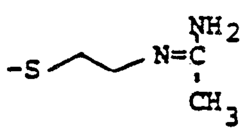,

$-S-(CH_2)_n-NH_2$,

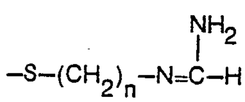,

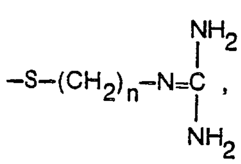,

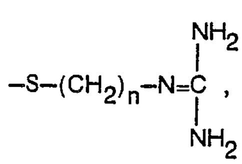,

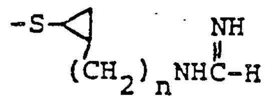

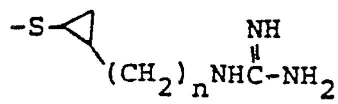

n = 1, 3, 4, 5 ou 6.

8. Composé de formule:

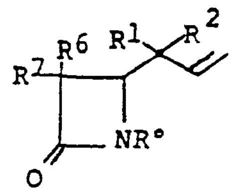

dans lequel R° est H ou un groupe protecteur facile à éliminer; et dans lequel $R^1$, $R^2$, $R^6$ et $R^7$ sont comme défini dans la revendication 1.

9. Composé selon la revendication 8 dans lequel R° est un tri(alkyl inférieur)silyle, dans lequel les parties alkyles ont de 1 à 6 atomes de carbone.

10. Composé de formule:

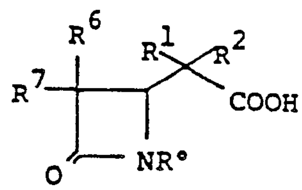

dans lequel R° est l'hydrogène ou un groupe protecteur facile à éliminer; et dans lequel $R^7$, $R^6$, $R^1$ et $R^2$ sont comme définis dans la revendication 1.

11. Composé de formule

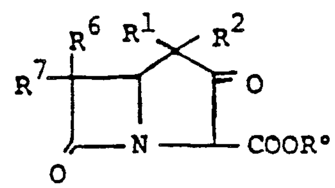

dans lequel R° est l'hydrogène, un sel cationique ou une partie ester pharmaceutiquement acceptable, ou un groupe protecteur facilement éliminable; et dans lequel $R^7$, $R^6$, $R^1$ et $R^2$ sont comme définis dans la revendication 1.

12. Procédé de préparation

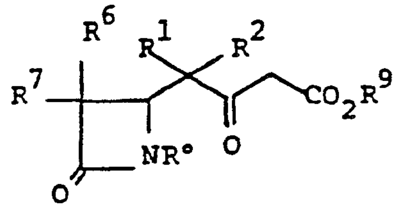

comprenant: l'oxydation de

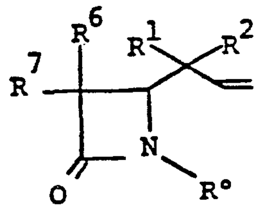

pour former

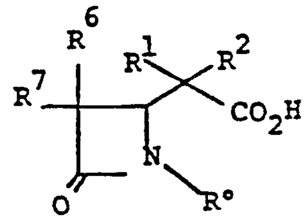

suivi du traitement avec $R^9O_2CCH_2CO_2^{\ominus}$; dans lequel $R^9$ est une partie ester pharmaceutiquement acceptable ou un groupe protecteur facilement éliminable et dans lequel $R^1$, $R^2$, $R^6$, et $R^7$ sont comme définis dans la revendication 1.

13. Procédé de la revendication 12 dans lequel $R^9O_2CCH_2CO_2^{\ominus}$ est utilisé sous forme de $(R^9O_2CCH_2CO_2)_2Mg$.

14. Procédé de préparation d'un composé de la revendication 1

a) ayant la structure dans laquelle A est $SR^8$ et ses sels et esters pharmaceutiquement acceptables, comprenant les étapes d'acylation ou halogénation et ensuite de traitement avec le thioréactif $HSR^8$ d'un composé de formule

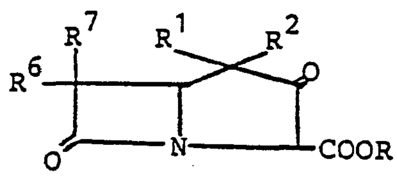

dans lequel $R^6$, $R^7$, $R^1$, $R^2$ et $R^8$ sont définis pour la revendication 1 indépendante et R est une partie ester pharmaceutiquement acceptable ou un groupe protecteur du carboxyle et facile à éliminer; ou

b) ayant la structure dans laquelle A est $R^8$ comprenant l'oxydation:

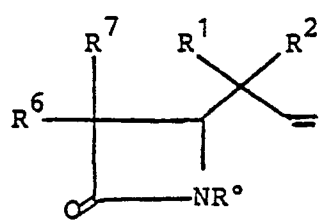

pour former

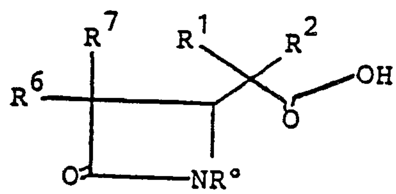

suivi d'une réaction avec du dicyclohexylcarbodiimide, éthylchloroformate, 2-fluoropyridine, 1-fluoro-2,4-dinitrobenzène, chlorure de thionyle ou chlorure d'oxalyle et en traitant avec $R_2^8$ CuMgX°, $R^8$MgX°, LiCu$R_2^8$ ou $R_2^8$Cd dans lesquels X° est bromo ou chloro pour fixer R

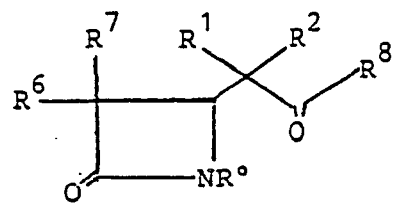

suivi par l'élimination de la protection de N et d'un traitement avec un glyoxylate pour donner

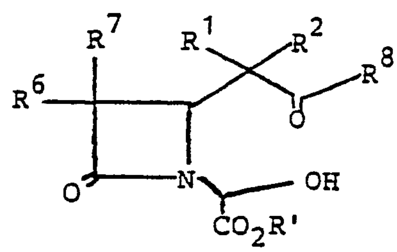

suivi par l'halogénation par un agent d'halogénation conventionnel et du traitement avec une triorganophosphine pour former

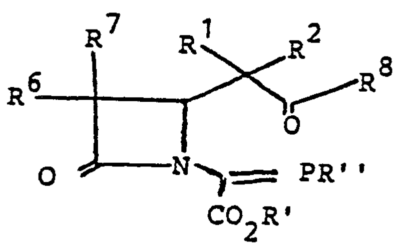

suivi de la cyclisation et de l'élimination de la protection, R° étant dans ce composé un groupe protecteur facile à éliminer; R' est un groupe protecteur facile à éliminer ou une partie ester pharmaceutiquement acceptable; et R" est un alkyle ayant jusqu'à 4 atomes de carbone ou un phényle.

15. Composition pharmaceutique antibiotique comprenant une quantité thérapeutiquement efficace d'un composé selon la revendication 1 et son véhicule pharmaceutique.

16. Composé selon la revendication 1b dans lequel $R^1$ et $R^2$ sont des: alkyle, ayant de 1 à 6 atomes de carbone, spirocyclopropyle, benzyle ou phényle; $R^6$ est l'hydrogène et $R^7$ est substitué ou non substitué: alkyle ayant de 1 à 6 atomes de carbone ou phénylalkyle; dans lequel le substituant est OH ou $NH_2$; et $R^8$ est choisi parmi:

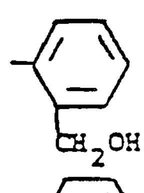

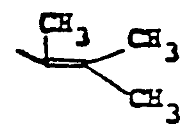

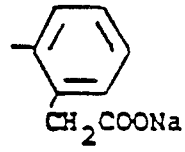

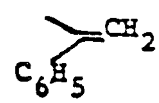

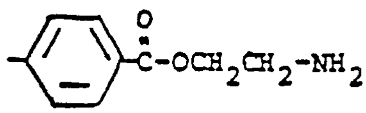

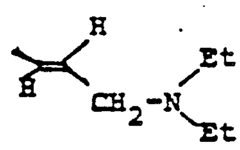

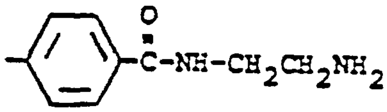

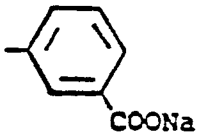

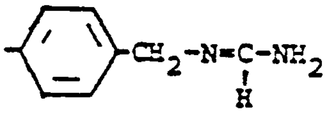

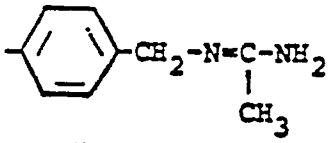

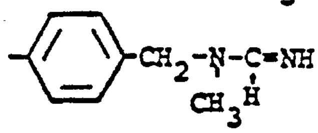

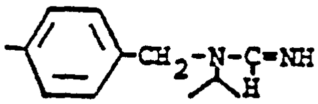

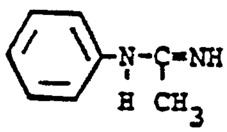

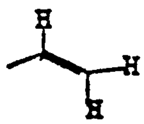

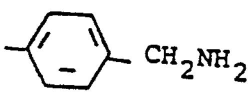

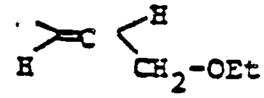

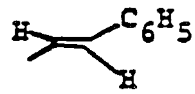

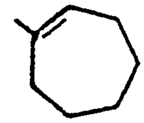

$-C\equiv CH$

$-C\equiv CCH_2NH_2$

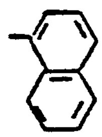

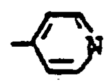

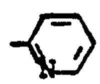

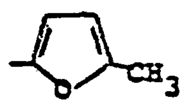

17. Composé selon la revendication 16 dans lequel $R^1$ et $R^2$ sont le méthyle, éthyle, isopropyle, cyclopropyle, t-butyle ou phényle; $R^7$ est le 1-hydroxyéthyle, méthyle ou hydroxyméthyle; et $R^6$ est l'hydrogène.

18. Composé selon les revendications 1 à 7, 16 et 17 dans lequel $R^8$ est choisi dans le groupe constitué de:

hydrogène, $-CH_2CH_2CH_2NH_2$, $-CH_2CH_2NH_2$, $-CH_3$, $-CH_2CH_2CH_2OH$, $-CH_2CH_2COOH$,

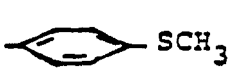 , $-CH_2CH=CH-SCH_3$, 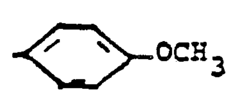,

$-CH=CHCH_2CH_2NH_2$, $-CH_2CH_2CH_2CH_2NH_2$, 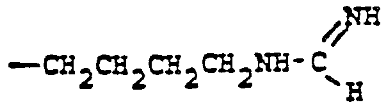 ;

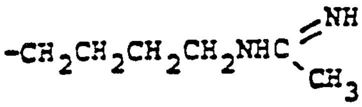 , 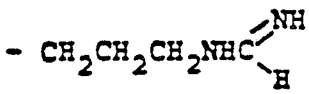 ,

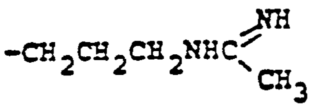 , 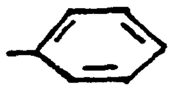 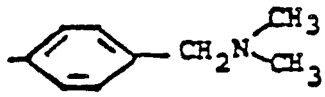 ,

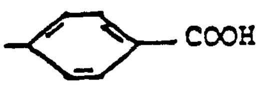 , 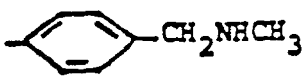 , 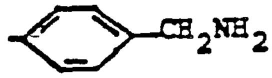 ,

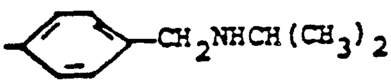 , 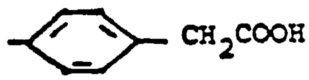 ,

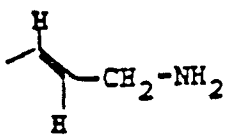 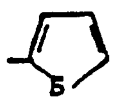

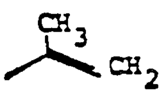 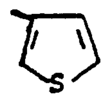

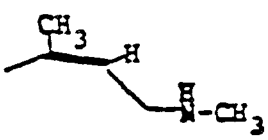 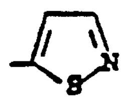

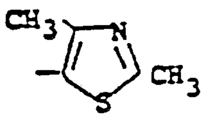 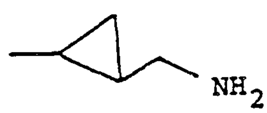

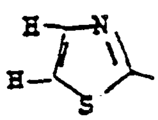

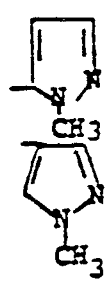 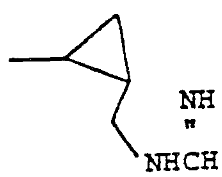

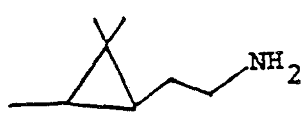

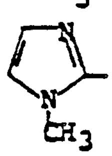

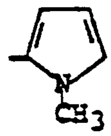

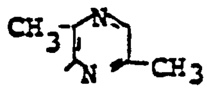

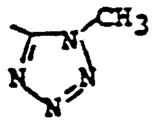

19. Composé selon la revendication 18 dans lequel $R^1$ et $R^2$ sont des: alkyle ayant de 1 à 6 atomes de carbone, phényle, cyclopropyle, ou spirocyclopropyle.

20. Composé selon la revendication 1b choisi parmi:

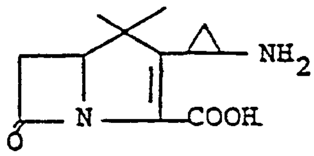
,

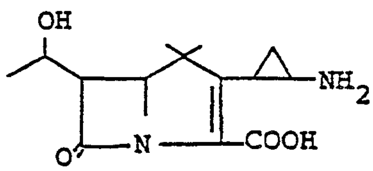
,

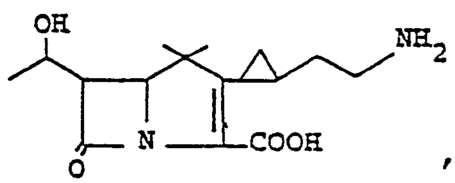
,

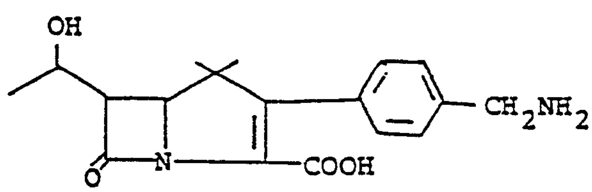

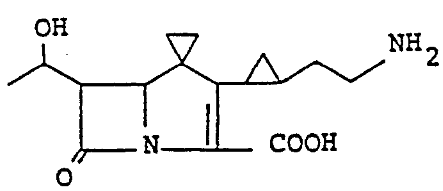
,

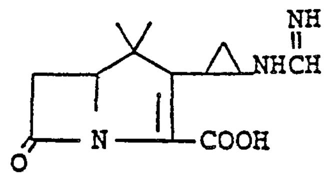

,

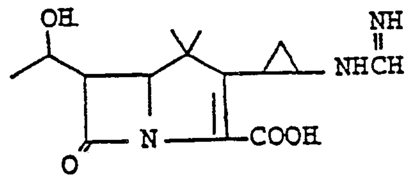

,

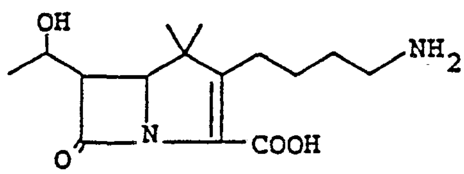

21. Procédé de préparation d'un composé selon la revendication 1b comprenant la cyclisation:

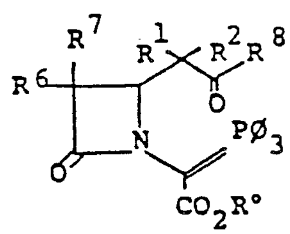

dans lequel ϕ est le phényle, et R° est une partie ester pharmaceutiquement acceptable ou un groupe de blocage facile à éliminer.

22. Composition pharmaceutique antibiotique comprenant une quantité thérapeutiquement efficace d'un composé selon les revendications 1b) et 16—20 et son véhicule pharmaceutique.

23. Composé ayant la formule

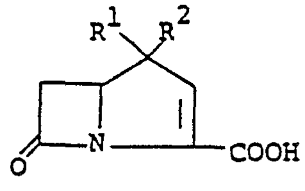

et ses sels et esters pharmaceutiquement acceptables dans lequel $R^1$ et $R^2$ sont choisis parmi le groupe constitué de radicaux subtitués et non substitutés: alkyle, phénylalkyle inférieur, cycloalkyle ayant de 3 à 6 atomes de carbone, cycloalkyle ayant de 1 à 7 atomes de carbone dans la chaîne et 3 à 6 atomes dans le mes de carbone dans la chaîne et 3 à 6 atomes dans le cycle et spirocycloalkyle ayant de 3 à 6 atomes de carbone; dans lequel ces substituants sur $R^1$ et $R^2$ sont choisis parmi le groupe constitué d'halogène, hydroxyle, amino et amino substitué, azido, cyano, carboxyle, alcoxyle, et mono-, di- et trialkylamino, chaque radical alkyle précédent ayant de 1 à 6 atomes de carbone.

24. Composé selon la revendication 23 dans lequel $R^1$ et $R^2$ sont choisis parmi les méthyle, phényle, éthyle, cyclopropyle, propyle, isopropyle, et spirocyclopropyle.

25. Composé ayant la structure

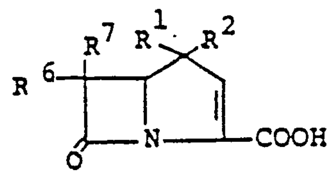

et ses sels et esters pharmaceutiquement acceptables; dans lequel $R^1$, $R^2$, $R^6$ et $R^7$ sont choisis indépendamment dans le groupe constitué de l'hydrogène, et des radicaux substitués et non substitués: alkyle ayant 1—6 atomes de carbone; phényle; phénylalkyle dans lequel la partie alkyle a 1—6 atomes de carbone; cycloalkyle et cycloalkylalkyle ayant de 3 à 6 atomes de carbone dans le cycle et 1 à 6 atomes de carbone dans la partie alkyle; et spirocycloalkyle ayant de 3 à 6 atomes de carbone; dans lequel le substituant ou les substituants sur $R^1$, $R^2$, $R^6$ et $R^7$ sont choisis parmi les chloro, bromo, fluoro, hydroxyle, amino, mono-, di- et trialkylamino (chaque alkyle ayant de 1 à 6 atomes de carbone), alcoxyle ayant de 1 à 6 atomes de carbone, cyano et carboxyle; dans lequel $R^1$ et $R^2$ ne sont pas l'hydrogène.

26. Composé selon la revendication 25 dans lequel $R^1$ et $R^2$ sont choisi parmi les: alkyle, cyclopropyle, spirocyclopropyle, et benzyle et phényle; $R^6$ est alkyle et phénylalkyl substitué par un hydroxyle ou amino; et $R^7$ est l'hydrogène ou un alkyle ou phénylalkyle substitué par un hydroxyle ou amino.

27. Composé selon la revendication 26 dans lequel $R^1$ et $R^2$ sont choisi parmi les: méthyle, éthyle, isopropyle, t-butyle, spirocyclopropyle, ou phényle; $R^6$ est le 1-hydroxyéthyle, méthyle ou hydroxyméthyle; et $R^7$ est l'hydrogène.

28. Composé selon la revendication 25 ayant la structure

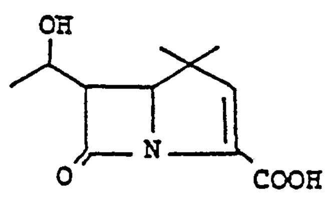

29. Composé selon la revendication 25 ayant la structure

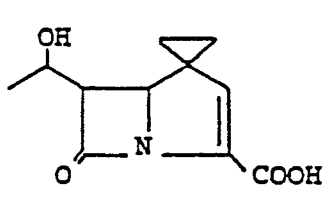

**Revendications pour l'Etat contractant: AT**

1. Procédé de préparation:

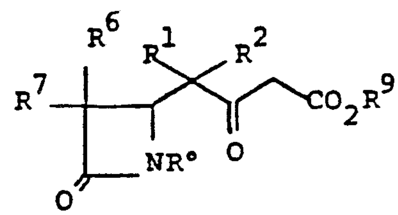

comprenant:
l'oxydation de

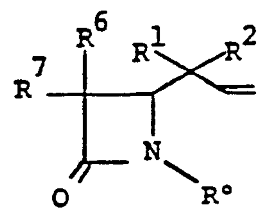

pour former

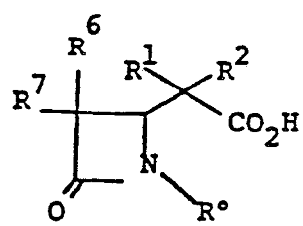

suivie du traitement avec $R^9O_2CCH_2CO_2^{\ominus}$; dans lequel $R^1$, $R^2$, $R^6$ et $R^7$ sont choisis indépendamment dans le groupe constitué de radicaux substitués et non substitué: alkyle, alcényle et alcynyle, ayant de 1 à 10 atomes de carbone; cycloalkyle, cycloalkylalkyle, et alkylcycloalkyle, ayant de 3 à 6 atomes de carbone dans le cycle cycloalkyle et de 1 à 6 atomes de carbone dans les parties alkyles; spirocycloalkyle ayant de 3 à 6 atomes de carbone; phényle, aralkyle, aralcényle et aralcynyle dans lesquels la partie aryle est le phényle et la chaîne alkyle a de 1 à 6 atomes de carbone; hétéroaryle, hétéroaralkyle, hétérocyclyque et hétérocyclylalkyle dans lesquels le substituant ou les substituants relatifs aux radicaux mentionnés ci-dessus sont choisis dans le groupe constitué de: amino, mono-, di-, et trialkylamino, hydroxyle, alcoxyle, mercapto, alkylthio, phénylthio, sulfamoyle, amidino, guanidino, nitro, chloro, bromo, fluoro, iodo, cyano et carboxy; et dans lesquels le ou les hétéroatomes dans les parties hétérocycliques mentionnées ci-dessus sont choisies dans le groupe constitué de 1 à 4 atomes d'oxygène, d'azote ou de soufre; et dans lesquels les parties alkyles des substituants cités ci-dessus ont de 1 à 6 atomes de carbone; et $R^6$, $R^7$ et peuvent aussi être l'hydrogène; $R^9$ est une partie ester pharmaceutiquement acceptable ou est un groupe protecteur facile à éliminer et R° est l'hydrogène ou un groupe protecteur facile à éliminer.

2. Procédé de la revendication 1 dans lequel $R^9O_2CCH_2COO^{\ominus}$ est mis en oeuvre sous forme de $(R^9O_2CCH_2CO_2)_2Mg$.

3. Procédé de préparation d'un composé ayant la structure:

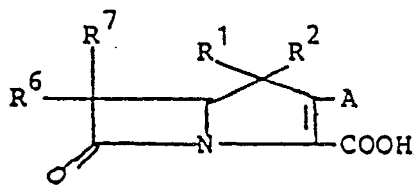

et leurs sels et esters pharmaceutiquement acceptables:

a) dans lesquels A est $SR^8$ et $R^1$, $R^2$, $R^6$, $R^7$ et $R^8$ sont indépendamment choisis dans le groupe constitué de radicaux substitués et non substitués: alkyle, alcényle et alcynyle ayant de 1 à 10 atomes de carbone, cycloalkyle, cycloalkylalkyle et alkylcycloalkyle, ayant de 3 à 6 atomes de carbone dans le cycle cycloalkyle et de 1 à 6 atomes de carbone dans les parties alkyles; spirocycloalkyle ayant de 3 à 6 atomes de carbone; phényle; aralkyle, aralcényle et aralcynyle dans lesquels la partie aryle est le phényle et la chaîne alkyle a de 1 à 6 atomes de carbone; hétéroaryle, hétéroaralkyle, hétérocyclyle et hétérocyclylalkyle dans lesquels le substituant ou les substituants relatifs aux radicaux mentionnés ci-dessus sont choisis dans le groupe constitué de: amino, mono-, di- et trialkylamino, hydroxyle, alcoxyle, mercapto, alkylthio, phénylthio, sulfamoyle, amidino, guanidino, nitro, chloro, bromo, fluoro, iodo, cyano et carboxy; et dans lesquels le ou les hétéroatomes dans les parties hétérocycliques mentionnées ci-dessus sont choisies dans le groupe constitué de 1 à 4 atomes d'oxygène, d'azote ou de soufre; et dans lesquels les parties alkyles des substituants cités ci-dessus ont de 1 à 6 atomes de carbone et $R^6$, $R^7$ et $R^8$ peuvent aussi représenter l'hydrogène; quand $R^6/R^7$ est l'hydrogène et $R^7/R^6$ est le l'hydroxyéthyle, alors $R^8$ n'est pas le 2-aminoéthyle ou un de ses dérivés azoté; comprenant les étapes d'acylation et d'halogénation et le traitement avec le réactif $HSR^8$ d'un composé de formule

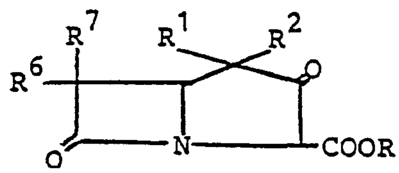

dans lequel $R^6$, $R^7$, $R^1$, $R^2$ et $R^8$ sont définis come ci-dessus R est une partie ester pharmaceutiquement acceptable ou un groupe protecteur du carboxyle facile à éliminer; ou

b) dans lesquels A est $R^8$ et $R^1$, $R^2$, $R^6$, $R^7$ et $R^8$ sont choisis indépendamment dans le groupe constitué de radicaux substitués ou non substitués: alkyle ayant de 1 à 6 atomes de carbone, aralkyle, alcényle et alcynyle ayant de 2 à 6 atomes de carbone, aryle et aralkyle ayant de 6 à 10 atomes de carbone dans le cycle et de 1 à 6 atomes de carbone dans la chaîne alkyle, hétérocyclyle, hétérocyclylthio ($R^8$ est exclu) et hétérocyclylalkyle ayant de 1 à 5 hétéroatoms dans le cycle choisis parmi O, N ou S et de 1 à 6 atomes de carbone dans la chaîne alkyle, cycloalkyle, spirocycloalkyle, et cycloalkylalkyle ayant de 3 à 6 atomes de carbone dans le cycle et de 1 à 6 atomes dans la partie alkyle; dans lesquels les substituants sur $R^1$, $R^2$, $R^6$, $R^7$ et $R^8$ sont choisis parmi les chloro, bromo, fluoro, iodo, hydroxyle, amino, mono-, di et trialkyl-amino substitué, (chaque alkyle ayant de 1 à 6 atomes de carbone) alcoxyle ayant de 1 à 6 atomes de carbone, guanidino, cyano, amidino et carboxyle; $R^6$, $R^7$ et $R^8$ peuvent aussi représenter l'hydrogène; comprenant l'oxydation de

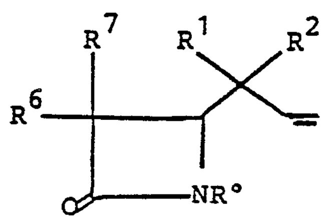

pour former

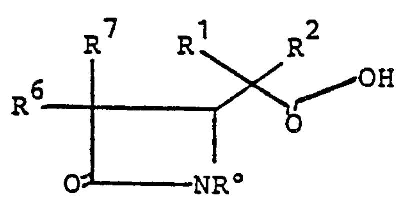

suivi de la réaction avec du dicyclohexylcarbodiimide, de l'éthylchloroformate, de la 2-fluoropyridine, du 1-fluoro-2,4-dinitrobenzène, du chlorure de thionyle ou du chlorure d'oxalyle et du traitement avec $R^8_2CuMgX^\circ$, $R^8MgX^\circ$, $LiCuR^8_2$ ou $R^8_2Cd$ dans lesquels $X^\circ$ est bromo ou chloro pour mettre en place $R^8$,

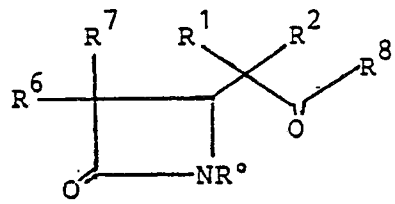

suivi de l'élimination de la protection de N et d'un traitement avec un glyoxylate pour donner

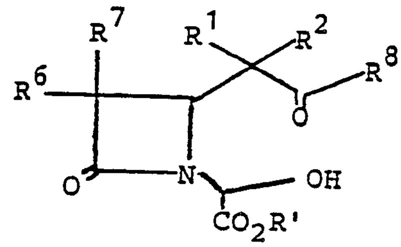

suivi par l'halogénation avec un agent conventionnel d'halogénation et du traitement avec une triorganophosphine pour former

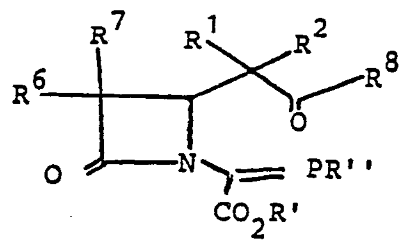

suivi de la cyclisation et de l'élimination de la protection, dans lequel R° est un groupe protecteur facile à éliminer; R' est un groupe protecteur facile à éliminer ou une partie ester pharmaceutiquement acceptable; et R" est un alkyle ayant jusqu'à 4 atomes de carbone ou le phényle.

4. Procéde de préparation d'un composé ayant la structure

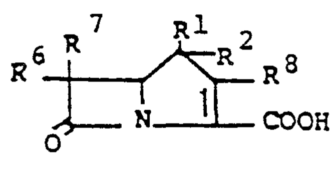

et ses sels et esters pharmacetiquement acceptables; dans lequel $R^1$, $R^2$, $R^6$, $R^7$ et $R^8$ sont choisis indépendamment comme défini dans la revendication 1, et comprenant la cyclisation

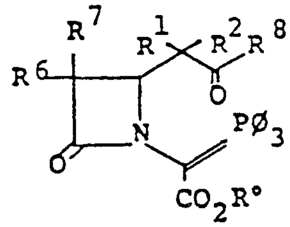

dans lequel φ est le phényle, R° est une partie ester pharmaceutiquement acceptable ou un groupe de blocage facile à éliminer.